# EUROPEAN PATENT SPECIFICATION

(11) **EP 3 058 936 B1**
(45) Date of publication and mention of the grant of the patent: **06.02.2019**
(21) Application number: 14796290.6
(22) Date of filing: 16.10.2014
(51) Int. Cl.: A61K 8/64, A61Q 5/00, A61Q 5/04, A61Q 5/06

(54) **PEPTIDE COMPOSITION AND USES THEREOF**
PEPTIDZUSAMMENSETZUNG UND VERWENDUNGEN DAVON
COMPOSITION PEPTIDIQUE ET UTILISATIONS RESPECTIVES

(30) Priority: 18.10.2013 PT 10724413
(43) Date of publication of application: 24.08.2016
(73) Proprietor: Universidade do Minho, 4704-553 Braga (PT)
(72) Inventor: CAVACO PAULO, Artur Manuel, P-4710-057 Braga (PT); FREITAS DA CRUZ, Célia, P-4810-035 Guimarães (PT); MACEDO FRANCESKO FERNANDES, Margarida Maria, P-4700-089 Braga (PT)
(74) Representative: Silvestre de Almeida Ferreira, Luís Humberto
(86) International application number: PCT/IB2014/065375
(87) International publication number: WO 2015/056216

(56) References cited:
- EP-A1- 1 705 187
- EP-A1- 1 705 187
- EP-A1- 1 705 188
- WO-A1-2005/049834
- WO-A1-2011/072991
- WO-A2-2004/048399
- FR-A1- 2 706 300
- FR-A1- 2 876 286
- JP-A- H0 656 889
- US-A- 5 028 419
- MICHAEL A ROGERS ET AL: "Genomic Characterization of the Human Type I Cuticular Hair Keratin hHa2 and Identification of an Adjacent Novel Type I Hair Keratin Gene hHa5", JOURNAL OF INVESTIGATIVE DERMATOLOGY, vol. 107, no. 4, 1 October 1996 (1996-10-01), pages 633-638, XP055391909, US ISSN: 0022-202X, DOI: 10.1111/1523-1747.ep12584243
- MICHAEL A. ROGERS ET AL: "Characterization of a 300 kbp Region of Human DNA Containing the Type II Hair Keratin Gene Domain", JOURNAL OF INVESTIGATIVE DERMATOLOGY, vol. 114, no. 3, 1 March 2000 (2000-03-01) , pages 464-472, XP055391920, US ISSN: 0022-202X, DOI: 10.1046/j.1523-1747.2000.00910.x
- PEREZ C ET AL: "Genomic organization and promoter characterization of two human UHS keratin genes", GENE, ELSEVIER, AMSTERDAM, NL, vol. 227, no. 2, 18 February 1999 (1999-02-18), pages 137-148, XP004158727, ISSN: 0378-1119, DOI: 10.1016/S0378-1119(98)00616-7
- MICHAEL A ROGERS ET AL: "Genomic Characterization of the Human Type I Cuticular Hair Keratin hHa2 and Identification of an Adjacent Novel Type I Hair Keratin Gene hHa5", JOURNAL OF INVESTIGATIVE DERMATOLOGY, vol. 107, no. 4, 1 October 1996 (1996-10-01), pages 633-638, XP055391909, US ISSN: 0022-202X, DOI: 10.1111/1523-1747.ep12584243
- MICHAEL A. ROGERS ET AL: "Characterization of a 300 kbp Region of Human DNA Containing the Type II Hair Keratin Gene Domain", JOURNAL OF INVESTIGATIVE DERMATOLOGY, vol. 114, no. 3, 1 March 2000 (2000-03-01) , pages 464-472, XP055391920, US ISSN: 0022-202X, DOI: 10.1046/j.1523-1747.2000.00910.x
- PEREZ C ET AL: "Genomic organization and promoter characterization of two human UHS keratin genes", GENE, ELSEVIER, AMSTERDAM, NL, vol. 227, no. 2, 18 February 1999 (1999-02-18), pages 137-148, XP004158727, ISSN: 0378-1119, DOI: 10.1016/S0378-1119(98)00616-7

## Description

### Technical Domain

The current application corresponds to a composition that comprises at least one peptide according to the claims, which are based on keratin and keratin associated proteins, containing 2 to 5 cysteines with the purpose of treatment and cosmetics of animal hair, in preference human hair.

### Prior Art

Human hair has a significant social role in most of the various world cultures, particularly for female population. Thus, there is a constant desire to improve and change hair characteristics, such as its natural texture.

There are several differences in hair characteristics between different human ethnicities, as well as between individuals of the same ethnicity, such as length, thickness, color and texture.

Hair is composed of approximately 65% to 95% protein. The remaining constituents include water, lipids, pigments and trace elements. The majority of the proteins present in human hair correspond to keratin and keratin-associated proteins.

Human hair fibers structure consists of cuticle, cortex and medulla. The cuticle constitutes about 15% by weight of the hair and consists of overlapping layers of cells, similar to a system of scales, with high content of cysteine. It provides a protective character to the hair fiber. The cortex is the middle region of the hair being responsible for the strength, elasticity and hair color. It is composed of several cell types and represents about 80% of the weight of the hair. The medulla corresponds to a central beam of cells, and is absent in some hairs.

Keratins and mainly keratin-associated proteins have high sulfur content, present in the cysteine amino acid. The presence of sulfur is essential to the hair structure, as it allows the formation of disulfide bonds between amino acids of the polypeptide chains, due to oxidation of cysteine. The existence of these bonds is largely responsible for the structure and texture of the hair.

There are several hair styling methods that involve breakage and reestablishment of disulfide bonds, allowing relaxation and straightening of the hair. However, the most effective methods currently used to modulate hair contain harmful chemicals such as sodium hydroxide, potassium hydroxide, lithium hydroxide, guanidine hydroxide, ammonium thioglycolate or sodium sulfate. These methods can damage the scalp and the hair fiber, leading to its weakening and reducing its tensile strength. Formaldehyde, an extremely toxic chemical, is also used in hair straightening products. Other hair treatments that do not involve so much damage to the hair and the consumer are usually very expensive, time-consuming and/or have low efficacy. Thus there is a constant demand for formulations that efficiently model the hair fiber without damage.

Peptides, proteins, amino acids and its derivatives have also been used in compositions for personal care products, namely hair conditioning and strengthening. For example, the document WO 00/23039 discloses a composition for hair treatment containing intermediate filament proteins, namely artificial keratin. The document EP 0488242 discloses a hair treating agent containing 3% to 10% by weight of cysteine and salts thereof, a polyhydric alcohol or a saccharide containing four to twenty carbon atoms, three or more hydroxyl groups in the molecule and no aldehyde or ketone group.

The current invention is distinguished by the use of peptides, while the other applications refer the use of, respectively, proteins and amino acids in isolation and together with other types of compounds. The peptides in this innovation peptide can penetrate into the human hair in order to improve hair fiber resistance.

The document WO 00/51556 discloses a hair treatment composition that contains four or more discrete amino acids selected from histidine, lysine, methionine, tyrosine, tryptophan or cysteine. This document describes peptides without referring sequences and providing a composition essentially based on histidine, lysine, methionine, tyrosine, tryptophan or cysteine.

The document PT 103484 describes a formulation for cosmetic applications that uses hydrophobic binding domains and/or carbohydrates, in order to enhance its properties and to repair hair damage. The binding domains used are hydrolyzed milk protein, a model of human surfactant protein as well as biologically active and synthetic peptides. The current invention is distinguished by the innovative use of synthetic peptide sequences analogous to keratin proteins instead of surfactant proteins. Furthermore, it does not rely on hydrophobic binding domains and/or carbohydrates, but in other interactions, namely disulfide bonds.

Enzymes have also been used as activating agents for hair treatment, such as in the document WO 00/64405. The document WO 2012/13593 discloses a cosmetic kit for hair conformational change that acts specifically in the disulfide bonds of the hair keratin, through enzyme activating agents and proteolytic enzymes.

As described in the last document there are hair treatments that include actions at the level of the hair disulfide bonds. Below we highlight some examples.

The document WO 97/11672 reports a method for permanent hair processing using tris(2-carboxyethyl)phosphine (TCEP), and other water-soluble tertiary phosphines to break disulfide bonds, whose reaction occurs in acidic environment. The document US 5635170 discloses a composition for permanent shaping of hair based on a keratin reducing agent, which contains N-glycyl-L-cysteine and/or L-cysteinyl-glycine. The pH range of this composition is 6.5 to 9.0. The document WO 2008/081348 refers a method and composition for permanent modulation of hair, through the use of 1% to 30% of N-alkyl-2-mercapto acetamide as a keratin reducing agent. It also contains at least one cationic surfactant for permanently shaping hair and the resulting process. The document WO 2006/001536 describes an agent for permanent hair processing that contains a derivative of mercaptocarboxylic acid, which allows processing and reduction of hair keratin in the acidic and neutral range of the pH. The document US 2010/0272666 discloses a hair cosmetic composition for hair treatment, containing 5 to 50 amino acids, without containing cysteine or its derivatives. Thus, this invention is distinguished by the existence of specific amino acid sequences, which contain cysteine, allowing the formation of disulfide bonds that stabilize and protect the hair fiber. Cysteine containing peptides for strengthening hair fibers have been described for example in EP 1 705 188 A1.

In a previous article by Fernandes et al. (Fernandes, Lima, Loureiro, Gomes, & Cavaco-Paulo, 2012), it is performed the toxicology evaluation of a peptide sequence for hair care use, containing 13 amino acids with two cysteines in its composition. However, in this article it is not mentioned or suggested that the percentage of cysteine in a peptide sequence may have some effect on the resistance of the hair. Also, in the present innovation, the number of amino acids of each peptide sequence is 6 to 12.

### General Description

Thus, the present invention aims to provide a composition for treatment of the hair, including animal and human hair, without the use of chemicals harmful to the hair fiber and consumer health and that does not present the drawbacks found in the state of the art.

The compositions described in the current invention, after prolonged use, provide hair with soft, shiny, undamaged texture and with the desired features. The peptide compositions with a specific number of amino acids and cysteines act synergically providing resistance to strength, toughness and elasticity to the hair. Therefore, the compositions of the current invention are particularly relevant for hair treatment, hair dying, hair perms, etc.

The present application describes a peptide composition for hair as a hair strengthener of human or animal hair, which comprises at least one peptide with 6-12 amino acids length (namely 6, 7, 8, 9, 10, 11, 12 amino acids), where 2-5 of those amino acids correspond to cysteine, preferably 2, 3, 4 or 5 of those amino acids are cysteines and dermatologically suitable excipients, wherein the peptides comprise at least one of the sequences of the following list with a degree of homology equal or higher than 90%: SEQ.ID NO:75; SEQ.ID NO:412; SEQ.ID NO:1131. The peptides are of nature to penetrate the hair, increasing it resistance and reducing it breakage.

In the embodiment, for improved results, the peptide (or peptides) of the peptide composition for hair care can comprise 10-11 amino acids.

In the embodiment of the peptide composition for hair care treatment, the referred peptides can also contain a percentage of hydrophobic amino acids, not higher than 60%, and preferably less than 41% for better results. Preferably, the composition can also comprise at least one hydrophobic amino acid selected from the following list: phenylalanine, alanine, leucine, methionine, isoleucine, tryptophan, proline, valine or their mixtures.

In yet another embodiment, the amount of cysteine of the peptide composition for hair treatment may vary from 10% to 50% of the total of amino acids of the peptide sequence, preferably 20-30%, and even more preferably 25%.

In an embodiment of the composition, with better results of the peptide (or peptides) of the peptide composition for hair treatment, the sequence of peptide(s) can comprise at least one sequence of the following list with a degree of homology greater than or equal to 90%: SEQ.ID NO:75, 412 and 1131, preferably with a degree of homology greater than or equal to 91%, 92%, 93%, 94%, 95%, 96%, 97%, 98%, 99%, 100%.

In an embodiment, improved results for the peptide (or peptides) of the peptide composition for hair treatment can comprise at least one of the sequences of the following list with a degree of homology equal or greater than 90%: SEQ.ID NO:75; SEQ.ID NO:412; SEQ.ID NO:1131; preferably with a degree of homology equal or greater than 91%, 92%, 93%, 94%, 95%, 96%, 97%, 98%, 99%, 100%.

In other embodiment, the concentration of the peptide of the peptide composition for hair treatment can vary between 0.001%-20% (w/w), preferably 0.01-5% (w/w).

In yet other embodiment, the peptide composition for hair treatment can comprise at least one excipient, selected from the following list: surfactants, emulsifiers, preservatives, thickeners, organic polymers, humectants, silicones, oils, fragrances, vitamins, buffers.

In another embodiment, the peptide composition for hair treatment can comprise at least one anionic surfactant selected from the following list: alkylbenzene sulfonates, ammonium lauryl sulfate, ammonium lauryl sulfate, ammonium xylenesulfonate, sodium C14-16 olefin sulfonate, sodium cocoyl sarcosinate, sodium laureth sulfate, sodium lauryl sulfate, sodium lauryl sulfoacetate, sodium myreth sulfate, sodium xylenesulfonate, TEA-dodecylbenzenesulfonate, ethyl PEG-15 cocamine sulfate, dioctyl sodium sulfosuccinate, or any mixture thereof.

In an embodiment, the peptide composition for hair treatment can comprise at least one amphoteric surfactant selected from the following list: cocamidopropyl betaine, coco betaine, cocoamphoacetate, cocoamphodipropionate, disodium cocoamphodiacetate, disodium cocoamphodipropionate, lauroamphoacetate, sodium cocoyl isethionate, or any mixture thereof.

In other embodiment, the peptide composition for hair treatment can comprise at least one cationic surfactant selected from the following list: quaternary ammonium compounds, behentrimonium chloride, behentrimonium methosulfate, benzalkonium chloride, betrimonium chloride, binnamidopropyltrimonium chloride, cocotrimonium chloride, dicetyldimonium chloride, dicocodimonium chloride, dihydrogenated tallow dimethylammonium chloride, hydrogenated Palm trimethylammonium chloride, laurtrimonium chloride, quaternium-15, quaternium-18 bentonite, quaternium-22 hectonite, stearalkonium chloride, tallowtrimonium chloride, tricetyldimonium chloride, or any mixture thereof.

In yet other embodiment, the peptide composition for hair treatment can comprise at least one non-ionic surfactant selected from the following list: decyl glucoside, laureth-10 (lauryl ether 10), laureth-23, Laureth-4, PEG-10 sorbitan laurate, polysorbate-(20, 21, 40, 60, 61, 65, 80, 81), PPG-1 trideceth-6, sorbitol, steareth-(2, 10, 15, 20), C11-21 pareth-(3-30), C12-20 acid PEG-8 ester, or their mixtures.

In yet other embodiment, the peptide composition for hair treatment can comprise at least one emulsifier selected from the following list: caprylic/capric/diglyceryl succinate, C10-15 pareth-(2,4,6,8) phosphate, C14-16 glycol palmitate, C18-20 glycol isostearate, ceteareth-(4-60), cocamidopropyl lauryl ether, deceth-(3-10), DIPA-hydrogenated cocoate, dipentaerythrityl hydroxystearate, dipentaerythrityl hydroxyisostearate, dipentaerythrityl hexacaprate/caprylate, dodoxynol-(5,6,7,9,12), nonoxynol-(1-35), octoxynol-(1-70), Octyldodeceth-(2,5,16,20,25), Palm kernel glycerides, or any mixture thereof.

In other embodiment, the peptide composition for hair treatment can comprise at least one preservative selected from the following list: butyl paraben, diazolidinyl urea, DMDM hydantoin, ethyl paraben, imidazolidinyl urea, iodopropynyl butylcarbamate, isobutyl paraben, methyl paraben, methylchloroisothiazolinone, methylisothiazolinone, phenoxyethanol, propyl paraben, sodium benzoate, or any mixture thereof.

In other embodiment, the peptide composition for hair treatment can comprise at least one thickner selected from the following list: aluminum stearates / isostearates / myristates / laurates / palmitates, glycol distearate, hydrogenated castor oil, hydrogenated castor oil hydroxystearate, hydrogenated castor oil isostearate, hydrogenated castor oil stearate, hydrogenated castor PEG-8 esters, PEG-150 distearate, or any mixture thereof.

In other embodiment, the peptide composition for hair treatment can comprise at least one natural polymer derived selected from the following list: carboxymethyl hydroxyethyl celulose, carboxymethyl hydroxypropyl guar, cellulose, ethyl celulose, hydroxybutyl methylcellulose, hydroxyethylcellulose, hydroxymethylcellulose, lauryl polyglucose, or any mixture thereof.

In other embodiment, the peptide composition for hair treatment can comprise at least one humectant selected from the following list: 1,2,6 hexanetriol, dipropylene glycol, glycerin, hexylene glycol, panthenol, phytantriol, propylene glycol, sodium PCA, sorbitol, triethylene glycol, polyglyceryl sorbitol, glucose, fructose, polydextrose, potassium PCA, hydrogenated honey, hyaluronic acid, inositol, hexanediol beeswax, hexanetriol beeswax, hydrolyzed elastin, hydrolyzed collagen, hydrolyzed silk, hydrolyzed keratin, erythritol, capryl glycol, isoceteth-(3-10, 20, 30), isolaureth-(3-10, 20, 30), laneth-(5-50), laureth-(1-30), steareth-(4-20), trideceth-(5-50), or any mixture thereof.

In other embodiment, the peptide composition for hair treatment can comprise at least one cationic polymer selected from the following list: polyquaternium-10, polyquaternium-7, polyquaternium-11m guar hydroxypropyltrimonium chloride, or any mixture thereof.

In other embodiment, the peptide composition for hair treatment can comprise at least one silicone selected from the following list: amodimethicone, amodimethicone, trideceth-12, cetrimonium, chloride mixture, behenoxy, dimethicone sparingly, cetearyl methicone, cetyl dimethicone, cyclomethicone, cyclopentasiloxane, dimethicone, dimethicone copolyol, dimethicone copolyol, dimethiconol, hydrolyzed wheat protein hydroxypropyl polysiloxane, stearoxy dimethicone sparingly, stearyl dimethicone, trimethylsilylamodimethicone, lauryl methicone copolyol, or any mixture thereof.

In yet other embodiment, the peptide composition for hair treatment can comprise at least one organic oil selected from the following list: mineral oil, paraffin, petrolatum, or any mixture thereof.

In yet other embodiment, the peptide composition for hair treatment can comprise at least one protein selected from the following list: cocodimonium hydroxypropyl hydrolyzed casein, cocodimonium hydroxypropyl hydrolyzed collagen, cocodimonium hydroxypropyl hydrolyzed hair keratin, cocodimonium hydroxypropyl hydrolyzed keratin, cocodimonium hydroxypropyl hydrolyzed rice protein, cocodimonium hydroxypropyl hydrolyzed silk, cocodimonium hydroxypropyl hydrolyzed soy protein, cocodimonium hydroxypropyl hydrolyzed wheat protein, cocodimonium hydroxypropyl silk amino acids, cocoyl hydrolyzed collagen, cocoyl hydrolyzed keratin, hydrolyzed keratin, hydrolyzed oat flour, hydrolyzed silk, hydrolyzed silk protein, hydrolyzed soy protein, hydrolyzed wheat protein, hydrolyzed wheat protein, keratin, potassium cocoyl hydrolyzed collagen, TEA-cocoyl hydrolyzed collagen, TEA-cocoyl hydrolyzed soy protein, or any mixture thereof.

In other embodiment, the peptide composition for hair treatment can comprise at least one vitamin selected from the following list: retinol, retinyl palmitate tocopherol acetate, or any mixture thereof.

In other embodiment, the peptide composition for hair treatment can comprise at least one ester emollient selected from the following list: butyl myristate, butyl stearate, C12-15 alkyl benzoate, caprylic/capric triglyceride, cetyl octanoate, cetyl stearate, cetearyl stearate, decyl oleate, dimethyl lauramine isostearate, glyceryl stearate, glyceryl adipate, glyceryl arachidate, glyceryl arachidonate, glyceryl behenate, glyceryl caprate, glyceryl caprylate, glyceryl caprylate/caprate, glyceryl citrate/lactate/linoleate/oleate, glyceryl cocoate, glyceryl diarachidate, glyceryl dibehenate, glyceryl dierucate, glyceryl dihydroxystearate, glyceryl diisopalmitate, glyceryl diisostearate, glyceryl dilaurate, glyceryl dilinoleate, glyceryl dimyristate, glyceryl dioleate, glyceryl dipalmitate, glyceryl dipalmitoleate, glyceryl diricinoleate, glyceryl distearate, glyceryl erucate, glycol stearate, isocetyl stearate, isopropyl myristate, isopropyl palmitate, isopropyl stearate, isostearyl stearate, octyl palmitate, octyl stearate, propylene glycol dicaprylate/dicaprate, sorbitan benzoate, sorbitan caprylate, sorbitan isostearate, sorbitan laurate, sorbitan tristearate, stearyl stearate, tocopheryl linoleate, or any mixture thereof.

In other embodiment, the peptide composition for hair treatment can comprise at least one alkanolamide selected from the following list: acetamide MEA, cocamide DEA, cocamide MEA, lactamide MEA, lauramide DEA, propylene glycol, lauramide MEA, lecithinamide DEA, linoleamide DEA, linoleamide MEA, linoleamide MIPA, myristamide DEA, myristamide MEA, myristamide MIPA, oleamide DEA, oleamide DEA, oleamide MEA, oleamide MIPA, soyamide DEA, stearamide MEA, or any mixture thereof.

In yet other embodiment, the peptide composition for hair treatment can comprise at least one amine selected from the following list: behentamidopropyl dimethylamine, cocamidopropyl dimethylamine, isostearamidopropyl dimethylamine, lauramidopropyl dimethylamine, myristamidopropyl dimethylamine, oleamidopropyl dimethylamine, palmitamidopropyl dimethylamine, stearamidopropyl dimethylamine, tallamidopropyl dimethylamine, or any mixture thereof.

In yet other embodiment, the peptide composition for hair treatment can comprise at least one pH adjuster selected from the following list: ascorbic acid, citric acid, sodium hydroxide, triethanolamine, or any mixture thereof.

In yet other embodiment, the peptide composition for hair treatment can comprise at least one salt selected from the following list: calcium chloride, magnesium chloride, magnesium sulfate, potassium chloride, potassium glycol sulfate, sodium chloride, or any mixture thereof.

In yet other embodiment, the peptide composition for hair treatment can comprise at least one aliphatic alcohol selected from the following list: behenyl alcohol, cetearyl alcohol, cetyl alcohol, isocetyl alcohol, isostearyl alcohol, lauryl alcohol, myristyl alcohol, stearyl alcohol, C30-50 alcohols, lanolin alcohol, or any mixture thereof.

In another embodiment, the peptide composition for hair treatment can comprise at least one UV filter/sunscreen selected from the following list: benzophenone-(2, 3, 4, 5, 6, 7, 8, 9, or 10), benzophenone-4, benzyl salicylate, benzylidene camphor sulfonic acid, bornelone, ethyl cinnamate, ethylhexyl methoxycinnamate (octyl methoxycinnamate), octoxynol-40, octoxynol-20, octyl methoxycinnamate, octyl salicylate, oxybenzone, phenyl ketone, PEG-25 PABA, polyacrylamidomethyl benzylidene camphor, or any mixture thereof.

In other embodiment, the peptide composition for hair treatment can comprise at least one natural oil selected from the following list: coconut oil, jojoba oil, olive oil, palm Oil, safflower oil, sesame seed oil, shea butter, sweet almond oil, wheat germ oil, or any mixture thereof.

In yet other embodiment, the peptide composition for hair treatment can comprise at least one amine oxide selected from the following list: cocamine oxide, lauramine oxide, or any mixture thereof.

In other embodiment, the peptide composition for hair treatment can comprise at least one chelate selected from the following list: diiospropyl oxalate, disodium EDTA, disodium EDTA-copper, HEDTA, oxalic acid, potassium citrate, sodium citrate, dodium oxalate, TEA-EDTA, tetrasodium EDTA, trisodium EDTA, trisodium HEDTA, or any mixture thereof.

In other embodiment, the peptide composition for hair treatment can comprise at least one fatty acid selected from the following list: arichidonic acid, capric acid, coconut fatty acid, lauric acid, linoleic acid, linolenic acid, myristic acid, palmitic acid, pantothenic acid, stearic acid, caproic acid, capryleth-(4, 6, 9) carboxylic acid, isostearic acid, or any mixture thereof.

In other embodiment, the peptide composition for hair treatment can comprise at least one agent antimicrobial/antibacterial selected from the following list: glyoxal, triclosan, or any mixture thereof.

In other embodiment, the peptide composition for hair treatment can comprise at least one PEG-modified material selected from the following list: PEG-150 pentaerythirtyl tetrastearate, PEG-(-2, -3, -4, -6, -8, -12, -20, -32, -50, -150, -175) distearate, PEG-10 castor oil, PEG-10 cocamine, PEG-10 cocoate, PEG-10 coconut oil esters, PEG-10 glyceryl oleate, PEG-10 glyceryl pibsa tallate, PEG-10 glyceryl stearate, PEG-10 hydrogenated lanolin, PEG-10 hydrogenated tallow amine, PEG-10 isolauryl thioether, PEG-10 isostearate, PEG-10 lanolate, PEG-10 lanolin, PEG-10 laurate, PEG-10 oleate, PEG-10 olive glycerides, PEG-10 polyglyceryl-2 laurate, PEG-10 propylene glycol, PEG-10 sorbitan laurate, PEG-10 soya sterol, PEG-10 soyamine, PEG-10 stearamine, PEG-10 stearate, PEG-10 stearyl benzonium chloride, PEG-10 tallate, PEG-10 tallow aminopropylamine, PEG-100, PEG-100 castor oil, PEG-100 hydrogenated castor oil, PEG-100 lanolin, PEG-100 stearate, PEG-40 hydrogenated castor Oil, PEG-60, PEG-55 propylene glycol distearate, or any mixture thereof.

In other embodiment, the peptide composition for hair treatment can comprise at least one polymer selected from the following list: carbomer, dodecanedioic acid/cetearyl alcohol/glycol copolymer, hydrogenated C6-14 olefin polymers, hydrogenated ethylene/propylene/styrene copolymer: polyacrylic acid, polymethyl methacrylate: polymer, polyvinyl acetate, polyvinyl alcohol, PPG, PPG-25-laureth-25, PPG-5 pentaerithrityl ether, PPG-75-PEG-300-hexylene glycol, polyvinylpyrrolidone, PVP/VA (polyvinylpyrrolidone/vinyl acetate copolymer), sodium carbomer, TEA-carbomer, poloxamer (100-407), poloxamine, polyacrylamidomethylpropane sulfonic acid, polyethylene terephthalate, or any mixture thereof.

In other embodiment, the peptide composition for hair treatment can comprise at least one antistatic agent selected from the following list: apricotamidopropyl ethyldimonium ethosulfate, apricotamidopropyl ethyldimonium lactate, cocamidopropyl ethyldimonium ethosulfate, cocamidopropyl ethyldimonium lactate, lauramidopropyl ethyldimonium ethosulfate, lauramidopropyl ethyldimonium lactate, linoleamidopropyl ethyldimonium ethosulfate, linoleamidopropyl ethyldimonium lactate, myristamidopropyl ethyldimonium ethosulfate, myristamidopropyl ethyldimonium lactate, oleamidopropyl ethyldimonium ethosulfate, oleamidopropyl ethyldimonium lactate, stearamidopropyl ethyldimonium ethosulfate, stearamidopropyl ethyldimonium lactate, or any mixture thereof.

In other embodiment, the peptide composition for hair treatment can comprise at least one alcohol selected from the following list: SD alcohol 40, witch hazel, isopropanol, or any mixture thereof.

In yet other embodiment, the peptide composition for hair treatment can comprise fragrances, oils or any mixture thereof.

In other embodiment, the peptide composition for hair treatment can be used in medicine, veterinary and/or for cosmetics, preferably for the treatment of hair, mainly for animal or human, particularly for treating diseases of the scalp, particularly scalp irritation, alopecia areata, lichen planus, folliculitis keloid of the neck, trichorrhexis nodosa, tricodistrophy, pili torti, tricorrexis invaginata, moniletrix, uncombable hair syndrome.

In other embodiment, the composition may comprise a dye agent linked to the N or C-terminal of the referred peptides.

In yet other embodiment is the use of the described composition for hair coloring.

Other aspect of the embodiment is the use of the described composition as a hair strengthener or as fixer of perms and/or curly hairs.

It is also described in this application shampoo, lotion, serum, cream, conditioner, foam, elixir, oil, aerosol or mask comprising the composition presented in this application.

The present application discloses a composition as claimed for hair treatment that comprises, in whole or in part, one or more peptide sequences of 6 to 12 amino acid residues based on keratin and keratin-associated proteins having 2 to 5 cysteine residues, preferably having 3 to 5 residues of cysteine, for treatment and cosmetics of the hair, preferably human hair, chemically pre-treated or not. Thus the presence of cysteine in the peptide sequence (higher than 10%, preferably more than 15%) in combination with a percentage of hydrophobic amino acids ensures that the peptides can have a lasting fixation in the hair, improving the human hair properties such as elasticity and strength.

Surprisingly, the described peptide compositions in which the peptide(s) comprise 2 to 5 cysteines allow penetration of the peptide(s) and enhance the properties of hair, preferably 3-5 cysteines. Thus, described peptide(s) containing 2-5 cysteine in order to allow hair penetration and enrichment of the hair properties, such as elasticity, resistance, reduce eventual hair damage, as well as improve and change hair characteristics.

The peptide compositions described in the present application surprisingly enrich and improve the properties and characteristics of the hair, such as elasticity, strength and appearance, repairing damaged keratinous fiber. Therefore, formulation's high cysteine content is used to improve and/or change its characteristics, such as hair curl or uncurl. The sequence of peptides can have also preferably a percentage of hydrophobic amino acids not exceeding 60%, improving even further the results. Examples of hydrophobic amino acids are phenylalanine, alanine, leucine, methionine, isoleucine, tryptophan, proline, valine, and others.

In the context of the present description, the peptide composition can also be applied to the hair and in particular to the human hair as, but not limited to, aqueous solution or conventional shampoo or conditioner. It can also be used as a lotion, foam, aerosol, gel, mask, and application formulation with or without subsequent rinsing.

The concentration of peptide to be used depends on several features such as the condition of the hair, the origin and the formulation of the hair care product.

### Detailed Description

The present application describes a composition for hair treatment that comprises different peptides, which are based in the structure of keratin and keratin associated proteins.

The compositions described in the present application allow surprisingly the dermo-cosmetic treatment of animal hair, including human hair, chemically pre-treated or not. The composition described in the present invention, through the use of specific peptides, allows the preparation of keratinous fiber damages, due to the high binding capacity of the keratin peptides, including through disulfide bridges.

The described compositions improve the properties and characteristics of the hair, such as elasticity, resistance and appearance, repairing putative damages of the hair.

The peptides here defined are peptide sequences which bind with a certain affinity to the hair. The peptides used in this invention are composed by 6 to 12 amino acids, and are constituted by a minimum of 2 and a maximum of 5 cysteines, preferably 3-5 cysteines.

The peptide composition for hair treatment described allows a resistance increase due to the presence of the cysteine-rich peptide, which leads to the resistance of the hair even after several rinsing.

Every peptide can be used together or separately, as well as all or part of the peptide sequence in the hair composition. Each peptide sequence contains amino acids with sulfur, specifically cysteine, which interacts with the hair and allows the formation of intermolecular cross-linking, stabilizing the keratinous fiber.

The peptide composition described uses a high content on cysteine in order to enrich the hair properties, such as improve elasticity and resistance, reduce putative damage of the hair, improve and/or change hair characteristics. Regarding the interaction with the keratinous fibers, the cysteine is 10% to 50% of the total amount of amino acids of the peptide sequence. Additionally, the number of amino acids of the peptide sequence is preferable from 6 to 12.

The peptides can be used separately or in combination of two or more peptides. The concentration of the peptide to be used depends on several characteristics, such as hair condition, origin and the formulation of the product for hair treatment. The content of the hair composition of the present invention is as example 1-0.001% (w/w) in mass.

The peptides of the present invention can be prepared by conventional methods of peptide synthesis, well known in the state of the art.

Additionally many companies provide customized services for peptide synthesis.

The current invention describes peptides that link to the hair, and which sequence of amino acids includes cysteines where the sequence is selected from the group sequences ID NO:75, 412 and 1131. The other sequences described herein are so as reference sequences.

The sequence of the 1239 peptides referred is listed in the table of the figure 1.

As example of hair, it was used virgin human hair tresses, acquired from the International Hair Importers and Products, Inc. (New York). The term virgin hair is applied to all the hair that was never subject or was at least 10 years without making any chemical treatment. Several different hair samples such as African, Asian and Caucasian hair are commercially available in several companies, such as the company mentioned above. Optionally, the hair samples can be treated, for example, using hydrogen peroxide to bleach the hair, needed for techniques such as hair dying.

In the context of this invention, the peptides can be applied to the hair, such as the human hair in the form of, but not limited to, aqueous or conventional preparation of shampoo or conditioner. It can also be in the form of lotion, foam, spray, gel, mask, formulation applied with or without subsequent rinsing.

This invention can be prepared by peptide coupling with an agent of these preparations directly or via a spacer.

This coupling interaction can be performed by covalent or non-covalent bonds, such as hydrogen bond, electrostatic interactions, hydrophobic interactions or van der Waals interactions. The spacercan be used to separate the peptide from the preparation agent, ensuring that the agent does not interfere with the peptide linkage to the hair.

The present invention can be understood more clearly and accurately by reading the following examples, which are indicative of preferred embodiments of the invention.

### Examples of applications

The examples that are within the scope of the claims represent different embodiments of the invention; all other examples are comparative examples.

### Reference Example 1:

The present application treats human hair through several commercial formulations with and without the use of the peptides from the sequence ID NO: 5. The hair was supplied from International Hair Importers and Products, Inc. (New York).

The tests were performed with human hair after 8 treatments of bleaching, at 50ºC in 0.1 M Na₂CO₃/NaHCO₃ buffer, at pH=9, 10% H₂O₂, for 1 hour.

Several formulations were tested:
- hair serum with 15% PG;
- hair mask.

The mask used in this application was a basic commercial formulation with water, denaturing alcohol, propylene glycol, ether dicaprylic, cetylstearyl alcohol, behentrimonium chloride, cetyl ester, polysorbate 20, hydrolyzed wheat protein, hydrolyzed wheat starch, benzyl alcohol and fragrance.

The hair serum used in this application was a basic commercial formulation with water, denaturing alcohol, propylene glycol, polysorbate 20, hydrolyzed wheat protein, hydrolyzed wheat starch, crosslinked polymer alkyl acrylate/C10-30, triethanolamine, benzyl alcohol, fragrance.

Each of the formulations was tested with and without the peptide sequence ID NO:5, which contains in the sequence 15% of cysteine. The formulations containing the peptide SEQ ID NO:5 had a concentration of peptide of 0.1mg/mL, in a ratio 1:1 (v/v).

To demonstrate the effect was also tested:
- a peptide whose sequence does not contain cysteine, with approximately 41% hydrophobic amino acids;
- a peptide which contains in its sequence 8% cysteine, with approximately 58% hydrophobic amino acids.

The hair mask was applied to the hair after 8 bleaching treatments, being left to act for 15 minutes, mimicking the procedure indicated in commercial masks. Posteriorly, the hair was washed. The serum was applied to the hair after 8 bleaching treatments, being left to act for 1 hour at 37ºC. Posteriorly, the hair was not washed, as in typical commercial procedures the serum should be applied in dry hair. The hair was also tested after 5 applications.

The peptide from the sequence ID NO: 5 was able to penetrate in the hair fiber for all the formulations.

After the treatment, mechanical tests were performed, using a cell with 2.5 N maximum load and a deformation rate of 1.5 mm/min. Each hair was individually mounted in the tensile jig by means of a paper template with a fixed gauge length of 20 mm.

**Table 1 - Young modulus of virgin hair without treatments and after 8 times bleaching treatments.**

| Hair type | Young modulus (MPa) |
|---|---|
| Virgin hair | 6579 |
| Hair after 8 time bleaching | 5294 |
| Serum(with a 15% cysteine and 50% hydrophobic amino acids peptide) | 7149 |
| Serum for comparison(with a 41% hydrophobic amino acid without cysteine peptide) | 6180 |
| Serum for comparison (with a 8% cysteine and 58% hydrophobic amino acid peptide) | 6456 |
| Serum for comparison (without peptide) | 6034 |

**Table 2 - Young modulus for different types of hair treatment. The peptide in these treatments is the peptide from sequence ID NO: 5.**

| Type of treatment | Young modulus after 1 application (MPa) | Young modulus after 5 applications (MPa) |
|---|---|---|
| Serum (with a 15% cysteine and 50% hydrophobic amino acid peptide) | 7149 | 7318 |
| Serum for comparison (without peptide) | 6034 | 6112 |
| Mask (with a 15% cysteine and 50% hydrophobic amino acid peptide) | 6175 | 7075 |
| Mask for comparison(without peptide) | 5514 | 5685 |

The formulations which contain the sequence ID NO:5 induce an increase in mechanical resistance of the damaged hair. After 5 applications, the hair treated with the sequence ID NO: 5 maintain the high resistance, having a higher increase in the resistance than without the peptide.

### Reference Example 2:

This example discloses the treatment of human hair with peptides containing cysteine, and in this case the peptide containing the sequence ID NO: 409, based in the assumption that small peptides are able to penetrate in the hair fiber cuticle.

The hair was supplied from International Hair Importers and Products, Inc. (New York). Hair fibers were pre-treated by bleaching. The formulation was tested in different hair types:
- virgin hair washed, with the cuticle intact and absence of chemical damages;
- hair after 8 bleaching treatments, at 50ºC in 0.1 M Na₂CO₃/NaHCO₃ buffer, at pH=9, 10% H₂O₂, for 1 hour.

The incorporation of the peptides was performed by direct application in the hair surface. The mechanical resistance tests were performed after the treatment of the hair with the peptide.

The measurements of mechanical resistance were performed using a cell with 2.5 N maximum load and a deformation rate of 1.5 mm/min. Each hair was individually mounted in the tensile jig by means of a paper template with a fixed gauge length of 20 mm.

As for the results obtained for the mechanical test showed that compared to the control, i.e., virgin hair without bleaching or peptide treatment (Young modulus: 4142 ± 590 MPa), bleaching reduced the Young modulus (2478± 567 MPa), while the treatment with the peptide sequence ID NO: 409 after bleaching increased the Young modulus to higher values than the virgin hair with no treatment (5649 ± 1022 MPa).

### Example 1:

This example discloses the treatment of human hair with a composition comprising peptides. In this example, the peptide with the sequence ID NO: 412 was tested. The hair was supplied from International Hair Importers and Products, Inc. (New York).

The formulation was tested in different hair types:
- virgin hair washed, with the cuticle intact and absence of chemical damages;
- hair after reduction treatment, at 37ºC in phosphate buffer at pH=8, with 3M GndHCl and 0.05M DTT for 2 hours.

For the treatment with the peptide SEQ ID NO: 412, concentrations of 0.01% (w/w) were used.

The average of the Young modulus for relaxed hair is 3002 MPa, while for relaxed hair fiber after peptide treatment at 0.01% is 4190 MPa. The Young modulus value for virgin hair without treatment is 5214 MPa.

In the maximum load test, for the relaxed hair fiber, the maximum of resistance were 96 MPa, while for the hair fiber relaxed after peptide treatment 126 MPa and for the virgin hair with no treatment 203 MPa.

Regarding hair stretching, the relaxed hair has an average of 51%, while after treatment with the peptide sequence ID NO: 412, has a stretching of 72%. For virgin hair, the average of hair stretching is 58%.

Therefore, it is evident that the peptides are capable to prevent the hair surface degradation and consequently, the hair treated with these peptides has a longer life span.

### Example 2:

In order to assess the interactions between the keratin and some peptides, a keratin solution was prepared. This procedure was performed by immersing African hair, acquired from the International Hair Importers and Products, Inc. (New York), in a solution containing 8 M urea, 0.2 M sodium dodecyl sulfate and 0.5 M sodium bisulfite. The mixture was heated to 50 °C for 24h in a shaker bath. The solution was dialyzed for several days against double-distilled water. The keratin solution was then concentrated using AMICON with a 3 kDa cut-off. The keratin was then conjugated with Alexa Fluor 647 carboxylic acid, succinimidyl ester in DMSO anhydrous 5%.

The reaction was incubated for 1h30min at room temperature and in the dark. The Alexa Fluor 647 that did not link to the keratin solution was separated by centrifugation in AMICON with a 3 kDa cut-off for 1h at 25ºC and 5000xg.

The keratin was then diluted to 1µg/mL in blocking buffer (3% BSA in tris-buffered saline (TBS) with 0.05% Tween 20). The peptides tested were SEQ.ID NO:179, SEQ.ID NO:75, SEQ.ID NO:432, SEQ.ID NO:951, SEQ.ID NO:1108, SEQ.ID NO:1131 and a peptide containing 13 amino acids, including 2 cysteines (X₃CX₅CX₃), where X represents one of known amino acid residues, with the exception of cysteine residue that is represented by the letter C. This peptide is similar to the one tested in Fernandes et al. (Fernandes, Lima, Loureiro, Gomes, & Cavaco-Paulo, 2012).

Several peptides in a concentration of 15fmol/mm², were attached to a glass through a hydrophilic linked moiety, and were then incubated with the keratin, marked with Alexa Fluor 647, for 2 hours at 37ºC.

After incubation, the glasses were rinsed in successive washing solutions: TBS + 0.1% Tween 20 and blocking buffer with 3%BSA in TBS + 0.1% Tween 20, for 3 minutes in each solution.

The imaging of the glasses was performed in Agilent G2565CA Microarray Scanner System. Three replicas of the each peptide incubation were performed and analyzed.

**Table 1 - Normalized intensity levels of peptide sequences. Therein SEQ ID NO's: 75 and 1131 are according to the present invention and the other SEQ ID NO's are reference examples.**

| Sequence | Number of amino acids | Cysteine content | Hydrophobic amino acids content | Intensity level (average ± standard deviation) |
|---|---|---|---|---|
| SEQ.ID NO:179 | 10 | 20% | 50% | 0.990 ± 0.014 |
| SEQ.ID NO:75 | 10 | 30% | 60% | 1.000 ± 0.000 |
| SEQ.ID NO:432 | 10 | 30% | 40% | 1.000 ± 0.000 |
| SEQ.ID NO:951 | 10 | 40% | 30% | 1.000 ± 0.000 |
| SEQ.ID NO:1108 | 11 | 46% | 18% | 1.000 ± 0.000 |
| SEQ.ID NO:1131 | 11 | 46% | 9% | 1.000 ± 0.000 |
| X₃CX₅CX₃ | 13 | 15% | 38% | 0.184 ± 0.084 |

The peptides SEQ.ID NO:75, SEQ.ID NO:432, SEQ.ID NO:951, SEQ.ID NO:1108, SEQ.ID NO:1131, with percentage of cysteine ranging from 30% to 46%, such as and percentage of hydrophobic amino acids ranging from 9% to 60% were able to obtain an intensity of 1, indicating a very high affinity to keratin. The peptide SEQ.ID NO:179, with 20% and 50% of cysteine and hydrophobic content, respectively showed a slightly inferior but still very high intensity (0.990±0.014). These peptides were compared with a peptide similar to the one described in Fernandes et al. (Fernandes, Lima, Loureiro, Gomes, & Cavaco-Paulo, 2012) containing 2 cysteines in a 13 amino acids sequence. The reduced percentage of cysteine (15%) and higher number of amino acids in the sequence (13 amino acids) lead to a decrease in the intensity to 0.184±0.084, showing an inferior affinity to keratin. This suggests that the higher number of amino acids difficult the reaction of the peptide with the hair keratins. This inferior affinity to keratin leads to less fixation of the peptides in the hair in posterior treatments and consequently providing less improvements in the recovery of the hair characteristics.

### List of peptide sequences

The peptides of SEQ ID NO's: 75, 412 and 1131 are according to the invention, the remaining sequences presented as reference examples.

The sequences of peptides are described by one letter code of amino acids. The code is as follows:
Amino acid - One Letter Code
Histidine - H
Arginine - R
Lysine - K
Isoleucine - I
Phenylalanine - F
Leucine - L
Tryptophan - W
Alanine- A
Methionine - M
Proline - P
Valine - V
Cysteine - C
Asparagine - N
Glycine - G
Serine - S
Glutamine - Q.
Tyrosine - Y
Threonine - T
Aspartic acid - D
Glutamic acid - E

| | |
|---|---|
| SEQ.ID NO:1 | APCAPRPSCG |
| SEQ.ID NO:2 | EACVPSVPCP |
| SEQ.ID NO:3 | ESCGTASGCA |
| SEQ.ID NO:4 | GLCAGTSACL |
| SEQ.ID NO:5 | GVCGPSPPCI |
| SEQ.ID NO:6 | HGCTLPGACN |
| SEQ.ID NO:7 | HSCTLPGACN |
| SEQ.ID NO:8 | KDCLQNSLCE |
| SEQ.ID NO:9 | LPCLPAASCG |
| SEQ.ID NO:10 | LPCYFTGSCN |
| SEQ.ID NO:11 | NFCLPSLSCR |
| SEQ.ID NO:12 | NPCATTNACD |
| SEQ.ID NO:13 | NPCATTNACE |
| SEQ.ID NO:14 | NPCATTNACS |
| SEQ.ID NO:15 | NPCGLRARCG |
| SEQ.ID NO:16 | NPCGPRSRCG |
| SEQ.ID NO:17 | NPCSTPASCT |
| SEQ.ID NO:18 | NPCSTSPSCV |
| SEQ.ID NO:19 | PACTSSSPCS |
| SEQ.ID NO:20 | SKCHESTVCP |
| SEQ.ID NO:21 | SPCVPRTVCV |
| SEQ.ID NO:22 | SSCSVETACL |
| SEQ.ID NO:23 | SVCSSGVNCR |
| SEQ.ID NO:24 | TACPLPGTCH |
| SEQ.ID NO:25 | TNCSPRPICV |
| SEQ.ID NO:26 | TSCVPPAPCT |
| SEQ.ID NO:27 | TTCTSSNTCE |
| SEQ.ID NO:28 | VPCVPSVPCT |
| SEQ.ID NO:29 | ATCGPSACIT |
| SEQ.ID NO:30 | GPCISNPCGL |
| SEQ.ID NO:31 | GPCLSNPCTS |
| SEQ.ID NO:32 | GSCVTNPCGP |
| SEQ.ID NO:33 | LTCFSITCSS |
| SEQ.ID NO:34 | NPCSTPSCTT |
| SEQ.ID NO:35 | PSCVTAPCAP |
| SEQ.ID NO:36 | SDCSSTHCSP |
| SEQ.ID NO:37 | SLCLPPTCHT |
| SEQ.ID NO:38 | SLCNLGSCGP |
| SEQ.ID NO:39 | SPCLVGNCAW |
| SEQ.ID NO:40 | TACLPGTCAT |
| SEQ.ID NO:41 | TSCLPALCLP |
| SEQ.ID NO:42 | TSCSSRPCVP |
| SEQ.ID NO:43 | TTCGGGSCGV |
| SEQ.ID NO:44 | VNCRPELCLG |
| SEQ.ID NO:45 | YVCQPMACLP |
| SEQ.ID NO:46 | AFSCISACGP |
| SEQ.ID NO:47 | GSVCSAPCNG |
| SEQ.ID NO:48 | GVVCGDLCAS |
| SEQ.ID NO:49 | GVVCGDLCVS |
| SEQ.ID NO:50 | LTGCLLPCYF |
| SEQ.ID NO:51 | NEDCKLPCNP |
| SEQ.ID NO:52 | NFSCVSACGP |
| SEQ.ID NO:53 | PPTCHTACPL |
| SEQ.ID NO:54 | PQPCATACKP |
| SEQ.ID NO:55 | SEDCKLPCNP |
| SEQ.ID NO:56 | SLGCRTSCSS |
| SEQ.ID NO:57 | SLSCRTSCSS |
| SEQ.ID NO:58 | SSSCPLGCTM |
| SEQ.ID NO:59 | TGSCNSPCLV |
| SEQ.ID NO:60 | TSSCPLGCTM |
| SEQ.ID NO:61 | VGSCGSSCRK |
| SEQ.ID NO:62 | VGVCGGSCKR |
| SEQ.ID NO:63 | VSNCNWFCEG |
| SEQ.ID NO:64 | ACGPRPGRCC |
| SEQ.ID NO:65 | ACGPRPSRCC |
| SEQ.ID NO:66 | CAPRPSCGPC |
| SEQ.ID NO:67 | CEPCSAYVIC |
| SEQ.ID NO:68 | CGLRARCGPC |
| SEQ.ID NO:69 | CGPRPGRCCI |
| SEQ.ID NO:70 | CGPRPSRCCI |
| SEQ.ID NO:71 | CGPRSRCGPC |
| SEQ.ID NO:72 | CGTSQKGCCN |
| SEQ.ID NO:73 | CHGCTLPGAC |
| SEQ.ID NO:74 | CHSCTLPGAC |
| SEQ.ID NO:75 | CLPCLPAASC |
| SEQ.ID NO:76 | CLPPTCHTAC |
| SEQ.ID NO:77 | CLSNPCTSCV |
| SEQ.ID NO:78 | CLVGNCAWCE |
| SEQ.ID NO:79 | CNPCSTPASC |
| SEQ.ID NO:80 | CNPCSTPSCT |
| SEQ.ID NO:81 | CNPCSTSPSC |
| SEQ.ID NO:82 | CNSPCLVGNC |
| SEQ.ID NO:83 | CRTSCSSRPC |
| SEQ.ID NO:84 | CSLKEHCSAC |
| SEQ.ID NO:85 | CSPRPICVPC |
| SEQ.ID NO:86 | CSSTMSYSCC |
| SEQ.ID NO:87 | CSTPASCTSC |
| SEQ.ID NO:88 | CSTPSCTTCV |
| SEQ.ID NO:89 | CTSCVPPAPC |
| SEQ.ID NO:90 | CTSSNTCEPC |
| SEQ.ID NO:91 | CVPPAPCTPC |
| SEQ.ID NO:92 | CVPPSCHGCT |
| SEQ.ID NO:93 | CVPPSCHSCT |
| SEQ.ID NO:94 | DCKLPCNPCA |
| SEQ.ID NO:95 | DCKLPCNPCS |
| SEQ.ID NO:96 | PCGTSQKGCC |
| SEQ.ID NO:97 | PCLSNPCTSC |
| SEQ.ID NO:98 | PCLVGNCAWC |
| SEQ.ID NO:99 | PCNPCSTPSC |
| SEQ.ID NO:100 | PCSTPSCTTC |
| SEQ.ID NO:101 | PCTTCGPTCG |
| SEQ.ID NO:102 | PCVPPSCHGC |
| SEQ.ID NO:103 | PCVPPSCHSC |
| SEQ.ID NO:104 | SCCLPSLGCR |
| SEQ.ID NO:105 | SCSEELQCCQ |
| SEQ.ID NO:106 | SCSPCSTTCT |
| SEQ.ID NO:107 | ASCSTSGTCG |
| SEQ.ID NO:108 | ASCYIPVGCQ |
| SEQ.ID NO:109 | ASCYVPVSCQ |
| SEQ.ID NO:110 | AVCTLPSSCQ |
| SEQ.ID NO:111 | DLCPTSVSCG |
| SEQ.ID NO:112 | EICWEPTSCQ |
| SEQ.ID NO:113 | ETCGEPTSCQ |
| SEQ.ID NO:114 | ETCNETTSCQ |
| SEQ.ID NO:115 | ETCWRPNSCQ |
| SEQ.ID NO:116 | GYCGYRPFCF |
| SEQ.ID NO:117 | KTCWEPASCQ |
| SEQ.ID NO:118 | KTCWEPTSCQ |
| SEQ.ID NO:119 | LDCVDTTPCK |
| SEQ.ID NO:120 | LGCGYGSFCG |
| SEQ.ID NO:121 | NSCGYGSGCG |
| SEQ.ID NO:122 | NYCPSNTMCE |
| SEQ.ID NO:123 | PACVTSYSCR |
| SEQ.ID NO:124 | PDCHVEGTCL |
| SEQ.ID NO:125 | PDCRVEGTCL |
| SEQ.ID NO:126 | PICSEPSPCS |
| SEQ.ID NO:127 | PICYIFKPCQ |
| SEQ.ID NO:128 | PLCYISNSCQ |
| SEQ.ID NO:129 | PPCGQPTPCS |
| SEQ.ID NO:130 | PPCHIPQPCV |
| SEQ.ID NO:131 | PSCGRLASCG |
| SEQ.ID NO:132 | PSCSESSICQ |
| SEQ.ID NO:133 | PSCSEVTSCP |
| SEQ.ID NO:134 | PSCSTSGTCG |
| SEQ.ID NO:135 | PSCSVSSGCQ |
| SEQ.ID NO:136 | PSCTESDSCK |
| SEQ.ID NO:137 | PSCYQTSSCG |
| SEQ.ID NO:138 | PTCFLLNSCQ |
| SEQ.ID NO:139 | PTCSVTSSCQ |
| SEQ.ID NO:140 | PTCWLLNNCH |
| SEQ.ID NO:141 | PTCYQRTSCV |
| SEQ.ID NO:142 | PTCYRRTSCV |
| SEQ.ID NO:143 | PTCYVVKRCP |
| SEQ.ID NO:144 | PVCFEATICE |
| SEQ.ID NO:145 | PVCFEATVCE |
| SEQ.ID NO:146 | PVCSRPASCS |
| SEQ.ID NO:147 | PVCSWVPACS |
| SEQ.ID NO:148 | QTCNESSYCL |
| SEQ.ID NO:149 | QTCWEPTSCQ |
| SEQ.ID NO:150 | SFCRLGYGCG |
| SEQ.ID NO:151 | SFCRRGSGCG |
| SEQ.ID NO:152 | SLCGYGYGCG |
| SEQ.ID NO:153 | SLCSTEVSCG |
| SEQ.ID NO:154 | SNCFGQLNCL |
| SEQ.ID NO:155 | SPCGQPTPCS |
| SEQ.ID NO:156 | SSCDQSSSCA |
| SEQ.ID NO:157 | SSCGQSSSCA |
| SEQ.ID NO:158 | SVCPEPVSCP |
| SEQ.ID NO:159 | TFCSFDKSCR |
| SEQ.ID NO:160 | TICSSDKSCR |
| SEQ.ID NO:161 | TLCVESSPCH |
| SEQ.ID NO:162 | TPCYQQSSCQ |
| SEQ.ID NO:163 | VTCSRQTTCI |
| SEQ.ID NO:164 | YGCGYGSGCG |
| SEQ.ID NO:165 | YGCGYGSGCR |
| SEQ.ID NO:166 | YGCIHSTHCG |
| SEQ.ID NO:167 | AACEPSACQS |
| SEQ.ID NO:168 | AACEPSPCQS |
| SEQ.ID NO:169 | AACTMSVCSS |
| SEQ.ID NO:170 | ADCLGGICLP |
| SEQ.ID NO:171 | ALCLPSSCHS |
| SEQ.ID NO:172 | ALCSPSTCQL |
| SEQ.ID NO:173 | APCLALVCAP |
| SEQ.ID NO:174 | APCLSLVCTP |
| SEQ.ID NO:175 | APCLTLVCTP |
| SEQ.ID NO:176 | APCVALLCRP |
| SEQ.ID NO:177 | ASCGSLLCRP |
| SEQ.ID NO:178 | ASCLSFLCRP |
| SEQ.ID NO:179 | ASCVSLLCRP |
| SEQ.ID NO:180 | AVCEPSPCQS |
| SEQ.ID NO:181 | AVCLPVSCQS |
| SEQ.ID NO:182 | AVCVPVRCQS |
| SEQ.ID NO:183 | AVCVPVSCQS |
| SEQ.ID NO:184 | DLCSPSTCQL |
| SEQ.ID NO:185 | DSCGSSSCGP |
| SEQ.ID NO:186 | DSCVQSNCFP |
| SEQ.ID NO:187 | FNCSTRNCSS |
| SEQ.ID NO:188 | GGCGSYGCSQ |
| SEQ.ID NO:189 | GSCGFGSCYG |
| SEQ.ID NO:190 | GSCSSRKCFS |
| SEQ.ID NO:191 | GVCLPSTCPH |
| SEQ.ID NO:192 | HSCEGYLCYS |
| SEQ.ID NO:193 | IVCAAPSCQS |
| SEQ.ID NO:194 | KTCSTTGCDP |
| SEQ.ID NO:195 | LACVSQPCQS |
| SEQ.ID NO:196 | LGCGYGGCGY |
| SEQ.ID NO:197 | LSCGSRSCSS |
| SEQ.ID NO:198 | LVCTPVSCVS |
| SEQ.ID NO:199 | NGCQETYCEP |
| SEQ.ID NO:200 | NSCRSLSCGS |
| SEQ.ID NO:201 | PACVISTCPR |
| SEQ.ID NO:202 | PGCLNQSCGS |
| SEQ.ID NO:203 | PPCGTAPCLT |
| SEQ.ID NO:204 | PPCTTALCRP |
| SEQ.ID NO:205 | PPCYLVSCTP |
| SEQ.ID NO:206 | PRCTRPICEP |
| SEQ.ID NO:207 | PSCPVSSCAQ |
| SEQ.ID NO:208 | PSCQPSVCVP |
| SEQ.ID NO:209 | PSCSVSNCYQ |
| SEQ.ID NO:210 | PSCSVSSCAQ |
| SEQ.ID NO:211 | PSCTSVLCRP |
| SEQ.ID NO:212 | PTCKSPSCEP |
| SEQ.ID NO:213 | PTCVISSCPR |
| SEQ.ID NO:214 | PTCVISTCPR |
| SEQ.ID NO:215 | PTCYQTICFR |
| SEQ.ID NO:216 | PVCGGVSCHT |
| SEQ.ID NO:217 | PVCGRVSCHT |
| SEQ.ID NO:218 | PVCNKPVCFV |
| SEQ.ID NO:219 | PVCPTPTCSV |
| SEQ.ID NO:220 | PVCRSTYCVP |
| SEQ.ID NO:221 | PVCSKSVCYV |
| SEQ.ID NO:222 | PVCSRPACYS |
| SEQ.ID NO:223 | PVCYVPTCSE |
| SEQ.ID NO:224 | QFCLSKSCQP |
| SEQ.ID NO:225 | RPCERTACQS |
| SEQ.ID NO:226 | RSCQTSFCGF |
| SEQ.ID NO:227 | RSCSSLGCGS |
| SEQ.ID NO:228 | RSCYSVGCGS |
| SEQ.ID NO:229 | RVCLPGSCDS |
| SEQ.ID NO:230 | SFCGFPSCST |
| SEQ.ID NO:231 | SFCGYPSCST |
| SEQ.ID NO:232 | SGCDPASCQP |
| SEQ.ID NO:233 | SGCGGSGCGG |
| SEQ.ID NO:234 | SGCQPSSCLA |
| SEQ.ID NO:235 | SHCQPPHCQL |
| SEQ.ID NO:236 | SICQPATCVA |
| SEQ.ID NO:237 | SLCVPVSCRP |
| SEQ.ID NO:238 | SNCLPTSCQP |
| SEQ.ID NO:239 | SPCLVSSCQP |
| SEQ.ID NO:240 | SPCQQSSCQE |
| SEQ.ID NO:241 | SPCQQSYCVP |
| SEQ.ID NO:242 | SPCSPAVCVS |
| SEQ.ID NO:243 | SRCQQPSCQP |
| SEQ.ID NO:244 | SRCYRPHCGQ |
| SEQ.ID NO:245 | SSCAPIYCRR |
| SEQ.ID NO:246 | SSCAPVYCRR |
| SEQ.ID NO:247 | SSCGKGGCGS |
| SEQ.ID NO:248 | SSCGKRGCGS |
| SEQ.ID NO:249 | SSCLPVSCRP |
| SEQ.ID NO:250 | SSCQPAYCTS |
| SEQ.ID NO:251 | SSCQPSYCRQ |
| SEQ.ID NO:252 | SSCQPVVCEP |
| SEQ.ID NO:253 | SSCTAVVCRP |
| SEQ.ID NO:254 | SSCYQPFCRS |
| SEQ.ID NO:255 | SSCYRPICGS |
| SEQ.ID NO:256 | SSCYRPTCGS |
| SEQ.ID NO:257 | SVCMSGSCQA |
| SEQ.ID NO:258 | SVCSDQGCDQ |
| SEQ.ID NO:259 | SVCSDQGCGL |
| SEQ.ID NO:260 | SVCSDQGCGQ |
| SEQ.ID NO:261 | SVCSDQGCSQ |
| SEQ.ID NO:262 | SVCSDQSCGQ |
| SEQ.ID NO:263 | SVCSHQGCGQ |
| SEQ.ID NO:264 | SVCSHQGCGR |
| SEQ.ID NO:265 | SVCVPVSCRP |
| SEQ.ID NO:266 | SYCRQASCVS |
| SEQ.ID NO:267 | TACEPSACQS |
| SEQ.ID NO:268 | TICTASPCQP |
| SEQ.ID NO:269 | TSCPETSCLP |
| SEQ.ID NO:270 | TSCQMTNCEQ |
| SEQ.ID NO:271 | TSCQPVHCET |
| SEQ.ID NO:272 | TSCQPVLCKS |
| SEQ.ID NO:273 | TSCQPVLCVP |
| SEQ.ID NO:274 | TSCVGFVCQP |
| SEQ.ID NO:275 | TSCVSNPCQV |
| SEQ.ID NO:276 | TTCFQPTCVS |
| SEQ.ID NO:277 | TTCFQPTCVT |
| SEQ.ID NO:278 | TTCFQPTCVY |
| SEQ.ID NO:279 | TTCISNPCST |
| SEQ.ID NO:280 | TWCQGSSCQP |
| SEQ.ID NO:281 | VGCQSSVCVP |
| SEQ.ID NO:282 | VPCQPSTCVF |
| SEQ.ID NO:283 | VSCEPSPCQS |
| SEQ.ID NO:284 | VSCGGPICLP |
| SEQ.ID NO:285 | VSCKPVLCVA |
| SEQ.ID NO:286 | VSCPSTSCRP |
| SEQ.ID NO:287 | VSCQSSVCMP |
| SEQ.ID NO:288 | VSCTRIVCVA |
| SEQ.ID NO:289 | VTCEPSPCQS |
| SEQ.ID NO:290 | VTCQTTVCRP |
| SEQ.ID NO:291 | YGCGYEGCRY |
| SEQ.ID NO:292 | AGSCQPSCSE |
| SEQ.ID NO:293 | ALLCRPLCGV |
| SEQ.ID NO:294 | ALVCEPVCLR |
| SEQ.ID NO:295 | ATICEPSCSV |
| SEQ.ID NO:296 | ATTCEPSCSV |
| SEQ.ID NO:297 | ATVCEPSCSV |
| SEQ.ID NO:298 | EGTCLPPCYL |
| SEQ.ID NO:299 | FSTCRPSCSG |
| SEQ.ID NO:300 | GFVCQPMCSH |
| SEQ.ID NO:301 | GLDCGYGCGY |
| SEQ.ID NO:302 | GLGCGYGCGY |
| SEQ.ID NO:303 | GLGCSYGCGH |
| SEQ.ID NO:304 | GLGCSYGCGL |
| SEQ.ID NO:305 | GSGCGYGCGY |
| SEQ.ID NO:306 | GTGCGYGCGY |
| SEQ.ID NO:307 | GVSCHTTCYR |
| SEQ.ID NO:308 | GYACNFPCSY |
| SEQ.ID NO:309 | GYGCGYGCGF |
| SEQ.ID NO:310 | HSPCQASCYV |
| SEQ.ID NO:311 | HTSCSPACQP |
| SEQ.ID NO:312 | HTSCSSGCQP |
| SEQ.ID NO:313 | IRWCHPDCHV |
| SEQ.ID NO:314 | IRWCRPDCRV |
| SEQ.ID NO:315 | ISSCGTGCGI |
| SEQ.ID NO:316 | KGGCGSGCGG |
| SEQ.ID NO:317 | KGGCGSSCSQ |
| SEQ.ID NO:318 | LVTCQDSCGS |
| SEQ.ID NO:319 | LVTCQESCQP |
| SEQ.ID NO:320 | MSICSSACTD |
| SEQ.ID NO:321 | MSICSSACTN |
| SEQ.ID NO:322 | MSVCSSACSD |
| SEQ.ID NO:323 | PAICEPSCSV |
| SEQ.ID NO:324 | PASCQKSCYR |
| SEQ.ID NO:325 | PIYCRRTCYH |
| SEQ.ID NO:326 | PNSCQTLCVE |
| SEQ.ID NO:327 | PQPCVPTCFL |
| SEQ.ID NO:328 | PSACQSGCTS |
| SEQ.ID NO:329 | PSPCEPSCSE |
| SEQ.ID NO:330 | PSPCQASCYI |
| SEQ.ID NO:331 | PSPCQSGCIS |
| SEQ.ID NO:332 | PSPCQSGCTD |
| SEQ.ID NO:333 | PSPCQSGCTS |
| SEQ.ID NO:334 | PTACQPTCYQ |
| SEQ.ID NO:335 | PTACQPTCYR |
| SEQ.ID NO:336 | PTPCSTTCRT |
| SEQ.ID NO:337 | PTSCQKSCYR |
| SEQ.ID NO:338 | PTSCQPSCES |
| SEQ.ID NO:339 | PTSCQTSCTL |
| SEQ.ID NO:340 | PVICEPSCSV |
| SEQ.ID NO:341 | PVSCVPVCSG |
| SEQ.ID NO:342 | PVTCVPRCTR |
| SEQ.ID NO:343 | PVYCRRTCYH |
| SEQ.ID NO:344 | PVYCRRTCYY |
| SEQ.ID NO:345 | PVYCVPVCSG |
| SEQ.ID NO:346 | QPGCESPCEP |
| SEQ.ID NO:347 | QQSCVSSCRR |
| SEQ.ID NO:348 | QTSCGSSCGQ |
| SEQ.ID NO:349 | QTTCHPSCGM |
| SEQ.ID NO:350 | QTTCRPSCGV |
| SEQ.ID NO:351 | RGGCGSGCGG |
| SEQ.ID NO:352 | RLACYSLCSG |
| SEQ.ID NO:353 | RPACYRPCYS |
| SEQ.ID NO:354 | RPFCFRRCYS |
| SEQ.ID NO:355 | RPICRPICSG |
| SEQ.ID NO:356 | RPLCYRRCYS |
| SEQ.ID NO:357 | RSPCQASCYV |
| SEQ.ID NO:358 | RVSCHTTCYR |
| SEQ.ID NO:359 | SAICRPTCPR |
| SEQ.ID NO:360 | SDSCKRDCKK |
| SEQ.ID NO:361 | SEGCGSGCGG |
| SEQ.ID NO:362 | SFLCRPACSR |
| SEQ.ID NO:363 | SGGCGSGCGG |
| SEQ.ID NO:364 | SGGCGSSCGG |
| SEQ.ID NO:365 | SGSCQAACGQ |
| SEQ.ID NO:366 | SLLCHPVCKS |
| SEQ.ID NO:367 | SLLCHPVCRS |
| SEQ.ID NO:368 | SLLCRPACSP |
| SEQ.ID NO:369 | SLLCRPACSR |
| SEQ.ID NO:370 | SLLCRPICRP |
| SEQ.ID NO:371 | SLLCRPMCSR |
| SEQ.ID NO:372 | SLLCRPTCSR |
| SEQ.ID NO:373 | SLLCRPVCQP |
| SEQ.ID NO:374 | SLLCRPVCRP |
| SEQ.ID NO:375 | SLLCRPVCRS |
| SEQ.ID NO:376 | SLLCRPVCSR |
| SEQ.ID NO:377 | SNPCQVTCSR |
| SEQ.ID NO:378 | SRGCGSGCGG |
| SEQ.ID NO:379 | SRSCQSPCYR |
| SEQ.ID NO:380 | SRSCQSSCYR |
| SEQ.ID NO:381 | SSGCGYGCGY |
| SEQ.ID NO:382 | SSGCPMACPG |
| SEQ.ID NO:383 | SSICQPICSE |
| SEQ.ID NO:384 | SSPCHTSCYY |
| SEQ.ID NO:385 | SSPCQPTCYV |
| SEQ.ID NO:386 | SSPCQQSCYV |
| SEQ.ID NO:387 | SSPCQTSCYR |
| SEQ.ID NO:388 | SSSCQQSCRV |
| SEQ.ID NO:389 | STVCQPACGV |
| SEQ.ID NO:390 | TDNCQETCGE |
| SEQ.ID NO:391 | TQPCYEPCLP |
| SEQ.ID NO:392 | TSSCGTGCGI |
| SEQ.ID NO:393 | TSSCQPSCGR |
| SEQ.ID NO:394 | TSSCTTPCYQ |
| SEQ.ID NO:395 | TSVCLPGCLN |
| SEQ.ID NO:396 | TTVCLPGCLN |
| SEQ.ID NO:397 | VANCQAPCST |
| SEQ.ID NO:398 | VDDCPESCWP |
| SEQ.ID NO:399 | VKRCPSVCPE |
| SEQ.ID NO:400 | VSSCQPSCSE |
| SEQ.ID NO:401 | YEGCRYGCGH |
| SEQ.ID NO:402 | YGRCRHGCHS |
| SEQ.ID NO:403 | YGYCRPSCYG |
| SEQ.ID NO:404 | YRDCQKTCWE |
| SEQ.ID NO:405 | YRGCQEICWE |
| SEQ.ID NO:406 | YRGCQETCWR |
| SEQ.ID NO:407 | YRGCQQTCWE |
| SEQ.ID NO:408 | YRSCRPSCYG |
| SEQ.ID NO:409 | GGVCGPSPPC |
| SEQ.ID NO:410 | GVCGPSPPCI |
| SEQ.ID NO:411 | VCGPSPPCIT |
| SEQ.ID NO:412 | CGPSPPCITT |
| SEQ.ID NO:413 | CAPIYCRRTC |
| SEQ.ID NO:414 | CAPSPCQASC |
| SEQ.ID NO:415 | CAPSPCQPAC |
| SEQ.ID NO:416 | CAPVYCRRTC |
| SEQ.ID NO:417 | CASSPCQQAC |
| SEQ.ID NO:418 | CASSSCQPAC |
| SEQ.ID NO:419 | CASSSCQQSC |
| SEQ.ID NO:420 | CCGNFSSHSC |
| SEQ.ID NO:421 | CCGYGGLGCG |
| SEQ.ID NO:422 | CCNYYGNSCG |
| SEQ.ID NO:423 | CCNYYRNSCG |
| SEQ.ID NO:424 | CCSRNFSSCS |
| SEQ.ID NO:425 | CDAGSCQPSC |
| SEQ.ID NO:426 | CDPCSLQEGC |
| SEQ.ID NO:427 | CDPSPCEPSC |
| SEQ.ID NO:428 | CDPVICEPSC |
| SEQ.ID NO:429 | CDQGLCQETC |
| SEQ.ID NO:430 | CEATTCEPSC |
| SEQ.ID NO:431 | CELPCGTPSC |
| SEQ.ID NO:432 | CEPAICEPSC |
| SEQ.ID NO:433 | CEPPCGTAPC |
| SEQ.ID NO:434 | CEPPCSAPSC |
| SEQ.ID NO:435 | CEPRSCASSC |
| SEQ.ID NO:436 | CEPSACQSGC |
| SEQ.ID NO:437 | CEPSCSVSNC |
| SEQ.ID NO:438 | CEPSCSVSSC |
| SEQ.ID NO:439 | CEPSPCQSGC |
| SEQ.ID NO:440 | CEPTACQPTC |
| SEQ.ID NO:441 | CEPTSCQTSC |
| SEQ.ID NO:442 | CEPVCLRPVC |
| SEQ.ID NO:443 | CETSSCQPRC |
| SEQ.ID NO:444 | CETTCFQPTC |
| SEQ.ID NO:445 | CFQPTCVSSC |
| SEQ.ID NO:446 | CFQPTCVTSC |
| SEQ.ID NO:447 | CFQPTCVYSC |
| SEQ.ID NO:448 | CGCGFRRLGC |
| SEQ.ID NO:449 | CGCGYRGLDC |
| SEQ.ID NO:450 | CGCNGYYGCY |
| SEQ.ID NO:451 | CGFGSCYGCG |
| SEQ.ID NO:452 | CGGSGCGGSC |
| SEQ.ID NO:453 | CGGSGSSCCV |
| SEQ.ID NO:454 | CGGVSCHTTC |
| SEQ.ID NO:455 | CGKGGCGSCG |
| SEQ.ID NO:456 | CGKRGCGSCG |
| SEQ.ID NO:457 | CGQDLCQETC |
| SEQ.ID NO:458 | CGQTSCGSSC |
| SEQ.ID NO:459 | CGQVLCQETC |
| SEQ.ID NO:460 | CGRDLCQETC |
| SEQ.ID NO:461 | CGRVSCHTTC |
| SEQ.ID NO:462 | CGSCGFGSCY |
| SEQ.ID NO:463 | CGSCGGSKGC |
| SEQ.ID NO:464 | CGSGCGVPVC |
| SEQ.ID NO:465 | CGSLLCRPTC |
| SEQ.ID NO:466 | CGSRCYVPVC |
| SEQ.ID NO:467 | CGSSSCGPQC |
| SEQ.ID NO:468 | CGSVCSDQGC |
| SEQ.ID NO:469 | CGSVCSDQSC |
| SEQ.ID NO:470 | CGSVCSHQGC |
| SEQ.ID NO:471 | CGSYGCSQCS |
| SEQ.ID NO:472 | CGVCLPSTCP |
| SEQ.ID NO:473 | CGYEGCRYGC |
| SEQ.ID NO:474 | CGYGCGYGCG |
| SEQ.ID NO:475 | CGYGGCGYGC |
| SEQ.ID NO:476 | CGYGSFCGCG |
| SEQ.ID NO:477 | CGYGSGCGCG |
| SEQ.ID NO:478 | CHPSCGMSSC |
| SEQ.ID NO:479 | CHPSCSISSC |
| SEQ.ID NO:480 | CHPTCYQTIC |
| SEQ.ID NO:481 | CHTSCSPACQ |
| SEQ.ID NO:482 | CHTSCSSGCQ |
| SEQ.ID NO:483 | CHTTCYRPAC |
| SEQ.ID NO:484 | CHTTCYRPTC |
| SEQ.ID NO:485 | CIHSPCQASC |
| SEQ.ID NO:486 | CIHSTHCGCN |
| SEQ.ID NO:487 | CIRSPCQASC |
| SEQ.ID NO:488 | CISSCYRPQC |
| SEQ.ID NO:489 | CISSPCQQSC |
| SEQ.ID NO:490 | CKPCSSQSSC |
| SEQ.ID NO:491 | CKPSCSQSSC |
| SEQ.ID NO:492 | CKPVCFKPIC |
| SEQ.ID NO:493 | CKPVCYVPTC |
| SEQ.ID NO:494 | CKPVSCVPVC |
| SEQ.ID NO:495 | CKPVYCVPVC |
| SEQ.ID NO:496 | CKTVYCKPIC |
| SEQ.ID NO:497 | CLNQSCGSNC |
| SEQ.ID NO:498 | CLNQSCGSSC |
| SEQ.ID NO:499 | CLPGCLNQSC |
| SEQ.ID NO:500 | CLPGSCDSCS |
| SEQ.ID NO:501 | CLPPCYLVSC |
| SEQ.ID NO:502 | CLPTSCQPSC |
| SEQ.ID NO:503 | CLSFLCRPAC |
| SEQ.ID NO:504 | CLVSSCQPSC |
| SEQ.ID NO:505 | CMPSPCQPAC |
| SEQ.ID NO:506 | CMSGSCQAAC |
| SEQ.ID NO:507 | CNESSYCLPC |
| SEQ.ID NO:508 | CPASCVSLLC |
| SEQ.ID NO:509 | CPMACPGSPC |
| SEQ.ID NO:510 | CPSSCTAVVC |
| SEQ.ID NO:511 | CPVTCEPSPC |
| SEQ.ID NO:512 | CQAACEPSAC |
| SEQ.ID NO:513 | CQAACEPSPC |
| SEQ.ID NO:514 | CQAACGQSVC |
| SEQ.ID NO:515 | CQAPCSTKNC |
| SEQ.ID NO:516 | CQAVCEPSPC |
| SEQ.ID NO:517 | CQDSCGSSSC |
| SEQ.ID NO:518 | CQHSSCQPTC |
| SEQ.ID NO:519 | CQISSCGTGC |
| SEQ.ID NO:520 | CQKSSCQPAC |
| SEQ.ID NO:521 | CQPMCSHAAC |
| SEQ.ID NO:522 | CQPPCTTALC |
| SEQ.ID NO:523 | CQPSCESSFC |
| SEQ.ID NO:524 | CQPSCSESTC |
| SEQ.ID NO:525 | CQPSCTSVLC |
| SEQ.ID NO:526 | CQPTCGGSSC |
| SEQ.ID NO:527 | CQPTCSRPSC |
| SEQ.ID NO:528 | CQPVCPTPTC |
| SEQ.ID NO:529 | CQPVLCKSSC |
| SEQ.ID NO:530 | CQPVVCEPSC |
| SEQ.ID NO:531 | CQQPSCQPAC |
| SEQ.ID NO:532 | CQQSCRVPVC |
| SEQ.ID NO:533 | CQQSCYVPVC |
| SEQ.ID NO:534 | CQQSGCQPAC |
| SEQ.ID NO:535 | CQQSSCHPAC |
| SEQ.ID NO:536 | CQQSSCKPAC |
| SEQ.ID NO:537 | CQQSSCQLAC |
| SEQ.ID NO:538 | CQQSSCQPAC |
| SEQ.ID NO:539 | CQQSSCQPTC |
| SEQ.ID NO:540 | CQQSSCQSAC |
| SEQ.ID NO:541 | CQQSSCVSCV |
| SEQ.ID NO:542 | CQQSYCVPVC |
| SEQ.ID NO:543 | CQSGCISSCT |
| SEQ.ID NO:544 | CQSGCTDSCT |
| SEQ.ID NO:545 | CQSGCTSSCT |
| SEQ.ID NO:546 | CQSSCYRPTC |
| SEQ.ID NO:547 | CQSVCYQPTC |
| SEQ.ID NO:548 | CQSVYCQPTC |
| SEQ.ID NO:549 | CQTACEPSAC |
| SEQ.ID NO:550 | CQTSSCGTGC |
| SEQ.ID NO:551 | CQTTCHPSCG |
| SEQ.ID NO:552 | CQTTCRPSCG |
| SEQ.ID NO:553 | CQTTCYRTTC |
| SEQ.ID NO:554 | CQTTRCRTTC |
| SEQ.ID NO:555 | CQVTCEPSPC |
| SEQ.ID NO:556 | CRNTSCQPTC |
| SEQ.ID NO:557 | CRPACSPLAC |
| SEQ.ID NO:558 | CRPACSRLAC |
| SEQ.ID NO:559 | CRPACSRPAC |
| SEQ.ID NO:560 | CRPMCSRPAC |
| SEQ.ID NO:561 | CRPSCGQTTC |
| SEQ.ID NO:562 | CRPSCGVSSC |
| SEQ.ID NO:563 | CRPSCSISSC |
| SEQ.ID NO:564 | CRPSCSQTTC |
| SEQ.ID NO:565 | CRPSYCGQSC |
| SEQ.ID NO:566 | CRPSYCISSC |
| SEQ.ID NO:567 | CRPSYCQTTC |
| SEQ.ID NO:568 | CRPTCSRLAC |
| SEQ.ID NO:569 | CRPTCSSGSC |
| SEQ.ID NO:570 | CRPTSCQNTC |
| SEQ.ID NO:571 | CRPVCRSTYC |
| SEQ.ID NO:572 | CRPVCSRPAC |
| SEQ.ID NO:573 | CRPVTCVPRC |
| SEQ.ID NO:574 | CRQSSCQPAC |
| SEQ.ID NO:575 | CRTTCFHPIC |
| SEQ.ID NO:576 | CRTTCFQPTC |
| SEQ.ID NO:577 | CRTTCYRPSC |
| SEQ.ID NO:578 | CRTTYCRPSC |
| SEQ.ID NO:579 | CRVTCEPSPC |
| SEQ.ID NO:580 | CRYGCGHRGC |
| SEQ.ID NO:581 | CSAPCVALLC |
| SEQ.ID NO:582 | CSDDSGSCCQ |
| SEQ.ID NO:583 | CSEDSSSCCQ |
| SEQ.ID NO:584 | CSEDSYSCCQ |
| SEQ.ID NO:585 | CSEGCGSGCG |
| SEQ.ID NO:586 | CSESSPSCCQ |
| SEQ.ID NO:587 | CSESSSSCCQ |
| SEQ.ID NO:588 | CSFDKSCRCG |
| SEQ.ID NO:589 | CSGASSLCCQ |
| SEQ.ID NO:590 | CSGASSPCCQ |
| SEQ.ID NO:591 | CSGASSSCCQ |
| SEQ.ID NO:592 | CSGASTSCCQ |
| SEQ.ID NO:593 | CSGGCGSGCG |
| SEQ.ID NO:594 | CSGGCGSSCG |
| SEQ.ID NO:595 | CSGISSSCCQ |
| SEQ.ID NO:596 | CSKDSSSCCQ |
| SEQ.ID NO:597 | CSKGACGSCG |
| SEQ.ID NO:598 | CSLSCGSRSC |
| SEQ.ID NO:599 | CSQDLCQETC |
| SEQ.ID NO:600 | CSRGCGSGCG |
| SEQ.ID NO:601 | CSRLSSACCG |
| SEQ.ID NO:602 | CSSCGKGGCG |
| SEQ.ID NO:603 | CSSCGKRGCG |
| SEQ.ID NO:604 | CSSDKSCRCG |
| SEQ.ID NO:605 | CSSGNFSSCC |
| SEQ.ID NO:606 | CSSSGCGSFC |
| SEQ.ID NO:607 | CSSSGCGSSC |
| SEQ.ID NO:608 | CSTPCYQPIC |
| SEQ.ID NO:609 | CSTTCRTSSC |
| SEQ.ID NO:610 | CSWVPACSCT |
| SEQ.ID NO:611 | CTFSPCQQAC |
| SEQ.ID NO:612 | CTMSVCSSAC |
| SEQ.ID NO:613 | CTRPICEPCR |
| SEQ.ID NO:614 | CTSSPCQHAC |
| SEQ.ID NO:615 | CTSSPCQQAC |
| SEQ.ID NO:616 | CTSSPCQQSC |
| SEQ.ID NO:617 | CTSSSCQQAC |
| SEQ.ID NO:618 | CVALLCRPLC |
| SEQ.ID NO:619 | CVALVCEPVC |
| SEQ.ID NO:620 | CVFSSCNTTC |
| SEQ.ID NO:621 | CVGFVCQPMC |
| SEQ.ID NO:622 | CVPRCTRPIC |
| SEQ.ID NO:623 | CVPSPCQVAC |
| SEQ.ID NO:624 | CVPSRCQASC |
| SEQ.ID NO:625 | CVPSSCQASC |
| SEQ.ID NO:626 | CVPVCNKPVC |
| SEQ.ID NO:627 | CVPVCSKSVC |
| SEQ.ID NO:628 | CVPVRCKPVC |
| SEQ.ID NO:629 | CVSLLCRPAC |
| SEQ.ID NO:630 | CVSLLCRPMC |
| SEQ.ID NO:631 | CVSLLCRPTC |
| SEQ.ID NO:632 | CVSLLCRPVC |
| SEQ.ID NO:633 | CVSNPCQVTC |
| SEQ.ID NO:634 | CVSRCYRPHC |
| SEQ.ID NO:635 | CVSSCFRPQC |
| SEQ.ID NO:636 | CVSSICQPIC |
| SEQ.ID NO:637 | CVSSPCQPTC |
| SEQ.ID NO:638 | CVVSCTPPSC |
| SEQ.ID NO:639 | CVVSCTPPTC |
| SEQ.ID NO:640 | CYCPKNSIFC |
| SEQ.ID NO:641 | CYEPCLPRGC |
| SEQ.ID NO:642 | CYRRCYSSCY |
| SEQ.ID NO:643 | GCCGYGGLGC |
| SEQ.ID NO:644 | GCGGCGSGCA |
| SEQ.ID NO:645 | GCGGCGSGCG |
| SEQ.ID NO:646 | GCGGCGSSCG |
| SEQ.ID NO:647 | GCGGCSSSCG |
| SEQ.ID NO:648 | GCGGSGSSCC |
| SEQ.ID NO:649 | GCGSGCAGCG |
| SEQ.ID NO:650 | GCGSGCGGCG |
| SEQ.ID NO:651 | GCGSGCGGCS |
| SEQ.ID NO:652 | GCGSSCGGCD |
| SEQ.ID NO:653 | GCGSSCGGCG |
| SEQ.ID NO:654 | GCGSSCSQCS |
| SEQ.ID NO:655 | GCGYSSSCCG |
| SEQ.ID NO:656 | GCKGGCGSCG |
| SEQ.ID NO:657 | GCSGCSGGCG |
| SEQ.ID NO:658 | ICSGASSLCC |
| SEQ.ID NO:659 | ICSGASSPCC |
| SEQ.ID NO:660 | MCCNYYGNSC |
| SEQ.ID NO:661 | MCCNYYRNSC |
| SEQ.ID NO:662 | MCYGYGCGCG |
| SEQ.ID NO:663 | NCCSRNFSSC |
| SEQ.ID NO:664 | PCSLQEGCCR |
| SEQ.ID NO:665 | PCSSQSSCCV |
| SEQ.ID NO:666 | SCCAPASSCQ |
| SEQ.ID NO:667 | SCCAPASTCQ |
| SEQ.ID NO:668 | SCCAPTSSCQ |
| SEQ.ID NO:669 | SCCGYRPLCY |
| SEQ.ID NO:670 | SCCVPASSCQ |
| SEQ.ID NO:671 | SCCVPTSSCQ |
| SEQ.ID NO:672 | SCGCSKGACG |
| SEQ.ID NO:673 | SCGGCDSSCG |
| SEQ.ID NO:674 | SCGGCGSGCG |
| SEQ.ID NO:675 | SCGGCGSSCG |
| SEQ.ID NO:676 | SCGGCKGGCG |
| SEQ.ID NO:677 | SCGGSKGCCG |
| SEQ.ID NO:678 | SCGSGCRGCG |
| SEQ.ID NO:679 | SCYGCGYGCI |
| SEQ.ID NO:680 | TCCVPVPSCG |
| SEQ.ID NO:681 | TCSDDSGSCC |
| SEQ.ID NO:682 | TCSEDSSSCC |
| SEQ.ID NO:683 | TCSEDSYSCC |
| SEQ.ID NO:684 | TCSESSPSCC |
| SEQ.ID NO:685 | TCSESSSSCC |
| SEQ.ID NO:686 | TCSKDSSSCC |
| SEQ.ID NO:687 | TCSRLSSACC |
| SEQ.ID NO:688 | VCCQPTPICD |
| SEQ.ID NO:689 | VCSEDSSSCC |
| SEQ.ID NO:690 | VCSGASSLCC |
| SEQ.ID NO:691 | VCSGASSPCC |
| SEQ.ID NO:692 | VCSGASSSCC |
| SEQ.ID NO:693 | VCSGASTSCC |
| SEQ.ID NO:694 | VCSGDSSCCQ |
| SEQ.ID NO:695 | VCSGISSSCC |
| SEQ.ID NO:696 | YCVPIPSCCA |
| SEQ.ID NO:697 | CASSCCTPSC |
| SEQ.ID NO:698 | CCDNCPPPCH |
| SEQ.ID NO:699 | CCEPCLPRGC |
| SEQ.ID NO:700 | CCGAASSCCR |
| SEQ.ID NO:701 | CCGCGGSGCG |
| SEQ.ID NO:702 | CCGPSSSCCQ |
| SEQ.ID NO:703 | CCGSGCGGCG |
| SEQ.ID NO:704 | CCKPYCSQCS |
| SEQ.ID NO:705 | CCMPVSSCCA |
| SEQ.ID NO:706 | CCNYYRNCCG |
| SEQ.ID NO:707 | CCPSCVVSSC |
| SEQ.ID NO:708 | CCPSYCVSSC |
| SEQ.ID NO:709 | CCQPICGSSC |
| SEQ.ID NO:710 | CCQPICVTSC |
| SEQ.ID NO:711 | CCQPTCLSSC |
| SEQ.ID NO:712 | CCQPTCLTSC |
| SEQ.ID NO:713 | CCQPTCVASC |
| SEQ.ID NO:714 | CCQPTCVTSC |
| SEQ.ID NO:715 | CCQPYCHPTC |
| SEQ.ID NO:716 | CCQQSSCVSC |
| SEQ.ID NO:717 | CCQSSCFKPC |
| SEQ.ID NO:718 | CCQSSCSKPC |
| SEQ.ID NO:719 | CCQSSCYKPC |
| SEQ.ID NO:720 | CCQTICRSTC |
| SEQ.ID NO:721 | CCQTTCHPSC |
| SEQ.ID NO:722 | CCQTTCRPSC |
| SEQ.ID NO:723 | CCRVPTCSCS |
| SEQ.ID NO:724 | CCSPGCQPTC |
| SEQ.ID NO:725 | CCSSGCGSSC |
| SEQ.ID NO:726 | CCSSSCGSCG |
| SEQ.ID NO:727 | CCTQEQNCCE |
| SEQ.ID NO:728 | CCVPIPSCCA |
| SEQ.ID NO:729 | CCVPISSCCA |
| SEQ.ID NO:730 | CCVPVCYQCK |
| SEQ.ID NO:731 | CCVPVPSCCA |
| SEQ.ID NO:732 | CCVPVPSCCV |
| SEQ.ID NO:733 | CCVPVSSCCA |
| SEQ.ID NO:734 | CDSSCCQPSC |
| SEQ.ID NO:735 | CDTCPPPCCK |
| SEQ.ID NO:736 | CEPCRRPVCC |
| SEQ.ID NO:737 | CEPSCCQPVC |
| SEQ.ID NO:738 | CEPSCCSAVC |
| SEQ.ID NO:739 | CETSCCQPSC |
| SEQ.ID NO:740 | CETTCCRTTC |
| SEQ.ID NO:741 | CFSGCGSSCC |
| SEQ.ID NO:742 | CGCSQSNCCK |
| SEQ.ID NO:743 | CGCSQSSCCK |
| SEQ.ID NO:744 | CGGCGGCGGC |
| SEQ.ID NO:745 | CGGCGGGCCG |
| SEQ.ID NO:746 | CGGCGSGCCV |
| SEQ.ID NO:747 | CGGCGSSCCV |
| SEQ.ID NO:748 | CGGGCCGSSC |
| SEQ.ID NO:749 | CGGSCCGSSC |
| SEQ.ID NO:750 | CGQSCCRPAC |
| SEQ.ID NO:751 | CGQSCCRPVC |
| SEQ.ID NO:752 | CGSCGCSQCN |
| SEQ.ID NO:753 | CGSCGCSQCS |
| SEQ.ID NO:754 | CGSFCCQSSC |
| SEQ.ID NO:755 | CGSGCCVPVC |
| SEQ.ID NO:756 | CGSSCCGSGC |
| SEQ.ID NO:757 | CGSSCCQPCY |
| SEQ.ID NO:758 | CGSSCCQPIC |
| SEQ.ID NO:759 | CGSSCCQPSC |
| SEQ.ID NO:760 | CGSSCCQSSC |
| SEQ.ID NO:761 | CGSSCCVPIC |
| SEQ.ID NO:762 | CGSSCCVPVC |
| SEQ.ID NO:763 | CGSSCSQCSC |
| SEQ.ID NO:764 | CGYGSCCGCG |
| SEQ.ID NO:765 | CHPRCCISSC |
| SEQ.ID NO:766 | CHPSCCESSC |
| SEQ.ID NO:767 | CHPSCCISSC |
| SEQ.ID NO:768 | CHPTCCQNTC |
| SEQ.ID NO:769 | CHPTCCQTIC |
| SEQ.ID NO:770 | CHPVCCQTTC |
| SEQ.ID NO:771 | CHPVCKSTCC |
| SEQ.ID NO:772 | CHPVCRSTCC |
| SEQ.ID NO:773 | CISSCCHPSC |
| SEQ.ID NO:774 | CISSCCKPSC |
| SEQ.ID NO:775 | CISSCCRPSC |
| SEQ.ID NO:776 | CISSCTPSCC |
| SEQ.ID NO:777 | CISSSCCPSC |
| SEQ.ID NO:778 | CKAVCCVPTC |
| SEQ.ID NO:779 | CKPCCSQASC |
| SEQ.ID NO:780 | CKPCCSQSRC |
| SEQ.ID NO:781 | CKPCCSQSSC |
| SEQ.ID NO:782 | CKPCCSSSGC |
| SEQ.ID NO:783 | CKPCSCFSGC |
| SEQ.ID NO:784 | CKPCSCSSGC |
| SEQ.ID NO:785 | CKPCYCSSGC |
| SEQ.ID NO:786 | CKPICCVPVC |
| SEQ.ID NO:787 | CKPQCCQSVC |
| SEQ.ID NO:788 | CKPSCCQTTC |
| SEQ.ID NO:789 | CKPVCCAPTC |
| SEQ.ID NO:790 | CKPVCCKPIC |
| SEQ.ID NO:791 | CKPVCCKSIC |
| SEQ.ID NO:792 | CKPVCCLPTC |
| SEQ.ID NO:793 | CKPVCCVPTC |
| SEQ.ID NO:794 | CKPVCCVPVC |
| SEQ.ID NO:795 | CKPVCCVSTC |
| SEQ.ID NO:796 | CKPYCCQSSC |
| SEQ.ID NO:797 | CKPYCSQCSC |
| SEQ.ID NO:798 | CKSNCCKPVC |
| SEQ.ID NO:799 | CKTVCCKPVC |
| SEQ.ID NO:800 | CLPPCCVVSC |
| SEQ.ID NO:801 | CLTSCCQPSC |
| SEQ.ID NO:802 | CNPCCSQSSC |
| SEQ.ID NO:803 | CPESCCELPC |
| SEQ.ID NO:804 | CPESCCEPHC |
| SEQ.ID NO:805 | CPESCCEPPC |
| SEQ.ID NO:806 | CPFSCPTTCC |
| SEQ.ID NO:807 | CPGDCFTCCT |
| SEQ.ID NO:808 | CPSCVVSSCC |
| SEQ.ID NO:809 | CPSYCVSSCC |
| SEQ.ID NO:810 | CPTTCCRTTC |
| SEQ.ID NO:811 | CQETCCRPSC |
| SEQ.ID NO:812 | CQHACCVPVC |
| SEQ.ID NO:813 | CQNTCCRTTC |
| SEQ.ID NO:814 | CQPACCQPTC |
| SEQ.ID NO:815 | CQPACCTASC |
| SEQ.ID NO:816 | CQPACCTSSC |
| SEQ.ID NO:817 | CQPACCTTSC |
| SEQ.ID NO:818 | CQPACCVPVC |
| SEQ.ID NO:819 | CQPACCVSSC |
| SEQ.ID NO:820 | CQPCCHPTCY |
| SEQ.ID NO:821 | CQPCCRPTSC |
| SEQ.ID NO:822 | CQPICCGSSC |
| SEQ.ID NO:823 | CQPICGSSCC |
| SEQ.ID NO:824 | CQPICVTSCC |
| SEQ.ID NO:825 | CQPNCCRPSC |
| SEQ.ID NO:826 | CQPRCCETSC |
| SEQ.ID NO:827 | CQPSCCRPAC |
| SEQ.ID NO:828 | CQPSCCSTPC |
| SEQ.ID NO:829 | CQPSCCSTTC |
| SEQ.ID NO:830 | CQPSCCVPSC |
| SEQ.ID NO:831 | CQPSCCVSSC |
| SEQ.ID NO:832 | CQPTCCGSSC |
| SEQ.ID NO:833 | CQPTCCHPSC |
| SEQ.ID NO:834 | CQPTCCQPTC |
| SEQ.ID NO:835 | CQPTCCRPRC |
| SEQ.ID NO:836 | CQPTCCRPSC |
| SEQ.ID NO:837 | CQPTCCRTTC |
| SEQ.ID NO:838 | CQPTCLSSCC |
| SEQ.ID NO:839 | CQPTCLTSCC |
| SEQ.ID NO:840 | CQPTCVASCC |
| SEQ.ID NO:841 | CQPTCVTSCC |
| SEQ.ID NO:842 | CQPVCCQPTC |
| SEQ.ID NO:843 | CQPYCHPTCC |
| SEQ.ID NO:844 | CQQACCMPVC |
| SEQ.ID NO:845 | CQQACCVPIC |
| SEQ.ID NO:846 | CQQACCVPVC |
| SEQ.ID NO:847 | CQQSCCVPVC |
| SEQ.ID NO:848 | CQQSCCVSVC |
| SEQ.ID NO:849 | CQSMCCQPTC |
| SEQ.ID NO:850 | CQSNCCVPVC |
| SEQ.ID NO:851 | CQSSCCKPCS |
| SEQ.ID NO:852 | CQSSCCQSSC |
| SEQ.ID NO:853 | CQSSCCVPVC |
| SEQ.ID NO:854 | CQSSCFKPCC |
| SEQ.ID NO:855 | CQSSCSKPCC |
| SEQ.ID NO:856 | CQSVCCQPTC |
| SEQ.ID NO:857 | CQTICRSTCC |
| SEQ.ID NO:858 | CQTTCCRPSC |
| SEQ.ID NO:859 | CQTTCCRTTC |
| SEQ.ID NO:860 | CRATCCRPSC |
| SEQ.ID NO:861 | CRGCGPSCCA |
| SEQ.ID NO:862 | CRPACCETTC |
| SEQ.ID NO:863 | CRPACCQNTC |
| SEQ.ID NO:864 | CRPCCWATTC |
| SEQ.ID NO:865 | CRPICRPACC |
| SEQ.ID NO:866 | CRPLCCQTTC |
| SEQ.ID NO:867 | CRPQCCQSVC |
| SEQ.ID NO:868 | CRPQCCQTTC |
| SEQ.ID NO:869 | CRPRCCISSC |
| SEQ.ID NO:870 | CRPSCCESSC |
| SEQ.ID NO:871 | CRPSCCETTC |
| SEQ.ID NO:872 | CRPSCCISSC |
| SEQ.ID NO:873 | CRPSCCKPQC |
| SEQ.ID NO:874 | CRPSCCMSSC |
| SEQ.ID NO:875 | CRPSCCQTTC |
| SEQ.ID NO:876 | CRPSCCRPSC |
| SEQ.ID NO:877 | CRPSCCVSRC |
| SEQ.ID NO:878 | CRPSCCVSSC |
| SEQ.ID NO:879 | CRPTCCETTC |
| SEQ.ID NO:880 | CRPTCCQNTC |
| SEQ.ID NO:881 | CRPTCCQTTC |
| SEQ.ID NO:882 | CRPVCCDPCS |
| SEQ.ID NO:883 | CRPVCCQTTC |
| SEQ.ID NO:884 | CRPVCQPACC |
| SEQ.ID NO:885 | CRPVCRPACC |
| SEQ.ID NO:886 | CRPVCRPTCC |
| SEQ.ID NO:887 | CRPVCRSTCC |
| SEQ.ID NO:888 | CRPYCCESSC |
| SEQ.ID NO:889 | CRRPVCCDPC |
| SEQ.ID NO:890 | CRSQCCQSVC |
| SEQ.ID NO:891 | CRTTCCHPSC |
| SEQ.ID NO:892 | CRTTCCQPIC |
| SEQ.ID NO:893 | CRTTCCQPTC |
| SEQ.ID NO:894 | CRTTCCRPSC |
| SEQ.ID NO:895 | CRTTCCRTTC |
| SEQ.ID NO:896 | CSCSSCGSCA |
| SEQ.ID NO:897 | CSCSSCGSCG |
| SEQ.ID NO:898 | CSCTSCGSCG |
| SEQ.ID NO:899 | CSPACQPTCC |
| SEQ.ID NO:900 | CSPGCQPTCC |
| SEQ.ID NO:901 | CSPSCCQTTC |
| SEQ.ID NO:902 | CSQCSCYKPC |
| SEQ.ID NO:903 | CSQSNCCKPC |
| SEQ.ID NO:904 | CSQSSCCKPC |
| SEQ.ID NO:905 | CSSGCGSCCQ |
| SEQ.ID NO:906 | CSSGCGSSCC |
| SEQ.ID NO:907 | CSSGCQPACC |
| SEQ.ID NO:908 | CSSSCCQPSC |
| SEQ.ID NO:909 | CSTPCCQPTC |
| SEQ.ID NO:910 | CSTTCCQPIC |
| SEQ.ID NO:911 | CTAVVCRPCC |
| SEQ.ID NO:912 | CTDSCTPSCC |
| SEQ.ID NO:913 | CTPSCCQPAC |
| SEQ.ID NO:914 | CTRPICEPCC |
| SEQ.ID NO:915 | CTSSCTPSCC |
| SEQ.ID NO:916 | CVPACSCSSC |
| SEQ.ID NO:917 | CVPACSCTSC |
| SEQ.ID NO:918 | CVPVCCKPVC |
| SEQ.ID NO:919 | CVPVCCVPTC |
| SEQ.ID NO:920 | CVPVCCVPVC |
| SEQ.ID NO:921 | CVSCVSSPCC |
| SEQ.ID NO:922 | CVSRCCRPQC |
| SEQ.ID NO:923 | CVSSCCKPQC |
| SEQ.ID NO:924 | CVSSCCQHSC |
| SEQ.ID NO:925 | CVSSCCQPFC |
| SEQ.ID NO:926 | CVSSCCQPSC |
| SEQ.ID NO:927 | CVSSCCRPQC |
| SEQ.ID NO:928 | CVSTCCRPTC |
| SEQ.ID NO:929 | CVTRCCSTPC |
| SEQ.ID NO:930 | CVTSCCQPAC |
| SEQ.ID NO:931 | CVTSCCQPSC |
| SEQ.ID NO:932 | CVYSCCQPFC |
| SEQ.ID NO:933 | CVYSCCQPSC |
| SEQ.ID NO:934 | GCCGCSEGCG |
| SEQ.ID NO:935 | GCCGCSGGCG |
| SEQ.ID NO:936 | GCCGCSRGCG |
| SEQ.ID NO:937 | GCCRPITCCP |
| SEQ.ID NO:938 | GCGSSCCQCS |
| SEQ.ID NO:939 | GCGVPVCCCS |
| SEQ.ID NO:940 | LCCPCQTTCS |
| SEQ.ID NO:941 | PCCCLRPVCG |
| SEQ.ID NO:942 | PCCCRPVTCQ |
| SEQ.ID NO:943 | PCCCVRPVCG |
| SEQ.ID NO:944 | PCCSQASCCV |
| SEQ.ID NO:945 | PCCSQSRCCV |
| SEQ.ID NO:946 | PCCSQSSCCK |
| SEQ.ID NO:947 | PCCSQSSCCV |
| SEQ.ID NO:948 | PCCWATTCCQ |
| SEQ.ID NO:949 | QCSCCKPYCS |
| SEQ.ID NO:950 | RCYVPVCCCK |
| SEQ.ID NO:951 | SCCAPVYCCK |
| SEQ.ID NO:952 | SCCISSSCCP |
| SEQ.ID NO:953 | SCCVSSCRCP |
| SEQ.ID NO:954 | SCGCSQCSCY |
| SEQ.ID NO:955 | SCGLENCCCP |
| SEQ.ID NO:956 | VCCGASSCCQ |
| SEQ.ID NO:957 | VCCGDSSCCQ |
| SEQ.ID NO:958 | CASSCCTPSCC |
| SEQ.ID NO:959 | CCCPSCVVSSC |
| SEQ.ID NO:960 | CCCPSYCVSSC |
| SEQ.ID NO:961 | CCCSSGCGSSC |
| SEQ.ID NO:962 | CCDTCPPPCCK |
| SEQ.ID NO:963 | CCEPHCCALSC |
| SEQ.ID NO:964 | CCEPPCCAPSC |
| SEQ.ID NO:965 | CCEPPCCATSC |
| SEQ.ID NO:966 | CCETSCCQPSC |
| SEQ.ID NO:967 | CCGSSCCGSGC |
| SEQ.ID NO:968 | CCGSSCCGSSC |
| SEQ.ID NO:969 | CCHPRCCISSC |
| SEQ.ID NO:970 | CCHPSCCESSC |
| SEQ.ID NO:971 | CCHPSCCISSC |
| SEQ.ID NO:972 | CCHPSCCVSSC |
| SEQ.ID NO:973 | CCHPTCCQNTC |
| SEQ.ID NO:974 | CCHPTCCQTIC |
| SEQ.ID NO:975 | CCISSCCKPSC |
| SEQ.ID NO:976 | CCISSCCRPSC |
| SEQ.ID NO:977 | CCISSSCCPSC |
| SEQ.ID NO:978 | CCKAVCCVPTC |
| SEQ.ID NO:979 | CCKPCCSQASC |
| SEQ.ID NO:980 | CCKPCCSQSRC |
| SEQ.ID NO:981 | CCKPCCSQSSC |
| SEQ.ID NO:982 | CCKPCCSSSGC |
| SEQ.ID NO:983 | CCKPCSCFSGC |
| SEQ.ID NO:984 | CCKPCSCSSGC |
| SEQ.ID NO:985 | CCKPCYCSSGC |
| SEQ.ID NO:986 | CCKPICCVPVC |
| SEQ.ID NO:987 | CCKPQCCQSVC |
| SEQ.ID NO:988 | CCKPVCCKPIC |
| SEQ.ID NO:989 | CCKPYCCQSSC |
| SEQ.ID NO:990 | CCKPYCSQCSC |
| SEQ.ID NO:991 | CCMPVCCKPVC |
| SEQ.ID NO:992 | CCMPVCCKTVC |
| SEQ.ID NO:993 | CCMSSCCKPQC |
| SEQ.ID NO:994 | CCNPCCSQSSC |
| SEQ.ID NO:995 | CCPGDCFTCCT |
| SEQ.ID NO:996 | CCPSCVVSSCC |
| SEQ.ID NO:997 | CCPSYCVSSCC |
| SEQ.ID NO:998 | CCQNTCCRTTC |
| SEQ.ID NO:999 | CCQPACCVSSC |
| SEQ.ID NO:1000 | CCQPCCHPTCY |
| SEQ.ID NO:1001 | CCQPCCRPTSC |
| SEQ.ID NO:1002 | CCQPICGSSCC |
| SEQ.ID NO:1003 | CCQPICVTSCC |
| SEQ.ID NO:1004 | CCQPNCCRPSC |
| SEQ.ID NO:1005 | CCQPSCCETSC |
| SEQ.ID NO:1006 | CCQPSCCRPAC |
| SEQ.ID NO:1007 | CCQPSCCSTPC |
| SEQ.ID NO:1008 | CCQPSCCSTTC |
| SEQ.ID NO:1009 | CCQPSCCVPSC |
| SEQ.ID NO:1010 | CCQPSCCVSSC |
| SEQ.ID NO:1011 | CCQPTCCHPSC |
| SEQ.ID NO:1012 | CCQPTCCQPTC |
| SEQ.ID NO:1013 | CCQPTCCRPRC |
| SEQ.ID NO:1014 | CCQPTCCRPSC |
| SEQ.ID NO:1015 | CCQPTCCRPTC |
| SEQ.ID NO:1016 | CCQPTCCRTTC |
| SEQ.ID NO:1017 | CCQPTCLSSCC |
| SEQ.ID NO:1018 | CCQPTCLTSCC |
| SEQ.ID NO:1019 | CCQPTCVASCC |
| SEQ.ID NO:1020 | CCQPTCVTSCC |
| SEQ.ID NO:1021 | CCQPYCHPTCC |
| SEQ.ID NO:1022 | CCQSMCCQPTC |
| SEQ.ID NO:1023 | CCQSNCCVPVC |
| SEQ.ID NO:1024 | CCQSSCCKPCS |
| SEQ.ID NO:1025 | CCQSSCCKPSC |
| SEQ.ID NO:1026 | CCQSSCCKPYC |
| SEQ.ID NO:1027 | CCQSSCCQSSC |
| SEQ.ID NO:1028 | CCQSSCCVPVC |
| SEQ.ID NO:1029 | CCQSSCFKPCC |
| SEQ.ID NO:1030 | CCQSSCSKPCC |
| SEQ.ID NO:1031 | CCQSSCYKPCC |
| SEQ.ID NO:1032 | CCQSVCCQPTC |
| SEQ.ID NO:1033 | CCQTICRSTCC |
| SEQ.ID NO:1034 | CCQTTCCRPSC |
| SEQ.ID NO:1035 | CCQTTCCRTTC |
| SEQ.ID NO:1036 | CCRPACCETTC |
| SEQ.ID NO:1037 | CCRPACCQNTC |
| SEQ.ID NO:1038 | CCRPLCCQTTC |
| SEQ.ID NO:1039 | CCRPQCCQSVC |
| SEQ.ID NO:1040 | CCRPQCCQTTC |
| SEQ.ID NO:1041 | CCRPSCCESSC |
| SEQ.ID NO:1042 | CCRPSCCETTC |
| SEQ.ID NO:1043 | CCRPSCCGSSC |
| SEQ.ID NO:1044 | CCRPSCCISSC |
| SEQ.ID NO:1045 | CCRPSCCKPQC |
| SEQ.ID NO:1046 | CCRPSCCQTTC |
| SEQ.ID NO:1047 | CCRPSCCVSRC |
| SEQ.ID NO:1048 | CCRPSCCVSSC |
| SEQ.ID NO:1049 | CCRPTCCQNTC |
| SEQ.ID NO:1050 | CCRPTCCQTTC |
| SEQ.ID NO:1051 | CCRPVCCDPCS |
| SEQ.ID NO:1052 | CCRTTCCQPTC |
| SEQ.ID NO:1053 | CCRTTCCRPSC |
| SEQ.ID NO:1054 | CCRTTCCRTTC |
| SEQ.ID NO:1055 | CCSCSSCGSCA |
| SEQ.ID NO:1056 | CCSPGCQPTCC |
| SEQ.ID NO:1057 | CCSQSSCCKPC |
| SEQ.ID NO:1058 | CCSSGCGSCCQ |
| SEQ.ID NO:1059 | CCSSGCGSSCC |
| SEQ.ID NO:1060 | CCSTPCCQPTC |
| SEQ.ID NO:1061 | CCVPACSCSSC |
| SEQ.ID NO:1062 | CCVPACSCTSC |
| SEQ.ID NO:1063 | CCVPICCKPIC |
| SEQ.ID NO:1064 | CCVPICCKPVC |
| SEQ.ID NO:1065 | CCVPVCCKPIC |
| SEQ.ID NO:1066 | CCVPVCCKPVC |
| SEQ.ID NO:1067 | CCVPVCCKSNC |
| SEQ.ID NO:1068 | CCVPVCCKTVC |
| SEQ.ID NO:1069 | CCVPVCCSSSC |
| SEQ.ID NO:1070 | CCVPVCCVPVC |
| SEQ.ID NO:1071 | CCVSSCCKPQC |
| SEQ.ID NO:1072 | CCVSSCCQHSC |
| SEQ.ID NO:1073 | CCVSSCCQPSC |
| SEQ.ID NO:1074 | CCVSSCCRPQC |
| SEQ.ID NO:1075 | CCVSTCCRPTC |
| SEQ.ID NO:1076 | CCVSVCCKPVC |
| SEQ.ID NO:1077 | CDSSCCQPSCC |
| SEQ.ID NO:1078 | CEPCCRPVCCD |
| SEQ.ID NO:1079 | CFKPCCCQSSC |
| SEQ.ID NO:1080 | CGDGCCCPSCY |
| SEQ.ID NO:1081 | CGGGCCGSSCC |
| SEQ.ID NO:1082 | CGGSCCGSSCC |
| SEQ.ID NO:1083 | CGLENCCCPSC |
| SEQ.ID NO:1084 | CGQSCCRPACC |
| SEQ.ID NO:1085 | CGQSCCRPVCC |
| SEQ.ID NO:1086 | CGSCCQSSCCN |
| SEQ.ID NO:1087 | CGSCGCSQCNC |
| SEQ.ID NO:1088 | CGSCGCSQCSC |
| SEQ.ID NO:1089 | CGSGCCGPVCC |
| SEQ.ID NO:1090 | CGSGCCVPVCC |
| SEQ.ID NO:1091 | CGSNCCQPCCR |
| SEQ.ID NO:1092 | CGSSCCQPCCH |
| SEQ.ID NO:1093 | CGSSCCQPCCR |
| SEQ.ID NO:1094 | CGSSCCQPCYC |
| SEQ.ID NO:1095 | CGSSCCQPSCC |
| SEQ.ID NO:1096 | CGSSCCQSSCC |
| SEQ.ID NO:1097 | CGSSCCVPICC |
| SEQ.ID NO:1098 | CGSSCCVPVCC |
| SEQ.ID NO:1099 | CGSSCSQCSCC |
| SEQ.ID NO:1100 | CGVPVCCCSCS |
| SEQ.ID NO:1101 | CHPRCCISSCC |
| SEQ.ID NO:1102 | CHPSCCESSCC |
| SEQ.ID NO:1103 | CHPSCCISSCC |
| SEQ.ID NO:1104 | CHPTCCQNTCC |
| SEQ.ID NO:1105 | CISSCCHPSCC |
| SEQ.ID NO:1106 | CISSCCKPSCC |
| SEQ.ID NO:1107 | CISSCCRPSCC |
| SEQ.ID NO:1108 | CISSSCCPSCC |
| SEQ.ID NO:1109 | CKPCCCSSGCG |
| SEQ.ID NO:1110 | CKPCCSQASCC |
| SEQ.ID NO:1111 | CKPCCSQSRCC |
| SEQ.ID NO:1112 | CKPCCSQSSCC |
| SEQ.ID NO:1113 | CKPQCCQSMCC |
| SEQ.ID NO:1114 | CKPQCCQSVCC |
| SEQ.ID NO:1115 | CKPVCCCVPAC |
| SEQ.ID NO:1116 | CKPVCCKPICC |
| SEQ.ID NO:1117 | CKPVCCMPVCC |
| SEQ.ID NO:1118 | CKPVCCVPVCC |
| SEQ.ID NO:1119 | CKPVCCVSVCC |
| SEQ.ID NO:1120 | CKPYCSQCSCC |
| SEQ.ID NO:1121 | CLPCCRPTCCQ |
| SEQ.ID NO:1122 | CLTSCCQPSCC |
| SEQ.ID NO:1123 | CMSSCCKPQCC |
| SEQ.ID NO:1124 | CNPCCSQSSCC |
| SEQ.ID NO:1125 | CPACCVSSCCQ |
| SEQ.ID NO:1126 | CPESCCEPHCC |
| SEQ.ID NO:1127 | CPESCCEPPCC |
| SEQ.ID NO:1128 | CPSCCESSCCR |
| SEQ.ID NO:1129 | CPSCCQTTCCR |
| SEQ.ID NO:1130 | CPSCCVSSCCR |
| SEQ.ID NO:1131 | CQCSCCKPYCS |
| SEQ.ID NO:1132 | CQETCCRPSCC |
| SEQ.ID NO:1133 | CQNTCCRTTCC |
| SEQ.ID NO:1134 | CQPACCTASCC |
| SEQ.ID NO:1135 | CQPACCTSSCC |
| SEQ.ID NO:1136 | CQPACCTTSCC |
| SEQ.ID NO:1137 | CQPACCVPVCC |
| SEQ.ID NO:1138 | CQPACCVSSCC |
| SEQ.ID NO:1139 | CQPCCHPTCCQ |
| SEQ.ID NO:1140 | CQPCCRPACCE |
| SEQ.ID NO:1141 | CQPCCRPACCQ |
| SEQ.ID NO:1142 | CQPCCRPTCCQ |
| SEQ.ID NO:1143 | CQPCYCPACCV |
| SEQ.ID NO:1144 | CQPICCGSSCC |
| SEQ.ID NO:1145 | CQPRCCETSCC |
| SEQ.ID NO:1146 | CQPSCCETSCC |
| SEQ.ID NO:1147 | CQPSCCRPACC |
| SEQ.ID NO:1148 | CQPSCCVPSCC |
| SEQ.ID NO:1149 | CQPSCCVSSCC |
| SEQ.ID NO:1150 | CQPTCCCPSYC |
| SEQ.ID NO:1151 | CQPTCCGSSCC |
| SEQ.ID NO:1152 | CQPTCCHPSCC |
| SEQ.ID NO:1153 | CQPTCCQPTCC |
| SEQ.ID NO:1154 | CQPTCCRPSCC |
| SEQ.ID NO:1155 | CQPTCCRPTCC |
| SEQ.ID NO:1156 | CQPTCCRTTCC |
| SEQ.ID NO:1157 | CQQACCMPVCC |
| SEQ.ID NO:1158 | CQQACCVPICC |
| SEQ.ID NO:1159 | CQQACCVPVCC |
| SEQ.ID NO:1160 | CQQSCCVPVCC |
| SEQ.ID NO:1161 | CQQSCCVSVCC |
| SEQ.ID NO:1162 | CQSNCCVPVCC |
| SEQ.ID NO:1163 | CQSSCCCPASC |
| SEQ.ID NO:1164 | CQSSCCKPCCS |
| SEQ.ID NO:1165 | CQSSCCKPCSC |
| SEQ.ID NO:1166 | CQSSCCKPYCC |
| SEQ.ID NO:1167 | CQSSCCNPCCS |
| SEQ.ID NO:1168 | CQSSCCQSSCC |
| SEQ.ID NO:1169 | CQSSCCVPVCC |
| SEQ.ID NO:1170 | CQSSCFKPCCC |
| SEQ.ID NO:1171 | CQSSCSKPCCC |
| SEQ.ID NO:1172 | CQSSCYKPCCC |
| SEQ.ID NO:1173 | CQSVCCQPTCC |
| SEQ.ID NO:1174 | CQTTCCCPSCV |
| SEQ.ID NO:1175 | CQTTCCRPSCC |
| SEQ.ID NO:1176 | CQTTCCRTTCC |
| SEQ.ID NO:1177 | CRPACCETTCC |
| SEQ.ID NO:1178 | CRPACCQNTCC |
| SEQ.ID NO:1179 | CRPCCCLRPVC |
| SEQ.ID NO:1180 | CRPCCCVRPVC |
| SEQ.ID NO:1181 | CRPCCWATTCC |
| SEQ.ID NO:1182 | CRPLCCQTTCC |
| SEQ.ID NO:1183 | CRPQCCQSVCC |
| SEQ.ID NO:1184 | CRPQCCQTTCC |
| SEQ.ID NO:1185 | CRPRCCISSCC |
| SEQ.ID NO:1186 | CRPSCCESSCC |
| SEQ.ID NO:1187 | CRPSCCISSCC |
| SEQ.ID NO:1188 | CRPSCCKPQCC |
| SEQ.ID NO:1189 | CRPSCCPSCCQ |
| SEQ.ID NO:1190 | CRPSCCQTTCC |
| SEQ.ID NO:1191 | CRPSCCRPQCC |
| SEQ.ID NO:1192 | CRPSCCVSRCC |
| SEQ.ID NO:1193 | CRPSCCVSSCC |
| SEQ.ID NO:1194 | CRPTCCQNTCC |
| SEQ.ID NO:1195 | CRPVCCCEPTC |
| SEQ.ID NO:1196 | CRPVCCCYSCE |
| SEQ.ID NO:1197 | CRTTCCHPSCC |
| SEQ.ID NO:1198 | CRTTCCRPSCC |
| SEQ.ID NO:1199 | CSCCKPYCSQC |
| SEQ.ID NO:1200 | CSKPCCCQSSC |
| SEQ.ID NO:1201 | CSPCCQPTCCR |
| SEQ.ID NO:1202 | CSPCCVSSCCQ |
| SEQ.ID NO:1203 | CSQCSCCKPCY |
| SEQ.ID NO:1204 | CSQCSCYKPCC |
| SEQ.ID NO:1205 | CSQSNCCKPCC |
| SEQ.ID NO:1206 | CSQSSCCKPCC |
| SEQ.ID NO:1207 | CSSSCCQPSCC |
| SEQ.ID NO:1208 | CTPSCCQPACC |
| SEQ.ID NO:1209 | CVASCCQPSCC |
| SEQ.ID NO:1210 | CVPICCCKPVC |
| SEQ.ID NO:1211 | CVPSCCQPCCH |
| SEQ.ID NO:1212 | CVPVCCCKPMC |
| SEQ.ID NO:1213 | CVPVCCCKPVC |
| SEQ.ID NO:1214 | CVPVCCKPVCC |
| SEQ.ID NO:1215 | CVSSCCKPQCC |
| SEQ.ID NO:1216 | CVSSCCQHSCC |
| SEQ.ID NO:1217 | CVSSCCQPCCH |
| SEQ.ID NO:1218 | CVSSCCQPCCR |
| SEQ.ID NO:1219 | CVSSCCQPFCC |
| SEQ.ID NO:1220 | CVSSCCQPSCC |
| SEQ.ID NO:1221 | CVSSCCRPQCC |
| SEQ.ID NO:1222 | CVTRCCSTPCC |
| SEQ.ID NO:1223 | CVTSCCQPACC |
| SEQ.ID NO:1224 | CVTSCCQPSCC |
| SEQ.ID NO:1225 | CVYSCCQPFCC |
| SEQ.ID NO:1226 | CVYSCCQPSCC |
| SEQ.ID NO:1227 | CYCPACCVSSC |
| SEQ.ID NO:1228 | CYKPCCCQSSC |
| SEQ.ID NO:1229 | CYKPCCCSSGC |
| SEQ.ID NO:1230 | MCCCVPACSCS |
| SEQ.ID NO:1231 | NCCVPVCCQCK |
| SEQ.ID NO:1232 | QCSCCKPCYCS |
| SEQ.ID NO:1233 | QCSCYKPCCCS |
| SEQ.ID NO:1234 | SCCVPICCQCK |
| SEQ.ID NO:1235 | SCCVPVCCQCK |
| SEQ.ID NO:1236 | SCGCSQCNCCK |
| SEQ.ID NO:1237 | SCGCSQCSCCK |
| SEQ.ID NO:1238 | VCCCVPACSCS |
| SEQ.ID NO:1239 | VCCCVPACSCT |

The present invention is of course in any way restricted to the embodiments herein described and one with ordinary skill in the area can provide many possibilities to modifications and substitutions of technical characteristics by equivalent ones, depending on each situation, as defined in the claims.

The preferred embodiments described above may obviously be combined. The following claims define further preferred embodiments.

## Claims

1. Peptide composition for hair treatment comprising at least one peptide with a sequence length of 6-12 amino acids, where 2-5 of those amino acids are cysteines, and dermatologically adequate excipients,
wherein the peptides comprise at least one of the sequences of the following list with a degree of homology equal or higher than 90%: SEQ.ID NO:75; SEQ.ID NO:412; SEQ.ID NO:1131 as a hair strengthener of human hair or of animal hair.

2. Composition according to the previous claim wherein peptides comprise at least one of the sequences of the following list: SEQ.ID NO:75; SEQ.ID NO:412; SEQ.ID NO:1131.

3. Composition according to the previous claims **characterized by** the concentration of peptide vary between 0.001%-20% (w/w), and preferably 0.01-5% (w/w).

4. Composition according to the previous claims comprising at least one of the excipients selected from the following list: surfactants, emulsifiers, preservatives, thickeners, organic polymers, humectants, silicones, oils, fragrances, vitamins, buffers, antimicrobial agents, antibacterial agents, disinfectants agents, or any mixture thereof.

5. Composition according to the previous claims comprising at least one of the excipients selected from the following list: anionic surfactant, amphoteric surfactant, cationic surfactant, non-ionic surfactant, emulsifier, preservative, thickener, natural polymer derived, humectant, silicone, organic oil, protein, fragrance, vitamin, emollient ester, alkanolamide, amine, buffer, pH adjustor, salt, aliphatic alcohol, UV filter, amine oxide, chelate, fatty acid, antimicrobial/ antibacterial agent, PEG material, polymer, anti-static agent, alcohol, or any mixture thereof.

6. Composition according to the previous claims comprising at least one surfactant selected from the following list: anionic surfactant, amphoteric surfactant, cationic surfactant, non-ionic surfactant.

7. Composition according to the previous claims comprising at least one compound selected from the following list: emulsifier, preservative, thickener, natural polymer derived, humectant, silicone, organic oil, protein, fragrance, vitamin, emollient ester, alkanolamide, amine, buffer, pH adjustor, salt, aliphatic alcohol, UV filter, amine oxide, chelate, fatty acid, antimicrobial agent, antibacterial agent, PEG material, polymer, anti-static agent, alcohol, or any mixture thereof.

8. Composition according to any of the previous claims comprising fragrance, oil or any mixture thereof.

9. Composition according to any of the previous claims comprising a dying agent/dye linked to the N or C terminal of the referred peptides.

10. Composition according to any of the previous claims for use in medicine and/or veterinary.

11. Composition according to any of the previous claims for use in hair treatment.

12. Composition according to any of the previous claims for treatment of hair scalp diseases, particularly scalp irritation, alopecia areata, lichen planus, folliculitis keloid of the neck, trichorrhexis nodosa, tricodistrophy, pili torti, tricorrexis invaginata, moniletrix, uncombable hair syndrome.

13. Use of the composition described in any of the previous claims in cosmetics.

14. Use of the composition described in any of the previous claims for use as hair strengthener of human hair or of animal hair.

15. Use of the composition described in any of the previous claims as hair curl or uncurl agent or as hair coloring.

16. Shampoo, lotion, serum, cream, conditioner, foam, elixir, oil, aerosol or mask comprising a composition described in any of the previous claims.

## Patentansprüche

1. Peptidzusammensetzung zur Haarbehandlung, umfassend mindestens ein Peptid mit einer Sequenzlänge von 6-12 Aminosäuren, wobei 2-5 dieser Aminosäuren Cysteine und dermatologisch geeignete Hilfsstoffe sind,
wobei die Peptide mindestens eine der aus folgender Liste ausgewählten Sequenzen mit einem Homologiegrad gleich oder größer als 90 % umfassen: SEQ.ID NO:75; SEQ.ID NO:412; SEQ.ID NO:1131 als Haarfestigungsmittel für menschliches oder tierisches Haar.

2. Zusammensetzung nach dem vorangehenden Anspruch, wobei die Peptide mindestens eine der Sequenzen aus folgender Liste umfassen: SEQ.ID NO:75; SEQ.ID NO:412; SEQ.ID NO:1131.

3. Zusammensetzung nach den vorangehenden Ansprüchen, **dadurch gekennzeichnet dass** die Peptidkonzentration zwischen 0,001 % - 20 % (w/w) und vorzugsweise 0,01 - 5 % (w/w) variiert.

4. Zusammensetzung nach den vorangehenden Ansprüchen, umfassend mindestens einen der aus folgender Liste ausgewählten Hilfsstoffe: Tenside, Emulgatoren, Konservierungsmittel, Verdickungsmittel, organische Polymere, Feuchthaltemittel, Silikone, Öle, Duftstoffe, Vitamine, Puffer, antimikrobielle Wirkstoffe, antibakterielle Wirkstoffe, Desinfektionsmittel oder eine beliebige Mischung davon.

5. Zusammensetzung nach den vorangehenden Ansprüchen, umfassend mindestens einen der aus folgender Liste ausgewählten Hilfsstoffe: anionisches Tensid, amphoteres Tensid, kationisches Tensid, nichtionisches Tensid, Emulgator, Konservierungsmittel, Verdickungsmittel, natürlich gewonnenes Polymer, Feuchthaltemittel, Silikon, organisches Öl, Protein, Duftstoff, Vitamin, Emollient-Ester, Alkanolamide, Amine, Puffer, pH-Stellmittel, Salz, aliphatischer Alkohol, UV-Filter, Aminoxid, Chelat, Fettsäure, antimikrobieller/ antibakterieller Wirkstoff, PEG-Material, Polymer, Antistatikum, Alkohol oder eine beliebige Mischung davon.

6. Zusammensetzung nach den vorangehenden Ansprüchen, umfassend mindestens eines der aus folgender Liste ausgewählten Tenside: anionisches Tensid, amphoteres Tensid, kationisches Tensid, nichtionisches Tensid.

7. Zusammensetzung nach den vorangehenden Ansprüchen, umfassend mindestens eine der aus folgender Liste ausgewählten Verbindung: Emulgator, Konservierungsmittel, Verdickungsmittel, natürlich gewonnenes Polymer, Feuchthaltemittel, Silikon, organisches Öl, Protein, Duftstoff, Vitamin, Emollient-Ester, Alkanolamide, Amine, Puffer, pH-Stellmittel, Salz, aliphatischer Alkohol, UV-Filter, Aminoxid, Chelat, Fettsäure, antimikrobieller Wirkstoff, antibakterieller Wirkstoff, PEG-Material, Polymer, Antistatikum, Alkohol oder eine beliebige Mischung davon.

8. Zusammensetzung nach den vorangehenden Ansprüchen, umfassend Duftstoff, Öl oder eine beliebige Mischung davon.

9. Zusammensetzung nach den vorangehenden Ansprüchen, umfassend ein Färbemittel/Farbstoff, das/der mit dem N- oder C-Terminus der genannten Peptide verknüpft ist.

10. Zusammensetzung nach den vorangehenden Ansprüchen zur Verwendung in der Medizin und/oder im Veterinärbereich.

11. Zusammensetzung nach den vorangehenden Ansprüchen zur Verwendung für die Haarbehandlung.

12. Zusammensetzung nach den vorangehenden Ansprüchen zur Behandlung von Erkrankungen der Kopfhaut, insbesondere Reizung der Kopfhaut, Alopecia areata, Lichen planus, Folliculitis keloidalis der Nackenregion, Trichorrhexis nodosa, Trichodystrophie, Pili torti, Trichorrhexis invaginata, Monilethrix, Syndrom der unkämmbaren Haare.

13. Verwendung der in den vorangehenden Ansprüchen beschriebenen Zusammensetzung in Kosmetika.

14. Verwendung der in den vorangehenden Ansprüchen beschriebenen Zusammensetzung zur Verwendung als Haarfestigungsmittel für menschliches oder tierisches Haar.

15. Verwendung der in den vorangehenden Ansprüchen beschriebenen Zusammensetzung als Locken- oder Glättungs- oder Haarfärbemittel.

16. Shampoo, Lotion, Serum, Creme, Haarspülung, Schaum, Elixier, Öl, Aerosol oder Maske, umfassend eine der in den vorangehenden Ansprüchen beschriebenen Zusammensetzungen.

## Revendications

1. Composition peptidique pour le traitement capilaire comprenant au moins un peptide avec une longueur de séquence de 6-12 aminoacides, dans lequel 2-5 de ces aminoacides sont des cystéines, et des excipients dermatologiquement adéquats,
dans laquelle les peptides comprennent au moins une des séquences de la liste suivante avec un degré d'homologie égal ou supérieur à 90 % : SEQ.ID NO :75 ; SEQ.ID NO :412 ; SEG.ID NO :1131 en tant que fortifiant capilaire de cheveux humains ou de poil animal.

2. Composition selon la revendication précédente, dans laquelle les peptides comprennent au moins une des séquences de la liste suivante : SEG.ID NO : 75 ; SEG.ID NO :412 ; SEQ.ID NO : 1131.

3. Composition selon les revendications précédentes, charactérisée en ce que la concentration de peptide varie entre 0,001 % - 20 % (p/p) et de préférence 0,01 - 5 % (p/p).

4. Composition selon les revendications précédentes comprenant au moins un des excipients sélectionnés à partir de la liste suivante : tensioactifs, émulsifiants, conservateurs, épaississants, polymères organiques, humectants, silicones, huiles, fragrances, vitamines, tampons, agents antimicrobiens, agents antibactériens, agents désinfectants, ou un quelconque mélange de ceux-ci.

5. Composition selon les revendications précédentes comprenant au moins un des excipients sélectionnés à partir de la liste suivante : tensioactif anionique, tensioactif amphotère, tensioactif cationique, tensioactif non-ionique, émulsifiant, conservateur, épaississant, polymère naturel dérivé, humectant, silicone, huile organique, protéine, fragrance, vitamine, ester émollient, alcanolamide, amine, tampon, ajusteur de pH, sel, alcool aliphatique, filtre UV, amine oxyde, chélate, acide gras, agent antimicrobien/antibactérien, matière PEG, polymère, agent antistatique, alcool ou un quelconque mélange de ceux-ci.

6. Composition selon les revendications précédentes comprenant au moins un tensioactif sélectionné à partir de la liste suivante : tensioactif anionique, tensioactif amphotère, tensioactif cationique, tensioactif non-ionique.

7. Composition selon les revendications précédentes comprenant au moins un composé sélectionné à partir de la liste suivante : émulsifiant, conservateur, épaississant, polymère naturel dérivé, humectant, silicone, huile organique, protéine, fragrance, vitamine, ester émollient, alcanolamide, amine, tampon, ajusteur de pH, sel, alcool aliphatique, filtre UV, amine oxyde, chélate, acide gras, agent antimicrobien, agent antibactérien, matière PEG, polymère, agent antistatique, alcool, ou un quelconque mélange de ceux-ci.

8. Composition selon l'une quelconque des revendications précédentes comprenant une fragrance, une huile, ou un quelconque mélange de celles-ci.

9. Composition selon l'une quelconque des revendications précédentes comprenant un agent colorant/ une coloration, lié au terminal N ou C des peptides en question.

10. Composition selon l'une quelconque des revendications précédentes pour une utilisation en médecine et/ou médecine vétérinaire.

11. Composition selon l'une quelconque des revendications précédentes pour une utilisation en traitement capilaire.

12. Composition selon l'une quelconque des revendications précédentes pour le traitement de maladies du cuir chevelu, particulièrement l'irritation du cuir chevelu, l'alopécie areata, le lichen plan, la folliculite chéloïdienne de la nuque, la trichorrhexix nodosa, la trichodystrophie, le pili torti, la tricorrexis invaginata, le monilethrix, le syndrome des cheveux incoiffables.

13. Utilisation de la composition décrite dans l'une quelconque des revendications précédentes en cosmétique.

14. Utilisation de la composition décrite dans l'une quelconque des revendications précédentes pour une utilisation en tant que fortifiant capilaire de cheveux humains et de poil animal.

15. Utilisation de la composition décrite dans l'une quelconque des revendications précédentes en tant qu'agent pour boucler ou déboucler les cheveux ou en tant que coloration pour cheveux.

16. Shampooing, lotion, sérum, crème, après-shampoing, mousse, élixir, huile, aérosol ou masque comprenant une composition décrite dans l'une quelconque des revendications précédentes.


### SEQUENCE LISTING

<110> UNIVERSIDADE DO MINHO
<120> ComposiÃ§Ãµes peptidicas e respectivos usos
<130> P159.6 WO
<150> PT107244
   <151> 2013-10-18
<160> 1239
<170> BiSSAP 1.3
<210> 1
   <211> 10
   <212> PRT
   <213> Homo sapiens
<400> 1
   Ala Pro Cys Ala Pro Arg Pro Ser Cys Gly
   1               5                   10
<210> 2
   <211> 10
   <212> PRT
   <213> Homo sapiens
<400> 2
   Glu Ala Cys Val Pro Ser Val Pro Cys Pro
   1               5                   10
<210> 3
   <211> 10
   <212> PRT
   <213> Homo sapiens
<400> 3
   Glu Ser Cys Gly Thr Ala Ser Gly Cys Ala
   1               5                   10
<210> 4
   <211> 10
   <212> PRT
   <213> Homo sapiens
<400> 4
   Gly Leu Cys Ala Gly Thr Ser Ala Cys Leu
   1               5                   10
<210> 5
   <211> 10
   <212> PRT
   <213> Homo sapiens
<400> 5
   Gly Val Cys Gly Pro Ser Pro Pro Cys Ile
   1               5                   10
<210> 6
   <211> 10
   <212> PRT
   <213> Homo sapiens
<400> 6
   His Gly Cys Thr Leu Pro Gly Ala Cys Asn
   1               5                   10
<210> 7
   <211> 10
   <212> PRT
   <213> Homo sapiens
<400> 7
   His Ser Cys Thr Leu Pro Gly Ala Cys Asn
   1               5                   10
<210> 8
   <211> 10
   <212> PRT
   <213> Homo sapiens
<400> 8
   Lys Asp Cys Leu Gln Asn Ser Leu Cys Glu
   1               5                   10
<210> 9
   <211> 10
   <212> PRT
   <213> Homo sapiens
<400> 9
   Leu Pro Cys Leu Pro Ala Ala Ser Cys Gly
   1               5                   10
<210> 10
   <211> 10
   <212> PRT
   <213> Homo sapiens
<400> 10
   Leu Pro Cys Tyr Phe Thr Gly Ser Cys Asn
   1               5                   10
<210> 11
   <211> 10
   <212> PRT
   <213> Homo sapiens
<400> 11
   Asn Phe Cys Leu Pro Ser Leu Ser Cys Arg
   1               5                   10
<210> 12
   <211> 10
   <212> PRT
   <213> Homo sapiens
<400> 12
   Asn Pro Cys Ala Thr Thr Asn Ala Cys Asp
   1               5                   10
<210> 13
   <211> 10
   <212> PRT
   <213> Homo sapiens
<400> 13
   Asn Pro Cys Ala Thr Thr Asn Ala Cys Glu
   1 5 10
<210> 14
   <211> 10
   <212> PRT
   <213> Homo sapiens
<400> 14
   Asn Pro Cys Ala Thr Thr Asn Ala Cys Ser
   1 5 10
<210> 15
   <211> 10
   <212> PRT
   <213> Homo sapiens
<400> 15
   Asn Pro Cys Gly Leu Arg Ala Arg Cys Gly
   1 5 10
<210> 16
   <211> 10
   <212> PRT
   <213> Homo sapiens
<400> 16
   Asn Pro Cys Gly Pro Arg Ser Arg Cys Gly
   1 5 10
<210> 17
   <211> 10
   <212> PRT
   <213> Homo sapiens
<400> 17
   Asn Pro Cys Ser Thr Pro Ala Ser Cys Thr
   1 5 10
<210> 18
   <211> 10
   <212> PRT
   <213> Homo sapiens
<400> 18
   Asn Pro Cys Ser Thr Ser Pro Ser Cys Val
   1 5 10
<210> 19
   <211> 10
   <212> PRT
   <213> Homo sapiens
<400> 19
   Pro Ala Cys Thr Ser Ser Ser Pro Cys Ser
   1 5 10
<210> 20
   <211> 10
   <212> PRT
   <213> Homo sapiens
<400> 20
   Ser Lys Cys His Glu Ser Thr Val Cys Pro
   1 5 10
<210> 21
   <211> 10
   <212> **PRT**
   <213> Homo sapiens
<400> 21
   Ser Pro Cys Val Pro Arg Thr Val Cys Val
   1 5 10
<210> 22
   <211> 10
   <212> PRT
   <213> Homo sapiens
<400> 22
   Ser Ser Cys Ser Val Glu Thr Ala Cys Leu
   1 5 10
<210> 23
   <211> 10
   <212> PRT
   <213> Homo sapiens
<400> 23
   Ser Val Cys Ser Ser Gly Val Asn Cys Arg
   1 5 10
<210> 24
   <211> 10
   <212> PRT
   <213> Homo sapiens
<400> 24
   Thr Ala Cys Pro Leu Pro Gly Thr Cys His
   1 5 10
<210> 25
   <211> 10
   <212> PRT
   <213> Homo sapiens
<400> 25
   Thr Asn Cys Ser Pro Arg Pro Ile Cys Val
   1 5 10
<210> 26
   <211> 10
   <212> PRT
   <213> Homo sapiens
<400> 26
   Thr Ser Cys Val Pro Pro Ala Pro Cys Thr
   1 5 10
<210> 27
   <211> 10
   <212> PRT
   <213> Homo sapiens
<400> 27
   Thr Thr Cys Thr Ser Ser Asn Thr Cys Glu
   1 5 10
<210> 28
   <211> 10
   <212> PRT
   <213> Homo sapiens
<400> 28
   Val Pro Cys Val Pro Ser Val Pro Cys Thr
   1 5 10
<210> 29
   <211> 10
   <212> PRT
   <213> Homo sapiens
<400> 29
   Ala Thr Cys Gly Pro Ser Ala Cys Ile Thr
   1 5 10
<210> 30
   <211> 10
   <212> PRT
   <213> Homo sapiens
<400> 30
   Gly Pro Cys Ile Ser Asn Pro Cys Gly Leu
   1 5 10
<210> 31
   <211> 10
   <212> PRT
   <213> Homo sapiens
<400> 31
   Gly Pro Cys Leu Ser Asn Pro Cys Thr Ser
   1 5 10
<210> 32
   <211> 10
   <212> PRT
   <213> Homo sapiens
<400> 32
   Gly Ser Cys Val Thr Asn Pro Cys Gly Pro
   1 5 10
<210> 33
   <211> 10
   <212> PRT
   <213> Homo sapiens
<400> 33
   Leu Thr Cys Phe Ser Ile Thr Cys Ser Ser
   1 5 10
<210> 34
   <211> 10
   <212> PRT
   <213> Homo sapiens
<400> 34
   Asn Pro Cys Ser Thr Pro Ser Cys Thr Thr
   1 5 10
<210> 35
   <211> 10
   <212> PRT
   <213> Homo sapiens
<400> 35
   Pro Ser Cys Val Thr Ala Pro Cys Ala Pro
   1 5 10
<210> 36
   <211> 10
   <212> PRT
   <213> Homo sapiens
<400> 36
   Ser Asp Cys Ser Ser Thr His Cys Ser Pro
   1 5 10
<210> 37
   <211> 10
   <212> PRT
   <213> Homo sapiens
<400> 37
   Ser Leu Cys Leu Pro Pro Thr Cys His Thr
   1               5                   10
<210> 38
   <211> 10
   <212> PRT
   <213> Homo sapiens
<400> 38
   Ser Leu Cys Asn Leu Gly Ser Cys Gly Pro
   1               5                   10
<210> 39
   <211> 10
   <212> PRT
   <213> Homo sapiens
<400> 39
   Ser Pro Cys Leu Val Gly Asn Cys Ala Trp
   1               5                   10
<210> 40
   <211> 10
   <212> PRT
   <213> Homo sapiens
<400> 40
   Thr Ala Cys Leu Pro Gly Thr Cys Ala Thr
   1               5                   10
<210> 41
   <211> 10
   <212> PRT
   <213> Homo sapiens
<400> 41
   Thr Ser Cys Leu Pro Ala Leu Cys Leu Pro
   1               5                   10
<210> 42
   <211> 10
   <212> PRT
   <213> Homo sapiens
<400> 42
   Thr Ser Cys Ser Ser Arg Pro Cys Val Pro
   1 5 10
<210> 43
   <211> 10
   <212> PRT
   <213> Homo sapiens
<400> 43
   Thr Thr Cys Gly Gly Gly Ser Cys Gly Val
   1 5 10
<210> 44
   <211> 10
   <212> PRT
   <213> Homo sapiens
<400> 44
   Val Asn Cys Arg Pro Glu Leu Cys Leu Gly
   1 5 10
<210> 45
   <211> 10
   <212> PRT
   <213> Homo sapiens
<400> 45
   Tyr Val Cys Gln Pro Met Ala Cys Leu Pro
   1 5 10
<210> 46
   <211> 10
   <212> PRT
   <213> Homo sapiens
<400> 46
   Ala Phe Ser Cys Ile Ser Ala Cys Gly Pro
   1 5 10
<210> 47
   <211> 10
   <212> PRT
   <213> Homo sapiens
<400> 47
   Gly Ser Val Cys Ser Ala Pro Cys Asn Gly
   1 5 10
<210> 48
   <211> 10
   <212> PRT
   <213> Homo sapiens
<400> 48
   Gly Val Val Cys Gly Asp Leu Cys Ala Ser
   1               5                   10
<210> 49
   <211> 10
   <212> PRT
   <213> Homo sapiens
<400> 49
   Gly Val Val Cys Gly Asp Leu Cys Val Ser
   1               5                   10
<210> 50
   <211> 10
   <212> PRT
   <213> Homo sapiens
<400> 50
   Leu Thr Gly Cys Leu Leu Pro Cys Tyr Phe
   1               5                   10
<210> 51
   <211> 10
   <212> PRT
   <213> Homo sapiens
<400> 51
   Asn Glu Asp Cys Lys Leu Pro Cys Asn Pro
   1               5                   10
<210> 52
   <211> 10
   <212> PRT
   <213> Homo sapiens
<400> 52
   Asn Phe Ser Cys Val Ser Ala Cys Gly Pro
   1               5                   10
<210> 53
   <211> 10
   <212> PRT
   <213> Homo sapiens
<400> 53
   Pro Pro Thr Cys His Thr Ala Cys Pro Leu
   1               5                   10
<210> 54
   <211> 10
   <212> PRT
   <213> Homo sapiens
<400> 54
   Pro Gln Pro Cys Ala Thr Ala Cys Lys Pro
   1               5                   10
<210> 55
   <211> 10
   <212> PRT
   <213> Homo sapiens
<400> 55
   Ser Glu Asp Cys Lys Leu Pro Cys Asn Pro
   1               5                   10
<210> 56
   <211> 10
   <212> PRT
   <213> Homo sapiens
<400> 56
   Ser Leu Gly Cys Arg Thr Ser Cys Ser Ser
   1               5                   10
<210> 57
   <211> 10
   <212> PRT
   <213> Homo sapiens
<400> 57
   Ser Leu Ser Cys Arg Thr Ser Cys Ser Ser
   1               5                   10
<210> 58
   <211> 10
   <212> PRT
   <213> Homo sapiens
<400> 58
   Ser Ser Ser Cys Pro Leu Gly Cys Thr Met
   1               5                   10
<210> 59
   <211> 10
   <212> PRT
   <213> Homo sapiens
<400> 59
   Thr Gly Ser Cys Asn Ser Pro Cys Leu Val
   1               5                   10
<210> 60
   <211> 10
   <212> PRT
   <213> Homo sapiens
<400> 60
   Thr Ser Ser Cys Pro Leu Gly Cys Thr Met
   1 5 10
<210> 61
   <211> 10
   <212> PRT
   <213> Homo sapiens
<400> 61
   Val Gly Ser Cys Gly Ser Ser Cys Arg Lys
   1 5 10
<210> 62
   <211> 10
   <212> PRT
   <213> Homo sapiens
<400> 62
   Val Gly Val Cys Gly Gly Ser Cys Lys Arg
   1 5 10
<210> 63
   <211> 10
   <212> PRT
   <213> Homo sapiens
<400> 63
   Val Ser Asn Cys Asn Trp Phe Cys Glu Gly
   1 5 10
<210> 64
   <211> 10
   <212> PRT
   <213> Homo sapiens
<400> 64
   Ala Cys Gly Pro Arg Pro Gly Arg Cys Cys
   1 5 10
<210> 65
   <211> 10
   <212> PRT
   <213> Homo sapiens
<400> 65
   Ala Cys Gly Pro Arg Pro Ser Arg Cys Cys
   1 5 10
<210> 66
   <211> 10
   <212> PRT
   <213> Homo sapiens
<400> 66
   Cys Ala Pro Arg Pro Ser Cys Gly Pro Cys
   1               5                   10
<210> 67
   <211> 10
   <212> PRT
   <213> Homo sapiens
<400> 67
   Cys Glu Pro Cys Ser Ala Tyr Val Ile Cys
   1               5                   10
<210> 68
   <211> 10
   <212> PRT
   <213> Homo sapiens
<400> 68
   Cys Gly Leu Arg Ala Arg Cys Gly Pro Cys
   1               5                   10
<210> 69
   <211> 10
   <212> PRT
   <213> Homo sapiens
<400> 69
   Cys Gly Pro Arg Pro Gly Arg Cys Cys Ile
   1               5                   10
<210> 70
   <211> 10
   <212> PRT
   <213> Homo sapiens
<400> 70
   Cys Gly Pro Arg Pro Ser Arg Cys Cys Ile
   1               5                   10
<210> 71
   <211> 10
   <212> PRT
   <213> Homo sapiens
<400> 71
   Cys Gly Pro Arg Ser Arg Cys Gly Pro Cys
   1 5 10
<210> 72
   <211> 10
   <212> PRT
   <213> Homo sapiens
<400> 72
   Cys Gly Thr Ser Gln Lys Gly Cys Cys Asn
   1 5 10
<210> 73
   <211> 10
   <212> PRT
   <213> Homo sapiens
<400> 73
   Cys His Gly Cys Thr Leu Pro Gly Ala Cys
   1 5 10
<210> 74
   <211> 10
   <212> PRT
   <213> Homo sapiens
<400> 74
   Cys His Ser Cys Thr Leu Pro Gly Ala Cys
   1 5 10
<210> 75
   <211> 10
   <212> PRT
   <213> Homo sapiens
<400> 75
   Cys Leu Pro Cys Leu Pro Ala Ala Ser Cys
   1 5 10
<210> 76
   <211> 10
   <212> PRT
   <213> Homo sapiens
<400> 76
   Cys Leu Pro Pro Thr Cys His Thr Ala Cys
   1 5 10
<210> 77
   <211> 10
   <212> PRT
   <213> Homo sapiens
<400> 77
   Cys Leu Ser Asn Pro Cys Thr Ser Cys Val
   1 5 10
<210> 78
   <211> 10
   <212> PRT
   <213> Homo sapiens
<400> 78
   Cys Leu Val Gly Asn Cys Ala Trp Cys Glu
   1 5 10
<210> 79
   <211> 10
   <212> PRT
   <213> Homo sapiens
<400> 79
   Cys Asn Pro Cys Ser Thr Pro Ala Ser Cys
   1 5 10
<210> 80
   <211> 10
   <212> PRT
   <213> Homo sapiens
<400> 80
   Cys Asn Pro Cys Ser Thr Pro Ser Cys Thr
   1 5 10
<210> 81
   <211> 10
   <212> PRT
   <213> Homo sapiens
<400> 81
   Cys Asn Pro Cys Ser Thr Ser Pro Ser Cys
   1 5 10
<210> 82
   <211> 10
   <212> PRT
   <213> Homo sapiens
<400> 82
   Cys Asn Ser Pro Cys Leu Val Gly Asn Cys
   1 5 10
<210> 83
   <211> 10
   <212> PRT
   <213> Homo sapiens
<400> 83
   Cys Arg Thr Ser Cys Ser Ser Arg Pro Cys
   1               5                   10
<210> 84
   <211> 10
   <212> PRT
   <213> Homo sapiens
<400> 84
   Cys Ser Leu Lys Glu His Cys Ser Ala Cys
   1               5                   10
<210> 85
   <211> 10
   <212> PRT
   <213> Homo sapiens
<400> 85
   Cys Ser Pro Arg Pro Ile Cys Val Pro Cys
   1               5                   10
<210> 86
   <211> 10
   <212> PRT
   <213> Homo sapiens
<400> 86
   Cys Ser Ser Thr Met Ser Tyr Ser Cys Cys
   1               5                   10
<210> 87
   <211> 10
   <212> PRT
   <213> Homo sapiens
<400> 87
   Cys Ser Thr Pro Ala Ser Cys Thr Ser Cys
   1               5                   10
<210> 88
   <211> 10
   <212> PRT
   <213> Homo sapiens
<400> 88
   Cys Ser Thr Pro Ser Cys Thr Thr Cys Val
   1               5                   10
<210> 89
   <211> 10
   <212> PRT
   <213> Homo sapiens
<400> 89
   Cys Thr Ser Cys Val Pro Pro Ala Pro Cys
   1 5 10
<210> 90
   <211> 10
   <212> PRT
   <213> Homo sapiens
<400> 90
   Cys Thr Ser Ser Asn Thr Cys Glu Pro Cys
   1 5 10
<210> 91
   <211> 10
   <212> PRT
   <213> Homo sapiens
<400> 91
   Cys Val Pro Pro Ala Pro Cys Thr Pro Cys
   1 5 10
<210> 92
   <211> 10
   <212> PRT
   <213> Homo sapiens
<400> 92
   Cys Val Pro Pro Ser Cys His Gly Cys Thr
   1 5 10
<210> 93
   <211> 10
   <212> PRT
   <213> Homo sapiens
<400> 93
   Cys Val Pro Pro Ser Cys His Ser Cys Thr
   1 5 10
<210> 94
   <211> 10
   <212> **PRT**
   <213> Homo sapiens
<400> 94
   Asp Cys Lys Leu Pro Cys Asn Pro Cys Ala
   1 5 10
<210> 95
   <211> 10
   <212> PRT
   <213> Homo sapiens
<400> 95
   Asp Cys Lys Leu Pro Cys Asn Pro Cys Ser
   1 5 10
<210> 96
   <211> 10
   <212> PRT
   <213> Homo sapiens
<400> 96
   Pro Cys Gly Thr Ser Gln Lys Gly Cys Cys
   1 5 10
<210> 97
   <211> 10
   <212> PRT
   <213> Homo sapiens
<400> 97
   Pro Cys Leu Ser Asn Pro Cys Thr Ser Cys
   1 5 10
<210> 98
   <211> 10
   <212> PRT
   <213> Homo sapiens
<400> 98
   Pro Cys Leu Val Gly Asn Cys Ala Trp Cys
   1 5 10
<210> 99
   <211> 10
   <212> PRT
   <213> Homo sapiens
<400> 99
   Pro Cys Asn Pro Cys Ser Thr Pro Ser Cys
   1 5 10
<210> 100
   <211> 10
   <212> PRT
   <213> Homo sapiens
<400> 100
   Pro Cys Ser Thr Pro Ser Cys Thr Thr Cys
   1 5 10
<210> 101
   <211> 10
   <212> PRT
   <213> Homo sapiens
<400> 101
   Pro Cys Thr Thr Cys Gly Pro Thr Cys Gly
   1 5 10
<210> 102
   <211> 10
   <212> PRT
   <213> Homo sapiens
<400> 102
   Pro Cys Val Pro Pro Ser Cys His Gly Cys
   1 5 10
<210> 103
   <211> 10
   <212> PRT
   <213> Homo sapiens
<400> 103
   Pro Cys Val Pro Pro Ser Cys His Ser Cys
   1 5 10
<210> 104
   <211> 10
   <212> PRT
   <213> Homo sapiens
<400> 104
   Ser Cys Cys Leu Pro Ser Leu Gly Cys Arg
   1 5 10
<210> 105
   <211> 10
   <212> PRT
   <213> Homo sapiens
<400> 105
   Ser Cys Ser Glu Glu Leu Gln Cys Cys Gln
   1 5 10
<210> 106
   <211> 10
   <212> PRT
   <213> Homo sapiens
<400> 106
   Ser Cys Ser Pro Cys Ser Thr Thr Cys Thr
   1 5 10
<210> 107
   <211> 10
   <212> PRT
   <213> Homo sapiens
<400> 107
   Ala Ser Cys Ser Thr Ser Gly Thr Cys Gly
   1 5 10
<210> 108
   <211> 10
   <212> PRT
   <213> Homo sapiens
<400> 108
   Ala Ser Cys Tyr Ile Pro Val Gly Cys Gln
   1 5 10
<210> 109
   <211> 10
   <212> PRT
   <213> Homo sapiens
<400> 109
   Ala Ser Cys Tyr Val Pro Val Ser Cys Gln
   1 5 10
<210> 110
   <211> 10
   <212> PRT
   <213> Homo sapiens
<400> 110
   Ala Val Cys Thr Leu Pro Ser Ser Cys Gln
   1 5 10
<210> 111
   <211> 10
   <212> PRT
   <213> Homo sapiens
<400> 111
   Asp Leu Cys Pro Thr Ser Val Ser Cys Gly
   1 5 10
<210> 112
   <211> 10
   <212> PRT
   <213> Homo sapiens
<400> 112
   Glu Ile Cys Trp Glu Pro Thr Ser Cys Gln
   1               5                   10
<210> 113
   <211> 10
   <212> PRT
   <213> Homo sapiens
<400> 113
   Glu Thr Cys Gly Glu Pro Thr Ser Cys Gln
   1               5                   10
<210> 114
   <211> 10
   <212> PRT
   <213> Homo sapiens
<400> 114
   Glu Thr Cys Asn Glu Thr Thr Ser Cys Gln
   1               5                   10
<210> 115
   <211> 10
   <212> PRT
   <213> Homo sapiens
<400> 115
   Glu Thr Cys Trp Arg Pro Asn Ser Cys Gln
   1               5                   10
<210> 116
   <211> 10
   <212> PRT
   <213> Homo sapiens
<400> 116
   Gly Tyr Cys Gly Tyr Arg Pro Phe Cys Phe
   1               5                   10
<210> 117
   <211> 10
   <212> PRT
   <213> Homo sapiens
<400> 117
   Lys Thr Cys Trp Glu Pro Ala Ser Cys Gln
   1               5                   10
<210> 118
   <211> 10
   <212> PRT
   <213> Homo sapiens
<400> 118
   Lys Thr Cys Trp Glu Pro Thr Ser Cys Gln
   1 5 10
<210> 119
   <211> 10
   <212> PRT
   <213> Homo sapiens
<400> 119
   Leu Asp Cys Val Asp Thr Thr Pro Cys Lys
   1 5 10
<210> 120
   <211> 10
   <212> PRT
   <213> Homo sapiens
<400> 120
   Leu Gly Cys Gly Tyr Gly Ser Phe Cys Gly
   1 5 10
<210> 121
   <211> 10
   <212> PRT
   <213> Homo sapiens
<400> 121
   Asn Ser Cys Gly Tyr Gly Ser Gly Cys Gly
   1 5 10
<210> 122
   <211> 10
   <212> PRT
   <213> Homo sapiens
<400> 122
   Asn Tyr Cys Pro Ser Asn Thr Met Cys Glu
   1 5 10
<210> 123
   <211> 10
   <212> PRT
   <213> Homo sapiens
<400> 123
   Pro Ala Cys Val Thr Ser Tyr Ser Cys Arg
   1 5 10
<210> 124
   <211> 10
   <212> PRT
   <213> Homo sapiens
<400> 124
   Pro Asp Cys His Val Glu Gly Thr Cys Leu
   1               5                   10
<210> 125
   <211> 10
   <212> PRT
   <213> Homo sapiens
<400> 125
   Pro Asp Cys Arg Val Glu Gly Thr Cys Leu
   1               5                   10
<210> 126
   <211> 10
   <212> PRT
   <213> Homo sapiens
<400> 126
   Pro Ile Cys Ser Glu Pro Ser Pro Cys Ser
   1               5                   10
<210> 127
   <211> 10
   <212> PRT
   <213> Homo sapiens
<400> 127
   Pro Ile Cys Tyr Ile Phe Lys Pro Cys Gln
   1               5                   10
<210> 128
   <211> 10
   <212> PRT
   <213> Homo sapiens
<400> 128
   Pro Leu Cys Tyr Ile Ser Asn Ser Cys Gln
   1               5                   10
<210> 129
   <211> 10
   <212> PRT
   <213> Homo sapiens
<400> 129
   Pro Pro Cys Gly Gln Pro Thr Pro Cys Ser
   1 5 10
<210> 130
   <211> 10
   <212> PRT
   <213> Homo sapiens
<400> 130
   Pro Pro Cys His Ile Pro Gln Pro Cys Val
   1 5 10
<210> 131
   <211> 10
   <212> PRT
   <213> Homo sapiens
<400> 131
   Pro Ser Cys Gly Arg Leu Ala Ser Cys Gly
   1 5 10
<210> 132
   <211> 10
   <212> PRT
   <213> Homo sapiens
<400> 132
   Pro Ser Cys Ser Glu Ser Ser Ile Cys Gln
   1 5 10
<210> 133
   <211> 10
   <212> PRT
   <213> Homo sapiens
<400> 133
   Pro Ser Cys Ser Glu Val Thr Ser Cys Pro
   1 5 10
<210> 134
   <211> 10
   <212> PRT
   <213> Homo sapiens
<400> 134
   Pro Ser Cys Ser Thr Ser Gly Thr Cys Gly
   1 5 10
<210> 135
   <211> 10
   <212> PRT
   <213> Homo sapiens
<400> 135
   Pro Ser Cys Ser Val Ser Ser Gly Cys Gln
   1 5 10
<210> 136
   <211> 10
   <212> PRT
   <213> Homo sapiens
<400> 136
   Pro Ser Cys Thr Glu Ser Asp Ser Cys Lys
   1 5 10
<210> 137
   <211> 10
   <212> PRT
   <213> Homo sapiens
<400> 137
   Pro Ser Cys Tyr Gln Thr Ser Ser Cys Gly
   1 5 10
<210> 138
   <211> 10
   <212> PRT
   <213> Homo sapiens
<400> 138
   Pro Thr Cys Phe Leu Leu Asn Ser Cys Gln
   1 5 10
<210> 139
   <211> 10
   <212> PRT
   <213> Homo sapiens
<400> 139
   Pro Thr Cys Ser Val Thr Ser Ser Cys Gln
   1 5 10
<210> 140
   <211> 10
   <212> PRT
   <213> Homo sapiens
<400> 140
   Pro Thr Cys Trp Leu Leu Asn Asn Cys His
   1 5 10
<210> 141
   <211> 10
   <212> PRT
   <213> Homo sapiens
<400> 141
   Pro Thr Cys Tyr Gln Arg Thr Ser Cys Val
   1 5 10
<210> 142
   <211> 10
   <212> PRT
   <213> Homo sapiens
<400> 142
   Pro Thr Cys Tyr Arg Arg Thr Ser Cys Val
   1 5 10
<210> 143
   <211> 10
   <212> PRT
   <213> Homo sapiens
<400> 143
   Pro Thr Cys Tyr Val Val Lys Arg Cys Pro
   1 5 10
<210> 144
   <211> 10
   <212> PRT
   <213> Homo sapiens
<400> 144
   Pro Val Cys Phe Glu Ala Thr Ile Cys Glu
   1 5 10
<210> 145
   <211> 10
   <212> PRT
   <213> Homo sapiens
<400> 145
   Pro Val Cys Phe Glu Ala Thr Val Cys Glu
   1 5 10
<210> 146
   <211> 10
   <212> PRT
   <213> Homo sapiens
<400> 146
   Pro Val Cys Ser Arg Pro Ala Ser Cys Ser
   1 5 10
<210> 147
   <211> 10
   <212> PRT
   <213> Homo sapiens
<400> 147
   Pro Val Cys Ser Trp Val Pro Ala Cys Ser
   1 5 10
<210> 148
   <211> 10
   <212> PRT
   <213> Homo sapiens
<400> 148
   Gln Thr Cys Asn Glu Ser Ser Tyr Cys Leu
   1 5 10
<210> 149
   <211> 10
   <212> PRT
   <213> Homo sapiens
<400> 149
   Gln Thr Cys Trp Glu Pro Thr Ser Cys Gln
   1 5 10
<210> 150
   <211> 10
   <212> PRT
   <213> Homo sapiens
<400> 150
   Ser Phe Cys Arg Leu Gly Tyr Gly Cys Gly
   1 5 10
<210> 151
   <211> 10
   <212> PRT
   <213> Homo sapiens
<400> 151
   Ser Phe Cys Arg Arg Gly Ser Gly Cys Gly
   1 5 10
<210> 152
   <211> 10
   <212> PRT
   <213> Homo sapiens
<400> 152
   Ser Leu Cys Gly Tyr Gly Tyr Gly Cys Gly
   1 5 10
<210> 153
   <211> 10
   <212> PRT
   <213> Homo sapiens
<400> 153
   Ser Leu Cys Ser Thr Glu Val Ser Cys Gly
   1 5 10
<210> 154
   <211> 10
   <212> PRT
   <213> Homo sapiens
<400> 154
   Ser Asn Cys Phe Gly Gln Leu Asn Cys Leu
   1 5 10
<210> 155
   <211> 10
   <212> PRT
   <213> Homo sapiens
<400> 155
   Ser Pro Cys Gly Gln Pro Thr Pro Cys Ser
   1 5 10
<210> 156
   <211> 10
   <212> PRT
   <213> Homo sapiens
<400> 156
   Ser Ser Cys Asp Gln Ser Ser Ser Cys Ala
   1 5 10
<210> 157
   <211> 10
   <212> PRT
   <213> Homo sapiens
<400> 157
   Ser Ser Cys Gly Gln Ser Ser Ser Cys Ala
   1 5 10
<210> 158
   <211> 10
   <212> PRT
   <213> Homo sapiens
<400> 158
   Ser Val Cys Pro Glu Pro Val Ser Cys Pro
   1 5 10
<210> 159
   <211> 10
   <212> PRT
   <213> Homo sapiens
<400> 159
   Thr Phe Cys Ser Phe Asp Lys Ser Cys Arg
   1 5 10
<210> 160
   <211> 10
   <212> PRT
   <213> Homo sapiens
<400> 160
   Thr Ile Cys Ser Ser Asp Lys Ser Cys Arg
   1 5 10
<210> 161
   <211> 10
   <212> PRT
   <213> Homo sapiens
<400> 161
   Thr Leu Cys Val Glu Ser Ser Pro Cys His
   1 5 10
<210> 162
   <211> 10
   <212> PRT
   <213> Homo sapiens
<400> 162
   Thr Pro Cys Tyr Gln Gln Ser Ser Cys Gln
   1 5 10
<210> 163
   <211> 10
   <212> PRT
   <213> Homo sapiens
<400> 163
   Val Thr Cys Ser Arg Gln Thr Thr Cys Ile
   1 5 10
<210> 164
   <211> 10
   <212> PRT
   <213> Homo sapiens
<400> 164
   Tyr Gly Cys Gly Tyr Gly Ser Gly Cys Gly
   1 5 10
<210> 165
   <211> 10
   <212> PRT
   <213> Homo sapiens
<400> 165
   Tyr Gly Cys Gly Tyr Gly Ser Gly Cys Arg
   1 5 10
<210> 166
   <211> 10
   <212> PRT
   <213> Homo sapiens
<400> 166
   Tyr Gly Cys Ile His Ser Thr His Cys Gly
   1 5 10
<210> 167
   <211> 10
   <212> PRT
   <213> Homo sapiens
<400> 167
   Ala Ala Cys Glu Pro Ser Ala Cys Gln Ser
   1 5 10
<210> 168
   <211> 10
   <212> PRT
   <213> Homo sapiens
<400> 168
   Ala Ala Cys Glu Pro Ser Pro Cys Gln Ser
   1 5 10
<210> 169
   <211> 10
   <212> PRT
   <213> Homo sapiens
<400> 169
   Ala Ala Cys Thr Met Ser Val Cys Ser Ser
   1 5 10
<210> 170
   <211> 10
   <212> PRT
   <213> Homo sapiens
<400> 170
   Ala Asp Cys Leu Gly Gly Ile Cys Leu Pro
   1 5 10
<210> 171
   <211> 10
   <212> PRT
   <213> Homo sapiens
<400> 171
   Ala Leu Cys Leu Pro Ser Ser Cys His Ser
   1 5 10
<210> 172
   <211> 10
   <212> PRT
   <213> Homo sapiens
<400> 172
   Ala Leu Cys Ser Pro Ser Thr Cys Gln Leu
   1 5 10
<210> 173
   <211> 10
   <212> PRT
   <213> Homo sapiens
<400> 173
   Ala Pro Cys Leu Ala Leu Val Cys Ala Pro
   1 5 10
<210> 174
   <211> 10
   <212> PRT
   <213> Homo sapiens
<400> 174
   Ala Pro Cys Leu Ser Leu Val Cys Thr Pro
   1 5 10
<210> 175
   <211> 10
   <212> PRT
   <213> Homo sapiens
<400> 175
   Ala Pro Cys Leu Thr Leu Val Cys Thr Pro
   1 5 10
<210> 176
   <211> 10
   <212> PRT
   <213> Homo sapiens
<400> 176
   Ala Pro Cys Val Ala Leu Leu Cys Arg Pro
   1               5                   10
<210> 177
   <211> 10
   <212> PRT
   <213> Homo sapiens
<400> 177
   Ala Ser Cys Gly Ser Leu Leu Cys Arg Pro
   1               5                   10
<210> 178
   <211> 10
   <212> PRT
   <213> Homo sapiens
<400> 178
   Ala Ser Cys Leu Ser Phe Leu Cys Arg Pro
   1               5                   10
<210> 179
   <211> 10
   <212> PRT
   <213> Homo sapiens
<400> 179
   Ala Ser Cys Val Ser Leu Leu Cys Arg Pro
   1               5                   10
<210> 180
   <211> 10
   <212> PRT
   <213> Homo sapiens
<400> 180
   Ala Val Cys Glu Pro Ser Pro Cys Gln Ser
   1               5                   10
<210> 181
   <211> 10
   <212> PRT
   <213> Homo sapiens
<400> 181
   Ala Val Cys Leu Pro Val Ser Cys Gln Ser
   1               5                   10
<210> 182
   <211> 10
   <212> PRT
   <213> Homo sapiens
<400> 182
   Ala Val Cys Val Pro Val Arg Cys Gln Ser
   1 5 10
<210> 183
   <211> 10
   <212> PRT
   <213> Homo sapiens
<400> 183
   Ala Val Cys Val Pro Val Ser Cys Gln Ser
   1 5 10
<210> 184
   <211> 10
   <212> PRT
   <213> Homo sapiens
<400> 184
   Asp Leu Cys Ser Pro Ser Thr Cys Gln Leu
   1 5 10
<210> 185
   <211> 10
   <212> PRT
   <213> Homo sapiens
<400> 185
   Asp Ser Cys Gly Ser Ser Ser Cys Gly Pro
   1 5 10
<210> 186
   <211> 10
   <212> PRT
   <213> Homo sapiens
<400> 186
   Asp Ser Cys Val Gln Ser Asn Cys Phe Pro
   1 5 10
<210> 187
   <211> 10
   <212> PRT
   <213> Homo sapiens
<400> 187
   Phe Asn Cys Ser Thr Arg Asn Cys Ser Ser
   1 5 10
<210> 188
   <211> 10
   <212> PRT
   <213> Homo sapiens
<400> 188
   Gly Gly Cys Gly Ser Tyr Gly Cys Ser Gln
   1 5 10
<210> 189
   <211> 10
   <212> PRT
   <213> Homo sapiens
<400> 189
   Gly Ser Cys Gly Phe Gly Ser Cys Tyr Gly
   1 5 10
<210> 190
   <211> 10
   <212> PRT
   <213> Homo sapiens
<400> 190
   Gly Ser Cys Ser Ser Arg Lys Cys Phe Ser
   1 5 10
<210> 191
   <211> 10
   <212> PRT
   <213> Homo sapiens
<400> 191
   Gly Val Cys Leu Pro Ser Thr Cys Pro His
   1 5 10
<210> 192
   <211> 10
   <212> PRT
   <213> Homo sapiens
<400> 192
   His Ser Cys Glu Gly Tyr Leu Cys Tyr Ser
   1 5 10
<210> 193
   <211> 10
   <212> PRT
   <213> Homo sapiens
<400> 193
   Ile Val Cys Ala Ala Pro Ser Cys Gln Ser
   1 5 10
<210> 194
   <211> 10
   <212> PRT
   <213> Homo sapiens
<400> 194
   Lys Thr Cys Ser Thr Thr Gly Cys Asp Pro
   1 5 10
<210> 195
   <211> 10
   <212> PRT
   <213> Homo sapiens
<400> 195
   Leu Ala Cys Val Ser Gln Pro Cys Gln Ser
   1 5 10
<210> 196
   <211> 10
   <212> PRT
   <213> Homo sapiens
<400> 196
   Leu Gly Cys Gly Tyr Gly Gly Cys Gly Tyr
   1 5 10
<210> 197
   <211> 10
   <212> PRT
   <213> Homo sapiens
<400> 197
   Leu Ser Cys Gly Ser Arg Ser Cys Ser Ser
   1 5 10
<210> 198
   <211> 10
   <212> PRT
   <213> Homo sapiens
<400> 198
   Leu Val Cys Thr Pro Val Ser Cys Val Ser
   1 5 10
<210> 199
   <211> 10
   <212> PRT
   <213> Homo sapiens
<400> 199
   Asn Gly Cys Gln Glu Thr Tyr Cys Glu Pro
   1 5 10
<210> 200
   <211> 10
   <212> PRT
   <213> Homo sapiens
<400> 200
   Asn Ser Cys Arg Ser Leu Ser Cys Gly Ser
   1 5 10
<210> 201
   <211> 10
   <212> PRT
   <213> Homo sapiens
<400> 201
   Pro Ala Cys Val Ile Ser Thr Cys Pro Arg
   1 5 10
<210> 202
   <211> 10
   <212> PRT
   <213> Homo sapiens
<400> 202
   Pro Gly Cys Leu Asn Gln Ser Cys Gly Ser
   1 5 10
<210> 203
   <211> 10
   <212> PRT
   <213> Homo sapiens
<400> 203
   Pro Pro Cys Gly Thr Ala Pro Cys Leu Thr
   1 5 10
<210> 204
   <211> 10
   <212> PRT
   <213> Homo sapiens
<400> 204
   Pro Pro Cys Thr Thr Ala Leu Cys Arg Pro
   1 5 10
<210> 205
   <211> 10
   <212> PRT
   <213> Homo sapiens
<400> 205
   Pro Pro Cys Tyr Leu Val Ser Cys Thr Pro
   1 5 10
<210> 206
   <211> 10
   <212> PRT
   <213> Homo sapiens
<400> 206
   Pro Arg Cys Thr Arg Pro Ile Cys Glu Pro
   1 5 10
<210> 207
   <211> 10
   <212> PRT
   <213> Homo sapiens
<400> 207
   Pro Ser Cys Pro Val Ser Ser Cys Ala Gln
   1 5 10
<210> 208
   <211> 10
   <212> PRT
   <213> Homo sapiens
<400> 208
   Pro Ser Cys Gln Pro Ser Val Cys Val Pro
   1 5 10
<210> 209
   <211> 10
   <212> PRT
   <213> Homo sapiens
<400> 209
   Pro Ser Cys Ser Val Ser Asn Cys Tyr Gln
   1 5 10
<210> 210
   <211> 10
   <212> PRT
   <213> Homo sapiens
<400> 210
   Pro Ser Cys Ser Val Ser Ser Cys Ala Gln
   1 5 10
<210> 211
   <211> 10
   <212> PRT
   <213> Homo sapiens
<400> 211
   Pro Ser Cys Thr Ser Val Leu Cys Arg Pro
   1 5 10
<210> 212
   <211> 10
   <212> PRT
   <213> Homo sapiens
<400> 212
   Pro Thr Cys Lys Ser Pro Ser Cys Glu Pro
   1 5 10
<210> 213
   <211> 10
   <212> PRT
   <213> Homo sapiens
<400> 213
   Pro Thr Cys Val Ile Ser Ser Cys Pro Arg
   1 5 10
<210> 214
   <211> 10
   <212> PRT
   <213> Homo sapiens
<400> 214
   Pro Thr Cys Val Ile Ser Thr Cys Pro Arg
   1 5 10
<210> 215
   <211> 10
   <212> PRT
   <213> Homo sapiens
<400> 215
   Pro Thr Cys Tyr Gln Thr Ile Cys Phe Arg
   1 5 10
<210> 216
   <211> 10
   <212> PRT
   <213> Homo sapiens
<400> 216
   Pro Val Cys Gly Gly Val Ser Cys His Thr
   1 5 10
<210> 217
   <211> 10
   <212> PRT
   <213> Homo sapiens
<400> 217
   Pro Val Cys Gly Arg Val Ser Cys His Thr
   1 5 10
<210> 218
   <211> 10
   <212> PRT
   <213> Homo sapiens
<400> 218
   Pro Val Cys Asn Lys Pro Val Cys Phe Val
   1 5 10
<210> 219
   <211> 10
   <212> PRT
   <213> Homo sapiens
<400> 219
   Pro Val Cys Pro Thr Pro Thr Cys Ser Val
   1 5 10
<210> 220
   <211> 10
   <212> PRT
   <213> Homo sapiens
<400> 220
   Pro Val Cys Arg Ser Thr Tyr Cys Val Pro
   1 5 10
<210> 221
   <211> 10
   <212> PRT
   <213> Homo sapiens
<400> 221
   Pro Val Cys Ser Lys Ser Val Cys Tyr Val
   1 5 10
<210> 222
   <211> 10
   <212> PRT
   <213> Homo sapiens
<400> 222
   Pro Val Cys Ser Arg Pro Ala Cys Tyr Ser
   1 5 10
<210> 223
   <211> 10
   <212> PRT
   <213> Homo sapiens
<400> 223
   Pro Val Cys Tyr Val Pro Thr Cys Ser Glu
   1 5 10
<210> 224
   <211> 10
   <212> PRT
   <213> Homo sapiens
<400> 224
   Gln Phe Cys Leu Ser Lys Ser Cys Gln Pro
   1 5 10
<210> 225
   <211> 10
   <212> PRT
   <213> Homo sapiens
<400> 225
   Arg Pro Cys Glu Arg Thr Ala Cys Gln Ser
   1 5 10
<210> 226
   <211> 10
   <212> PRT
   <213> Homo sapiens
<400> 226
   Arg Ser Cys Gln Thr Ser Phe Cys Gly Phe
   1 5 10
<210> 227
   <211> 10
   <212> PRT
   <213> Homo sapiens
<400> 227
   Arg Ser Cys Ser Ser Leu Gly Cys Gly Ser
   1 5 10
<210> 228
   <211> 10
   <212> PRT
   <213> Homo sapiens
<400> 228
   Arg Ser Cys Tyr Ser Val Gly Cys Gly Ser
   1               5                   10
<210> 229
   <211> 10
   <212> PRT
   <213> Homo sapiens
<400> 229
   Arg Val Cys Leu Pro Gly Ser Cys Asp Ser
   1               5                   10
<210> 230
   <211> 10
   <212> PRT
   <213> Homo sapiens
<400> 230
   Ser Phe Cys Gly Phe Pro Ser Cys Ser Thr
   1               5                   10
<210> 231
   <211> 10
   <212> PRT
   <213> Homo sapiens
<400> 231
   Ser Phe Cys Gly Tyr Pro Ser Cys Ser Thr
   1               5                   10
<210> 232
   <211> 10
   <212> PRT
   <213> Homo sapiens
<400> 232
   Ser Gly Cys Asp Pro Ala Ser Cys Gln Pro
   1               5                   10
<210> 233
   <211> 10
   <212> PRT
   <213> Homo sapiens
<400> 233
   Ser Gly Cys Gly Gly Ser Gly Cys Gly Gly
   1               5                   10
<210> 234
   <211> 10
   <212> PRT
   <213> Homo sapiens
<400> 234
   Ser Gly Cys Gln Pro Ser Ser Cys Leu Ala
   1               5                   10
<210> 235
   <211> 10
   <212> PRT
   <213> Homo sapiens
<400> 235
   Ser His Cys Gln Pro Pro His Cys Gln Leu
   1               5                   10
<210> 236
   <211> 10
   <212> PRT
   <213> Homo sapiens
<400> 236
   Ser Ile Cys Gln Pro Ala Thr Cys Val Ala
   1               5                   10
<210> 237
   <211> 10
   <212> PRT
   <213> Homo sapiens
<400> 237
   Ser Leu Cys Val Pro Val Ser Cys Arg Pro
   1               5                   10
<210> 238
   <211> 10
   <212> PRT
   <213> Homo sapiens
<400> 238
   Ser Asn Cys Leu Pro Thr Ser Cys Gln Pro
   1               5                   10
<210> 239
   <211> 10
   <212> PRT
   <213> Homo sapiens
<400> 239
   Ser Pro Cys Leu Val Ser Ser Cys Gln Pro
   1               5                   10
<210> 240
   <211> 10
   <212> PRT
   <213> Homo sapiens
<400> 240
   Ser Pro Cys Gln Gln Ser Ser Cys Gln Glu
   1 5 10
<210> 241
   <211> 10
   <212> PRT
   <213> Homo sapiens
<400> 241
   Ser Pro Cys Gln Gln Ser Tyr Cys Val Pro
   1 5 10
<210> 242
   <211> 10
   <212> PRT
   <213> Homo sapiens
<400> 242
   Ser Pro Cys Ser Pro Ala Val Cys Val Ser
   1 5 10
<210> 243
   <211> 10
   <212> PRT
   <213> Homo sapiens
<400> 243
   Ser Arg Cys Gln Gln Pro Ser Cys Gln Pro
   1 5 10
<210> 244
   <211> 10
   <212> PRT
   <213> Homo sapiens
<400> 244
   Ser Arg Cys Tyr Arg Pro His Cys Gly Gln
   1 5 10
<210> 245
   <211> 10
   <212> PRT
   <213> Homo sapiens
<400> 245
   Ser Ser Cys Ala Pro Ile Tyr Cys Arg Arg
   1 5 10
<210> 246
   <211> 10
   <212> PRT
   <213> Homo sapiens
<400> 246
   Ser Ser Cys Ala Pro Val Tyr Cys Arg Arg
   1 5 10
<210> 247
   <211> 10
   <212> PRT
   <213> Homo sapiens
<400> 247
   Ser Ser Cys Gly Lys Gly Gly Cys Gly Ser
   1 5 10
<210> 248
   <211> 10
   <212> PRT
   <213> Homo sapiens
<400> 248
   Ser Ser Cys Gly Lys Arg Gly Cys Gly Ser
   1 5 10
<210> 249
   <211> 10
   <212> PRT
   <213> Homo sapiens
<400> 249
   Ser Ser Cys Leu Pro Val Ser Cys Arg Pro
   1 5 10
<210> 250
   <211> 10
   <212> PRT
   <213> Homo sapiens
<400> 250
   Ser Ser Cys Gln Pro Ala Tyr Cys Thr Ser
   1 5 10
<210> 251
   <211> 10
   <212> PRT
   <213> Homo sapiens
<400> 251
   Ser Ser Cys Gln Pro Ser Tyr Cys Arg Gln
   1               5                   10
<210> 252
   <211> 10
   <212> PRT
   <213> Homo sapiens
<400> 252
   Ser Ser Cys Gln Pro Val Val Cys Glu Pro
   1               5                   10
<210> 253
   <211> 10
   <212> PRT
   <213> Homo sapiens
<400> 253
   Ser Ser Cys Thr Ala Val Val Cys Arg Pro
   1               5                   10
<210> 254
   <211> 10
   <212> PRT
   <213> Homo sapiens
<400> 254
   Ser Ser Cys Tyr Gln Pro Phe Cys Arg Ser
   1               5                   10
<210> 255
   <211> 10
   <212> PRT
   <213> Homo sapiens
<400> 255
   Ser Ser Cys Tyr Arg Pro Ile Cys Gly Ser
   1               5                   10
<210> 256
   <211> 10
   <212> PRT
   <213> Homo sapiens
<400> 256
   Ser Ser Cys Tyr Arg Pro Thr Cys Gly Ser
   1               5                   10
<210> 257
   <211> 10
   <212> PRT
   <213> Homo sapiens
<400> 257
   Ser Val Cys Met Ser Gly Ser Cys Gln Ala
   1 5 10
<210> 258
   <211> 10
   <212> PRT
   <213> Homo sapiens
<400> 258
   Ser Val Cys Ser Asp Gln Gly Cys Asp Gln
   1 5 10
<210> 259
   <211> 10
   <212> PRT
   <213> Homo sapiens
<400> 259
   Ser Val Cys Ser Asp Gln Gly Cys Gly Leu
   1 5 10
<210> 260
   <211> 10
   <212> PRT
   <213> Homo sapiens
<400> 260
   Ser Val Cys Ser Asp Gln Gly Cys Gly Gln
   1 5 10
<210> 261
   <211> 10
   <212> PRT
   <213> Homo sapiens
<400> 261
   Ser Val Cys Ser Asp Gln Gly Cys Ser Gln
   1 5 10
<210> 262
   <211> 10
   <212> PRT
   <213> Homo sapiens
<400> 262
   Ser Val Cys Ser Asp Gln Ser Cys Gly Gln
   1 5 10
<210> 263
   <211> 10
   <212> PRT
   <213> Homo sapiens
<400> 263
   Ser Val Cys Ser His Gln Gly Cys Gly Gln
   1 5 10
<210> 264
   <211> 10
   <212> PRT
   <213> Homo sapiens
<400> 264
   Ser Val Cys Ser His Gln Gly Cys Gly Arg
   1 5 10
<210> 265
   <211> 10
   <212> PRT
   <213> Homo sapiens
<400> 265
   Ser Val Cys Val Pro Val Ser Cys Arg Pro
   1 5 10
<210> 266
   <211> 10
   <212> PRT
   <213> Homo sapiens
<400> 266
   Ser Tyr Cys Arg Gln Ala Ser Cys Val Ser
   1 5 10
<210> 267
   <211> 10
   <212> PRT
   <213> Homo sapiens
<400> 267
   Thr Ala Cys Glu Pro Ser Ala Cys Gln Ser
   1 5 10
<210> 268
   <211> 10
   <212> PRT
   <213> Homo sapiens
<400> 268
   Thr Ile Cys Thr Ala Ser Pro Cys Gln Pro
   1 5 10
<210> 269
   <211> 10
   <212> PRT
   <213> Homo sapiens
<400> 269
   Thr Ser Cys Pro Glu Thr Ser Cys Leu Pro
   1 5 10
<210> 270
   <211> 10
   <212> PRT
   <213> Homo sapiens
<400> 270
   Thr Ser Cys Gln Met Thr Asn Cys Glu Gln
   1 5 10
<210> 271
   <211> 10
   <212> PRT
   <213> Homo sapiens
<400> 271
   Thr Ser Cys Gln Pro Val His Cys Glu Thr
   1 5 10
<210> 272
   <211> 10
   <212> PRT
   <213> Homo sapiens
<400> 272
   Thr Ser Cys Gln Pro Val Leu Cys Lys Ser
   1 5 10
<210> 273
   <211> 10
   <212> PRT
   <213> Homo sapiens
<400> 273
   Thr Ser Cys Gln Pro Val Leu Cys Val Pro
   1 5 10
<210> 274
   <211> 10
   <212> PRT
   <213> Homo sapiens
<400> 274
   Thr Ser Cys Val Gly Phe Val Cys Gln Pro
   1 5 10
<210> 275
   <211> 10
   <212> PRT
   <213> Homo sapiens
<400> 275
   Thr Ser Cys Val Ser Asn Pro Cys Gln Val
   1 5 10
<210> 276
   <211> 10
   <212> PRT
   <213> Homo sapiens
<400> 276
   Thr Thr Cys Phe Gln Pro Thr Cys Val Ser
   1 5 10
<210> 277
   <211> 10
   <212> PRT
   <213> Homo sapiens
<400> 277
   Thr Thr Cys Phe Gln Pro Thr Cys Val Thr
   1 5 10
<210> 278
   <211> 10
   <212> PRT
   <213> Homo sapiens
<400> 278
   Thr Thr Cys Phe Gln Pro Thr Cys Val Tyr
   1 5 10
<210> 279
   <211> 10
   <212> PRT
   <213> Homo sapiens
<400> 279
   Thr Thr Cys Ile Ser Asn Pro Cys Ser Thr
   1 5 10
<210> 280
   <211> 10
   <212> PRT
   <213> Homo sapiens
<400> 280
   Thr Trp Cys Gln Gly Ser Ser Cys Gln Pro
   1 5 10
<210> 281
   <211> 10
   <212> PRT
   <213> Homo sapiens
<400> 281
   Val Gly Cys Gln Ser Ser Val Cys Val Pro
   1 5 10
<210> 282
   <211> 10
   <212> PRT
   <213> Homo sapiens
<400> 282
   Val Pro Cys Gln Pro Ser Thr Cys Val Phe
   1 5 10
<210> 283
   <211> 10
   <212> PRT
   <213> Homo sapiens
<400> 283
   Val Ser Cys Glu Pro Ser Pro Cys Gln Ser
   1 5 10
<210> 284
   <211> 10
   <212> PRT
   <213> Homo sapiens
<400> 284
   Val Ser Cys Gly Gly Pro Ile Cys Leu Pro
   1 5 10
<210> 285
   <211> 10
   <212> PRT
   <213> Homo sapiens
<400> 285
   Val Ser Cys Lys Pro Val Leu Cys Val Ala
   1 5 10
<210> 286
   <211> 10
   <212> PRT
   <213> Homo sapiens
<400> 286
   Val Ser Cys Pro Ser Thr Ser Cys Arg Pro
   1 5 10
<210> 287
   <211> 10
   <212> PRT
   <213> Homo sapiens
<400> 287
   Val Ser Cys Gln Ser Ser Val Cys Met Pro
   1 5 10
<210> 288
   <211> 10
   <212> PRT
   <213> Homo sapiens
<400> 288
   Val Ser Cys Thr Arg Ile Val Cys Val Ala
   1 5 10
<210> 289
   <211> 10
   <212> PRT
   <213> Homo sapiens
<400> 289
   Val Thr Cys Glu Pro Ser Pro Cys Gln Ser
   1 5 10
<210> 290
   <211> 10
   <212> PRT
   <213> Homo sapiens
<400> 290
   Val Thr Cys Gln Thr Thr Val Cys Arg Pro
   1 5 10
<210> 291
   <211> 10
   <212> PRT
   <213> Homo sapiens
<400> 291
   Tyr Gly Cys Gly Tyr Glu Gly Cys Arg Tyr
   1 5 10
<210> 292
   <211> 10
   <212> PRT
   <213> Homo sapiens
<400> 292
   Ala Gly Ser Cys Gln Pro Ser Cys Ser Glu
   1               5                   10
<210> 293
   <211> 10
   <212> PRT
   <213> Homo sapiens
<400> 293
   Ala Leu Leu Cys Arg Pro Leu Cys Gly Val
   1               5                   10
<210> 294
   <211> 10
   <212> PRT
   <213> Homo sapiens
<400> 294
   Ala Leu Val Cys Glu Pro Val Cys Leu Arg
   1               5                   10
<210> 295
   <211> 10
   <212> PRT
   <213> Homo sapiens
<400> 295
   Ala Thr Ile Cys Glu Pro Ser Cys Ser Val
   1               5                   10
<210> 296
   <211> 10
   <212> PRT
   <213> Homo sapiens
<400> 296
   Ala Thr Thr Cys Glu Pro Ser Cys Ser Val
   1               5                   10
<210> 297
   <211> 10
   <212> PRT
   <213> Homo sapiens
<400> 297
   Ala Thr Val Cys Glu Pro Ser Cys Ser Val
   1               5                   10
<210> 298
   <211> 10
   <212> PRT
   <213> Homo sapiens
<400> 298
   Glu Gly Thr Cys Leu Pro Pro Cys Tyr Leu
   1               5                   10
<210> 299
   <211> 10
   <212> PRT
   <213> Homo sapiens
<400> 299
   Phe Ser Thr Cys Arg Pro Ser Cys Ser Gly
   1               5                   10
<210> 300
   <211> 10
   <212> PRT
   <213> Homo sapiens
<400> 300
   Gly Phe Val Cys Gln Pro Met Cys Ser His
   1               5                   10
<210> 301
   <211> 10
   <212> PRT
   <213> Homo sapiens
<400> 301
   Gly Leu Asp Cys Gly Tyr Gly Cys Gly Tyr
   1               5                   10
<210> 302
   <211> 10
   <212> PRT
   <213> Homo sapiens
<400> 302
   Gly Leu Gly Cys Gly Tyr Gly Cys Gly Tyr
   1               5                   10
<210> 303
   <211> 10
   <212> PRT
   <213> Homo sapiens
<400> 303
   Gly Leu Gly Cys Ser Tyr Gly Cys Gly His
   1 5 10
<210> 304
   <211> 10
   <212> PRT
   <213> Homo sapiens
<400> 304
   Gly Leu Gly Cys Ser Tyr Gly Cys Gly Leu
   1 5 10
<210> 305
   <211> 10
   <212> PRT
   <213> Homo sapiens
<400> 305
   Gly Ser Gly Cys Gly Tyr Gly Cys Gly Tyr
   1 5 10
<210> 306
   <211> 10
   <212> PRT
   <213> Homo sapiens
<400> 306
   Gly Thr Gly Cys Gly Tyr Gly Cys Gly Tyr
   1 5 10
<210> 307
   <211> 10
   <212> PRT
   <213> Homo sapiens
<400> 307
   Gly Val Ser Cys His Thr Thr Cys Tyr Arg
   1 5 10
<210> 308
   <211> 10
   <212> PRT
   <213> Homo sapiens
<400> 308
   Gly Tyr Ala Cys Asn Phe Pro Cys Ser Tyr
   1 5 10
<210> 309
   <211> 10
   <212> PRT
   <213> Homo sapiens
<400> 309
   Gly Tyr Gly Cys Gly Tyr Gly Cys Gly Phe
   1               5                   10
<210> 310
   <211> 10
   <212> PRT
   <213> Homo sapiens
<400> 310
   His Ser Pro Cys Gln Ala Ser Cys Tyr Val
   1               5                   10
<210> 311
   <211> 10
   <212> PRT
   <213> Homo sapiens
<400> 311
   His Thr Ser Cys Ser Pro Ala Cys Gln Pro
   1               5                   10
<210> 312
   <211> 10
   <212> PRT
   <213> Homo sapiens
<400> 312
   His Thr Ser Cys Ser Ser Gly Cys Gln Pro
   1               5                   10
<210> 313
   <211> 10
   <212> PRT
   <213> Homo sapiens
<400> 313
   Ile Arg Trp Cys His Pro Asp Cys His Val
   1               5                   10
<210> 314
   <211> 10
   <212> PRT
   <213> Homo sapiens
<400> 314
   Ile Arg Trp Cys Arg Pro Asp Cys Arg Val
   1               5                   10
<210> 315
   <211> 10
   <212> PRT
   <213> Homo sapiens
<400> 315
   Ile Ser Ser Cys Gly Thr Gly Cys Gly Ile
   1 5 10
<210> 316
   <211> 10
   <212> PRT
   <213> Homo sapiens
<400> 316
   Lys Gly Gly Cys Gly Ser Gly Cys Gly Gly
   1 5 10
<210> 317
   <211> 10
   <212> PRT
   <213> Homo sapiens
<400> 317
   Lys Gly Gly Cys Gly Ser Ser Cys Ser Gln
   1 5 10
<210> 318
   <211> 10
   <212> PRT
   <213> Homo sapiens
<400> 318
   Leu Val Thr Cys Gln Asp Ser Cys Gly Ser
   1 5 10
<210> 319
   <211> 10
   <212> PRT
   <213> Homo sapiens
<400> 319
   Leu Val Thr Cys Gln Glu Ser Cys Gln Pro
   1 5 10
<210> 320
   <211> 10
   <212> PRT
   <213> Homo sapiens
<400> 320
   Met Ser Ile Cys Ser Ser Ala Cys Thr Asp
   1 5 10
<210> 321
   <211> 10
   <212> PRT
   <213> Homo sapiens
<400> 321
   Met Ser Ile Cys Ser Ser Ala Cys Thr Asn
   1               5                   10
<210> 322
   <211> 10
   <212> PRT
   <213> Homo sapiens
<400> 322
   Met Ser Val Cys Ser Ser Ala Cys Ser Asp
   1               5                   10
<210> 323
   <211> 10
   <212> PRT
   <213> Homo sapiens
<400> 323
   Pro Ala Ile Cys Glu Pro Ser Cys Ser Val
   1               5                   10
<210> 324
   <211> 10
   <212> PRT
   <213> Homo sapiens
<400> 324
   Pro Ala Ser Cys Gln Lys Ser Cys Tyr Arg
   1               5                   10
<210> 325
   <211> 10
   <212> PRT
   <213> Homo sapiens
<400> 325
   Pro Ile Tyr Cys Arg Arg Thr Cys Tyr His
   1               5                   10
<210> 326
   <211> 10
   <212> PRT
   <213> Homo sapiens
<400> 326
   Pro Asn Ser Cys Gln Thr Leu Cys Val Glu
   1               5                   10
<210> 327
   <211> 10
   <212> PRT
   <213> Homo sapiens
<400> 327
   Pro Gln Pro Cys Val Pro Thr Cys Phe Leu
   1 5 10
<210> 328
   <211> 10
   <212> PRT
   <213> Homo sapiens
<400> 328
   Pro Ser Ala Cys Gln Ser Gly Cys Thr Ser
   1 5 10
<210> 329
   <211> 10
   <212> PRT
   <213> Homo sapiens
<400> 329
   Pro Ser Pro Cys Glu Pro Ser Cys Ser Glu
   1 5 10
<210> 330
   <211> 10
   <212> PRT
   <213> Homo sapiens
<400> 330
   Pro Ser Pro Cys Gln Ala Ser Cys Tyr Ile
   1 5 10
<210> 331
   <211> 10
   <212> PRT
   <213> Homo sapiens
<400> 331
   Pro Ser Pro Cys Gln Ser Gly Cys Ile Ser
   1 5 10
<210> 332
   <211> 10
   <212> PRT
   <213> Homo sapiens
<400> 332
   Pro Ser Pro Cys Gln Ser Gly Cys Thr Asp
   1 5 10
<210> 333
   <211> 10
   <212> PRT
   <213> Homo sapiens
<400> 333
   Pro Ser Pro Cys Gln Ser Gly Cys Thr Ser
   1 5 10
<210> 334
   <211> 10
   <212> PRT
   <213> Homo sapiens
<400> 334
   Pro Thr Ala Cys Gln Pro Thr Cys Tyr Gln
   1 5 10
<210> 335
   <211> 10
   <212> PRT
   <213> Homo sapiens
<400> 335
   Pro Thr Ala Cys Gln Pro Thr Cys Tyr Arg
   1 5 10
<210> 336
   <211> 10
   <212> PRT
   <213> Homo sapiens
<400> 336
   Pro Thr Pro Cys Ser Thr Thr Cys Arg Thr
   1 5 10
<210> 337
   <211> 10
   <212> PRT
   <213> Homo sapiens
<400> 337
   Pro Thr Ser Cys Gln Lys Ser Cys Tyr Arg
   1 5 10
<210> 338
   <211> 10
   <212> PRT
   <213> Homo sapiens
<400> 338
   Pro Thr Ser Cys Gln Pro Ser Cys Glu Ser
   1 5 10
<210> 339
   <211> 10
   <212> PRT
   <213> Homo sapiens
<400> 339
   Pro Thr Ser Cys Gln Thr Ser Cys Thr Leu
   1 5 10
<210> 340
   <211> 10
   <212> PRT
   <213> Homo sapiens
<400> 340
   Pro Val Ile Cys Glu Pro Ser Cys Ser Val
   1 5 10
<210> 341
   <211> 10
   <212> PRT
   <213> Homo sapiens
<400> 341
   Pro Val Ser Cys Val Pro Val Cys Ser Gly
   1 5 10
<210> 342
   <211> 10
   <212> PRT
   <213> Homo sapiens
<400> 342
   Pro Val Thr Cys Val Pro Arg Cys Thr Arg
   1 5 10
<210> 343
   <211> 10
   <212> PRT
   <213> Homo sapiens
<400> 343
   Pro Val Tyr Cys Arg Arg Thr Cys Tyr His
   1 5 10
<210> 344
   <211> 10
   <212> PRT
   <213> Homo sapiens
<400> 344
   Pro Val Tyr Cys Arg Arg Thr Cys Tyr Tyr
   1 5 10
<210> 345
   <211> 10
   <212> PRT
   <213> Homo sapiens
<400> 345
   Pro Val Tyr Cys Val Pro Val Cys Ser Gly
   1 5 10
<210> 346
   <211> 10
   <212> PRT
   <213> Homo sapiens
<400> 346
   Gln Pro Gly Cys Glu Ser Pro Cys Glu Pro
   1 5 10
<210> 347
   <211> 10
   <212> PRT
   <213> Homo sapiens
<400> 347
   Gln Gln Ser Cys Val Ser Ser Cys Arg Arg
   1 5 10
<210> 348
   <211> 10
   <212> PRT
   <213> Homo sapiens
<400> 348
   Gln Thr Ser Cys Gly Ser Ser Cys Gly Gln
   1 5 10
<210> 349
   <211> 10
   <212> PRT
   <213> Homo sapiens
<400> 349
   Gln Thr Thr Cys His Pro Ser Cys Gly Met
   1 5 10
<210> 350
   <211> 10
   <212> PRT
   <213> Homo sapiens
<400> 350
   Gln Thr Thr Cys Arg Pro Ser Cys Gly Val
   1 5 10
<210> 351
   <211> 10
   <212> PRT
   <213> Homo sapiens
<400> 351
   Arg Gly Gly Cys Gly Ser Gly Cys Gly Gly
   1 5 10
<210> 352
   <211> 10
   <212> PRT
   <213> Homo sapiens
<400> 352
   Arg Leu Ala Cys Tyr Ser Leu Cys Ser Gly
   1 5 10
<210> 353
   <211> 10
   <212> PRT
   <213> Homo sapiens
<400> 353
   Arg Pro Ala Cys Tyr Arg Pro Cys Tyr Ser
   1 5 10
<210> 354
   <211> 10
   <212> PRT
   <213> Homo sapiens
<400> 354
   Arg Pro Phe Cys Phe Arg Arg Cys Tyr Ser
   1 5 10
<210> 355
   <211> 10
   <212> PRT
   <213> Homo sapiens
<400> 355
   Arg Pro Ile Cys Arg Pro Ile Cys Ser Gly
   1 5 10
<210> 356
   <211> 10
   <212> PRT
   <213> Homo sapiens
<400> 356
   Arg Pro Leu Cys Tyr Arg Arg Cys Tyr Ser
   1 5 10
<210> 357
   <211> 10
   <212> PRT
   <213> Homo sapiens
<400> 357
   Arg Ser Pro Cys Gln Ala Ser Cys Tyr Val
   1 5 10
<210> 358
   <211> 10
   <212> PRT
   <213> Homo sapiens
<400> 358
   Arg Val Ser Cys His Thr Thr Cys Tyr Arg
   1 5 10
<210> 359
   <211> 10
   <212> PRT
   <213> Homo sapiens
<400> 359
   Ser Ala Ile Cys Arg Pro Thr Cys Pro Arg
   1 5 10
<210> 360
   <211> 10
   <212> PRT
   <213> Homo sapiens
<400> 360
   Ser Asp Ser Cys Lys Arg Asp Cys Lys Lys
   1 5 10
<210> 361
   <211> 10
   <212> PRT
   <213> Homo sapiens
<400> 361
   Ser Glu Gly Cys Gly Ser Gly Cys Gly Gly
   1 5 10
<210> 362
   <211> 10
   <212> PRT
   <213> Homo sapiens
<400> 362
   Ser Phe Leu Cys Arg Pro Ala Cys Ser Arg
   1 5 10
<210> 363
   <211> 10
   <212> PRT
   <213> Homo sapiens
<400> 363
   Ser Gly Gly Cys Gly Ser Gly Cys Gly Gly
   1 5 10
<210> 364
   <211> 10
   <212> PRT
   <213> Homo sapiens
<400> 364
   Ser Gly Gly Cys Gly Ser Ser Cys Gly Gly
   1 5 10
<210> 365
   <211> 10
   <212> PRT
   <213> Homo sapiens
<400> 365
   Ser Gly Ser Cys Gln Ala Ala Cys Gly Gln
   1 5 10
<210> 366
   <211> 10
   <212> PRT
   <213> Homo sapiens
<400> 366
   Ser Leu Leu Cys His Pro Val Cys Lys Ser
   1 5 10
<210> 367
   <211> 10
   <212> PRT
   <213> Homo sapiens
<400> 367
   Ser Leu Leu Cys His Pro Val Cys Arg Ser
   1 5 10
<210> 368
   <211> 10
   <212> PRT
   <213> Homo sapiens
<400> 368
   Ser Leu Leu Cys Arg Pro Ala Cys Ser Pro
   1 5 10
<210> 369
   <211> 10
   <212> PRT
   <213> Homo sapiens
<400> 369
   Ser Leu Leu Cys Arg Pro Ala Cys Ser Arg
   1 5 10
<210> 370
   <211> 10
   <212> PRT
   <213> Homo sapiens
<400> 370
   Ser Leu Leu Cys Arg Pro Ile Cys Arg Pro
   1 5 10
<210> 371
   <211> 10
   <212> PRT
   <213> Homo sapiens
<400> 371
   Ser Leu Leu Cys Arg Pro Met Cys Ser Arg
   1 5 10
<210> 372
   <211> 10
   <212> PRT
   <213> Homo sapiens
<400> 372
   Ser Leu Leu Cys Arg Pro Thr Cys Ser Arg
   1 5 10
<210> 373
   <211> 10
   <212> PRT
   <213> Homo sapiens
<400> 373
   Ser Leu Leu Cys Arg Pro Val Cys Gln Pro
   1 5 10
<210> 374
   <211> 10
   <212> PRT
   <213> Homo sapiens
<400> 374
   Ser Leu Leu Cys Arg Pro Val Cys Arg Pro
   1 5 10
<210> 375
   <211> 10
   <212> PRT
   <213> Homo sapiens
<400> 375
   Ser Leu Leu Cys Arg Pro Val Cys Arg Ser
   1 5 10
<210> 376
   <211> 10
   <212> PRT
   <213> Homo sapiens
<400> 376
   Ser Leu Leu Cys Arg Pro Val Cys Ser Arg
   1 5 10
<210> 377
   <211> 10
   <212> PRT
   <213> Homo sapiens
<400> 377
   Ser Asn Pro Cys Gln Val Thr Cys Ser Arg
   1 5 10
<210> 378
   <211> 10
   <212> PRT
   <213> Homo sapiens
<400> 378
   Ser Arg Gly Cys Gly Ser Gly Cys Gly Gly
   1 5 10
<210> 379
   <211> 10
   <212> PRT
   <213> Homo sapiens
<400> 379
   Ser Arg Ser Cys Gln Ser Pro Cys Tyr Arg
   1 5 10
<210> 380
   <211> 10
   <212> PRT
   <213> Homo sapiens
<400> 380
   Ser Arg Ser Cys Gln Ser Ser Cys Tyr Arg
   1 5 10
<210> 381
   <211> 10
   <212> PRT
   <213> Homo sapiens
<400> 381
   Ser Ser Gly Cys Gly Tyr Gly Cys Gly Tyr
   1 5 10
<210> 382
   <211> 10
   <212> PRT
   <213> Homo sapiens
<400> 382
   Ser Ser Gly Cys Pro Met Ala Cys Pro Gly
   1 5 10
<210> 383
   <211> 10
   <212> PRT
   <213> Homo sapiens
<400> 383
   Ser Ser Ile Cys Gln Pro Ile Cys Ser Glu
   1 5 10
<210> 384
   <211> 10
   <212> PRT
   <213> Homo sapiens
<400> 384
   Ser Ser Pro Cys His Thr Ser Cys Tyr Tyr
   1 5 10
<210> 385
   <211> 10
   <212> PRT
   <213> Homo sapiens
<400> 385
   Ser Ser Pro Cys Gln Pro Thr Cys Tyr Val
   1               5                   10
<210> 386
   <211> 10
   <212> PRT
   <213> Homo sapiens
<400> 386
   Ser Ser Pro Cys Gln Gln Ser Cys Tyr Val
   1               5                   10
<210> 387
   <211> 10
   <212> PRT
   <213> Homo sapiens
<400> 387
   Ser Ser Pro Cys Gln Thr Ser Cys Tyr Arg
   1               5                   10
<210> 388
   <211> 10
   <212> PRT
   <213> Homo sapiens
<400> 388
   Ser Ser Ser Cys Gln Gln Ser Cys Arg Val
   1               5                   10
<210> 389
   <211> 10
   <212> PRT
   <213> Homo sapiens
<400> 389
   Ser Thr Val Cys Gln Pro Ala Cys Gly Val
   1               5                   10
<210> 390
   <211> 10
   <212> PRT
   <213> Homo sapiens
<400> 390
   Thr Asp Asn Cys Gln Glu Thr Cys Gly Glu
   1 5 10
<210> 391
   <211> 10
   <212> PRT
   <213> Homo sapiens
<400> 391
   Thr Gln Pro Cys Tyr Glu Pro Cys Leu Pro
   1 5 10
<210> 392
   <211> 10
   <212> PRT
   <213> Homo sapiens
<400> 392
   Thr Ser Ser Cys Gly Thr Gly Cys Gly Ile
   1 5 10
<210> 393
   <211> 10
   <212> PRT
   <213> Homo sapiens
<400> 393
   Thr Ser Ser Cys Gln Pro Ser Cys Gly Arg
   1 5 10
<210> 394
   <211> 10
   <212> PRT
   <213> Homo sapiens
<400> 394
   Thr Ser Ser Cys Thr Thr Pro Cys Tyr Gln
   1 5 10
<210> 395
   <211> 10
   <212> PRT
   <213> Homo sapiens
<400> 395
   Thr Ser Val Cys Leu Pro Gly Cys Leu Asn
   1 5 10
<210> 396
   <211> 10
   <212> PRT
   <213> Homo sapiens
<400> 396
   Thr Thr Val Cys Leu Pro Gly Cys Leu Asn
   1 5 10
<210> 397
   <211> 10
   <212> PRT
   <213> Homo sapiens
<400> 397
   Val Ala Asn Cys Gln Ala Pro Cys Ser Thr
   1 5 10
<210> 398
   <211> 10
   <212> PRT
   <213> Homo sapiens
<400> 398
   Val Asp Asp Cys Pro Glu Ser Cys Trp Pro
   1 5 10
<210> 399
   <211> 10
   <212> PRT
   <213> Homo sapiens
<400> 399
   Val Lys Arg Cys Pro Ser Val Cys Pro Glu
   1 5 10
<210> 400
   <211> 10
   <212> PRT
   <213> Homo sapiens
<400> 400
   Val Ser Ser Cys Gln Pro Ser Cys Ser Glu
   1 5 10
<210> 401
   <211> 10
   <212> PRT
   <213> Homo sapiens
<400> 401
   Tyr Glu Gly Cys Arg Tyr Gly Cys Gly His
   1 5 10
<210> 402
   <211> 10
   <212> PRT
   <213> Homo sapiens
<400> 402
   Tyr Gly Arg Cys Arg His Gly Cys His Ser
   1               5                   10
<210> 403
   <211> 10
   <212> PRT
   <213> Homo sapiens
<400> 403
   Tyr Gly Tyr Cys Arg Pro Ser Cys Tyr Gly
   1               5                   10
<210> 404
   <211> 10
   <212> PRT
   <213> Homo sapiens
<400> 404
   Tyr Arg Asp Cys Gln Lys Thr Cys Trp Glu
   1               5                   10
<210> 405
   <211> 10
   <212> PRT
   <213> Homo sapiens
<400> 405
   Tyr Arg Gly Cys Gln Glu Ile Cys Trp Glu
   1               5                   10
<210> 406
   <211> 10
   <212> PRT
   <213> Homo sapiens
<400> 406
   Tyr Arg Gly Cys Gln Glu Thr Cys Trp Arg
   1               5                   10
<210> 407
   <211> 10
   <212> PRT
   <213> Homo sapiens
<400> 407
   Tyr Arg Gly Cys Gln Gln Thr Cys Trp Glu
   1               5                   10
<210> 408
   <211> 10
   <212> PRT
   <213> Homo sapiens
<400> 408
   Tyr Arg Ser Cys Arg Pro Ser Cys Tyr Gly
   1 5 10
<210> 409
   <211> 10
   <212> PRT
   <213> Homo sapiens
<400> 409
   Gly Gly Val Cys Gly Pro Ser Pro Pro Cys
   1 5 10
<210> 410
   <211> 10
   <212> PRT
   <213> Homo sapiens
<400> 410
   Gly Val Cys Gly Pro Ser Pro Pro Cys Ile
   1 5 10
<210> 411
   <211> 10
   <212> PRT
   <213> Homo sapiens
<400> 411
   Val Cys Gly Pro Ser Pro Pro Cys Ile Thr
   1 5 10
<210> 412
   <211> 10
   <212> PRT
   <213> Homo sapiens
<400> 412
   Cys Gly Pro Ser Pro Pro Cys Ile Thr Thr
   1 5 10
<210> 413
   <211> 10
   <212> PRT
   <213> Homo sapiens
<400> 413
   Cys Ala Pro Ile Tyr Cys Arg Arg Thr Cys
   1 5 10
<210> 414
   <211> 10
   <212> PRT
   <213> Homo sapiens
<400> 414
   Cys Ala Pro Ser Pro Cys Gln Ala Ser Cys
   1               5                   10
<210> 415
   <211> 10
   <212> PRT
   <213> Homo sapiens
<400> 415
   Cys Ala Pro Ser Pro Cys Gln Pro Ala Cys
   1               5                   10
<210> 416
   <211> 10
   <212> PRT
   <213> Homo sapiens
<400> 416
   Cys Ala Pro Val Tyr Cys Arg Arg Thr Cys
   1               5                   10
<210> 417
   <211> 10
   <212> PRT
   <213> Homo sapiens
<400> 417
   Cys Ala Ser Ser Pro Cys Gln Gln Ala Cys
   1               5                   10
<210> 418
   <211> 10
   <212> PRT
   <213> Homo sapiens
<400> 418
   Cys Ala Ser Ser Ser Cys Gln Pro Ala Cys
   1               5                   10
<210> 419
   <211> 10
   <212> PRT
   <213> Homo sapiens
<400> 419
   Cys Ala Ser Ser Ser Cys Gln Gln Ser Cys
   1               5                   10
<210> 420
   <211> 10
   <212> PRT
   <213> Homo sapiens
<400> 420
   Cys Cys Gly Asn Phe Ser Ser His Ser Cys
   1               5                   10
<210> 421
   <211> 10
   <212> PRT
   <213> Homo sapiens
<400> 421
   Cys Cys Gly Tyr Gly Gly Leu Gly Cys Gly
   1               5                   10
<210> 422
   <211> 10
   <212> PRT
   <213> Homo sapiens
<400> 422
   Cys Cys Asn Tyr Tyr Gly Asn Ser Cys Gly
   1               5                   10
<210> 423
   <211> 10
   <212> PRT
   <213> Homo sapiens
<400> 423
   Cys Cys Asn Tyr Tyr Arg Asn Ser Cys Gly
   1               5                   10
<210> 424
   <211> 10
   <212> PRT
   <213> Homo sapiens
<400> 424
   Cys Cys Ser Arg Asn Phe Ser Ser Cys Ser
   1               5                   10
<210> 425
   <211> 10
   <212> PRT
   <213> Homo sapiens
<400> 425
   Cys Asp Ala Gly Ser Cys Gln Pro Ser Cys
   1 5 10
<210> 426
   <211> 10
   <212> PRT
   <213> Homo sapiens
<400> 426
   Cys Asp Pro Cys Ser Leu Gln Glu Gly Cys
   1 5 10
<210> 427
   <211> 10
   <212> PRT
   <213> Homo sapiens
<400> 427
   Cys Asp Pro Ser Pro Cys Glu Pro Ser Cys
   1 5 10
<210> 428
   <211> 10
   <212> PRT
   <213> Homo sapiens
<400> 428
   Cys Asp Pro Val Ile Cys Glu Pro Ser Cys
   1 5 10
<210> 429
   <211> 10
   <212> PRT
   <213> Homo sapiens
<400> 429
   Cys Asp Gln Gly Leu Cys Gln Glu Thr Cys
   1 5 10
<210> 430
   <211> 10
   <212> PRT
   <213> Homo sapiens
<400> 430
   Cys Glu Ala Thr Thr Cys Glu Pro Ser Cys
   1 5 10
<210> 431
   <211> 10
   <212> PRT
   <213> Homo sapiens
<400> 431
   Cys Glu Leu Pro Cys Gly Thr Pro Ser Cys
   1 5 10
<210> 432
   <211> 10
   <212> PRT
   <213> Homo sapiens
<400> 432
   Cys Glu Pro Ala Ile Cys Glu Pro Ser Cys
   1 5 10
<210> 433
   <211> 10
   <212> PRT
   <213> Homo sapiens
<400> 433
   Cys Glu Pro Pro Cys Gly Thr Ala Pro Cys
   1 5 10
<210> 434
   <211> 10
   <212> PRT
   <213> Homo sapiens
<400> 434
   Cys Glu Pro Pro Cys Ser Ala Pro Ser Cys
   1 5 10
<210> 435
   <211> 10
   <212> PRT
   <213> Homo sapiens
<400> 435
   Cys Glu Pro Arg Ser Cys Ala Ser Ser Cys
   1 5 10
<210> 436
   <211> 10
   <212> PRT
   <213> Homo sapiens
<400> 436
   Cys Glu Pro Ser Ala Cys Gln Ser Gly Cys
   1 5 10
<210> 437
   <211> 10
   <212> PRT
   <213> Homo sapiens
<400> 437
   Cys Glu Pro Ser Cys Ser Val Ser Asn Cys
   1 5 10
<210> 438
   <211> 10
   <212> PRT
   <213> Homo sapiens
<400> 438
   Cys Glu Pro Ser Cys Ser Val Ser Ser Cys
   1 5 10
<210> 439
   <211> 10
   <212> PRT
   <213> Homo sapiens
<400> 439
   Cys Glu Pro Ser Pro Cys Gln Ser Gly Cys
   1 5 10
<210> 440
   <211> 10
   <212> PRT
   <213> Homo sapiens
<400> 440
   Cys Glu Pro Thr Ala Cys Gln Pro Thr Cys
   1 5 10
<210> 441
   <211> 10
   <212> PRT
   <213> Homo sapiens
<400> 441
   Cys Glu Pro Thr Ser Cys Gln Thr Ser Cys
   1 5 10
<210> 442
   <211> 10
   <212> PRT
   <213> Homo sapiens
<400> 442
   Cys Glu Pro Val Cys Leu Arg Pro Val Cys
   1 5 10
<210> 443
   <211> 10
   <212> PRT
   <213> Homo sapiens
<400> 443
   Cys Glu Thr Ser Ser Cys Gln Pro Arg Cys
   1 5 10
<210> 444
   <211> 10
   <212> PRT
   <213> Homo sapiens
<400> 444
   Cys Glu Thr Thr Cys Phe Gln Pro Thr Cys
   1 5 10
<210> 445
   <211> 10
   <212> PRT
   <213> Homo sapiens
<400> 445
   Cys Phe Gln Pro Thr Cys Val Ser Ser Cys
   1 5 10
<210> **446**
   <211> 10
   <212> PRT
   <213> Homo sapiens
<400> 446
   Cys Phe Gln Pro Thr Cys Val Thr Ser Cys
   1 5 10
<210> 447
   <211> 10
   <212> PRT
   <213> Homo sapiens
<400> 447
   Cys Phe Gln Pro Thr Cys Val Tyr Ser Cys
   1 5 10
<210> 448
   <211> 10
   <212> PRT
   <213> Homo sapiens
<400> 448
   Cys Gly Cys Gly Phe Arg Arg Leu Gly Cys
   1 5 10
<210> 449
   <211> 10
   <212> PRT
   <213> Homo sapiens
<400> 449
   Cys Gly Cys Gly Tyr Arg Gly Leu Asp Cys
   1 5 10
<210> 450
   <211> 10
   <212> PRT
   <213> Homo sapiens
<400> 450
   Cys Gly Cys Asn Gly Tyr Tyr Gly Cys Tyr
   1 5 10
<210> 451
   <211> 10
   <212> PRT
   <213> Homo sapiens
<400> 451
   Cys Gly Phe Gly Ser Cys Tyr Gly Cys Gly
   1 5 10
<210> 452
   <211> 10
   <212> PRT
   <213> Homo sapiens
<400> 452
   Cys Gly Gly Ser Gly Cys Gly Gly Ser Cys
   1 5 10
<210> 453
   <211> 10
   <212> PRT
   <213> Homo sapiens
<400> 453
   Cys Gly Gly Ser Gly Ser Ser Cys Cys Val
   1 5 10
<210> 454
   <211> 10
   <212> PRT
   <213> Homo sapiens
<400> 454
   Cys Gly Gly Val Ser Cys His Thr Thr Cys
   1 5 10
<210> 455
   <211> 10
   <212> PRT
   <213> Homo sapiens
<400> 455
   Cys Gly Lys Gly Gly Cys Gly Ser Cys Gly
   1 5 10
<210> 456
   <211> 10
   <212> PRT
   <213> Homo sapiens
<400> 456
   Cys Gly Lys Arg Gly Cys Gly Ser Cys Gly
   1 5 10
<210> 457
   <211> 10
   <212> PRT
   <213> Homo sapiens
<400> 457
   Cys Gly Gln Asp Leu Cys Gln Glu Thr Cys
   1 5 10
<210> 458
   <211> 10
   <212> PRT
   <213> Homo sapiens
<400> 458
   Cys Gly Gln Thr Ser Cys Gly Ser Ser Cys
   1 5 10
<210> 459
   <211> 10
   <212> PRT
   <213> Homo sapiens
<400> 459
   Cys Gly Gln Val Leu Cys Gln Glu Thr Cys
   1 5 10
<210> 460
   <211> 10
   <212> PRT
   <213> Homo sapiens
<400> 460
   Cys Gly Arg Asp Leu Cys Gln Glu Thr Cys
   1               5                   10
<210> 461
   <211> 10
   <212> PRT
   <213> Homo sapiens
<400> 461
   Cys Gly Arg Val Ser Cys His Thr Thr Cys
   1               5                   10
<210> 462
   <211> 10
   <212> PRT
   <213> Homo sapiens
<400> 462
   Cys Gly Ser Cys Gly Phe Gly Ser Cys Tyr
   1               5                   10
<210> 463
   <211> 10
   <212> PRT
   <213> Homo sapiens
<400> 463
   Cys Gly Ser Cys Gly Gly Ser Lys Gly Cys
   1               5                   10
<210> 464
   <211> 10
   <212> PRT
   <213> Homo sapiens
<400> 464
   Cys Gly Ser Gly Cys Gly Val Pro Val Cys
   1               5                   10
<210> 465
   <211> 10
   <212> PRT
   <213> Homo sapiens
<400> 465
   Cys Gly Ser Leu Leu Cys Arg Pro Thr Cys
   1               5                   10
<210> 466
   <211> 10
   <212> PRT
   <213> Homo sapiens
<400> 466
   Cys Gly Ser Arg Cys Tyr Val Pro Val Cys
   1 5 10
<210> 467
   <211> 10
   <212> PRT
   <213> Homo sapiens
<400> 467
   Cys Gly Ser Ser Ser Cys Gly Pro Gln Cys
   1 5 10
<210> 468
   <211> 10
   <212> PRT
   <213> Homo sapiens
<400> 468
   Cys Gly Ser Val Cys Ser Asp Gln Gly Cys
   1 5 10
<210> 469
   <211> 10
   <212> PRT
   <213> Homo sapiens
<400> 469
   Cys Gly Ser Val Cys Ser Asp Gln Ser Cys
   1 5 10
<210> 470
   <211> 10
   <212> PRT
   <213> Homo sapiens
<400> 470
   Cys Gly Ser Val Cys Ser His Gln Gly Cys
   1 5 10
<210> 471
   <211> 10
   <212> PRT
   <213> Homo sapiens
<400> 471
   Cys Gly Ser Tyr Gly Cys Ser Gln Cys Ser
   1 5 10
<210> 472
   <211> 10
   <212> PRT
   <213> Homo sapiens
<400> 472
   Cys Gly Val Cys Leu Pro Ser Thr Cys Pro
   1               5                   10
<210> 473
   <211> 10
   <212> PRT
   <213> Homo sapiens
<400> 473
   Cys Gly Tyr Glu Gly Cys Arg Tyr Gly Cys
   1               5                   10
<210> 474
   <211> 10
   <212> PRT
   <213> Homo sapiens
<400> 474
   Cys Gly Tyr Gly Cys Gly Tyr Gly Cys Gly
   1               5                   10
<210> 475
   <211> 10
   <212> PRT
   <213> Homo sapiens
<400> 475
   Cys Gly Tyr Gly Gly Cys Gly Tyr Gly Cys
   1               5                   10
<210> 476
   <211> 10
   <212> PRT
   <213> Homo sapiens
<400> 476
   Cys Gly Tyr Gly Ser Phe Cys Gly Cys Gly
   1               5                   10
<210> 477
   <211> 10
   <212> PRT
   <213> Homo sapiens
<400> 477
   Cys Gly Tyr Gly Ser Gly Cys Gly Cys Gly
   1 5 10
<210> 478
   <211> 10
   <212> PRT
   <213> Homo sapiens
<400> 478
   Cys His Pro Ser Cys Gly Met Ser Ser Cys
   1 5 10
<210> 479
   <211> 10
   <212> PRT
   <213> Homo sapiens
<400> 479
   Cys His Pro Ser Cys Ser Ile Ser Ser Cys
   1 5 10
<210> 480
   <211> 10
   <212> PRT
   <213> Homo sapiens
<400> 480
   Cys His Pro Thr Cys Tyr Gln Thr Ile Cys
   1 5 10
<210> 481
   <211> 10
   <212> PRT
   <213> Homo sapiens
<400> 481
   Cys His Thr Ser Cys Ser Pro Ala Cys Gln
   1 5 10
<210> 482
   <211> 10
   <212> PRT
   <213> Homo sapiens
<400> 482
   Cys His Thr Ser Cys Ser Ser Gly Cys Gln
   1 5 10
<210> 483
   <211> 10
   <212> PRT
   <213> Homo sapiens
<400> 483
   Cys His Thr Thr Cys Tyr Arg Pro Ala Cys
   1 5 10
<210> 484
   <211> 10
   <212> PRT
   <213> Homo sapiens
<400> 484
   Cys His Thr Thr Cys Tyr Arg Pro Thr Cys
   1 5 10
<210> 485
   <211> 10
   <212> PRT
   <213> Homo sapiens
<400> 485
   Cys Ile His Ser Pro Cys Gln Ala Ser Cys
   1 5 10
<210> 486
   <211> 10
   <212> PRT
   <213> Homo sapiens
<400> 486
   Cys Ile His Ser Thr His Cys Gly Cys Asn
   1 5 10
<210> 487
   <211> 10
   <212> PRT
   <213> Homo sapiens
<400> 487
   Cys Ile Arg Ser Pro Cys Gln Ala Ser Cys
   1 5 10
<210> 488
   <211> 10
   <212> PRT
   <213> Homo sapiens
<400> 488
   Cys Ile Ser Ser Cys Tyr Arg Pro Gln Cys
   1 5 10
<210> 489
   <211> 10
   <212> PRT
   <213> Homo sapiens
<400> 489
   Cys Ile Ser Ser Pro Cys Gln Gln Ser Cys
   1               5                   10
<210> 490
   <211> 10
   <212> PRT
   <213> Homo sapiens
<400> 490
   Cys Lys Pro Cys Ser Ser Gln Ser Ser Cys
   1               5                   10
<210> 491
   <211> 10
   <212> PRT
   <213> Homo sapiens
<400> 491
   Cys Lys Pro Ser Cys Ser Gln Ser Ser Cys
   1               5                   10
<210> 492
   <211> 10
   <212> PRT
   <213> Homo sapiens
<400> 492
   Cys Lys Pro Val Cys Phe Lys Pro Ile Cys
   1               5                   10
<210> 493
   <211> 10
   <212> PRT
   <213> Homo sapiens
<400> 493
   Cys Lys Pro Val Cys Tyr Val Pro Thr Cys
   1               5                   10
<210> 494
   <211> 10
   <212> PRT
   <213> Homo sapiens
<400> 494
   Cys Lys Pro Val Ser Cys Val Pro Val Cys
   1               5                   10
<210> 495
   <211> 10
   <212> PRT
   <213> Homo sapiens
<400> 495
   Cys Lys Pro Val Tyr Cys Val Pro Val Cys
   1 5 10
<210> 496
   <211> 10
   <212> PRT
   <213> Homo sapiens
<400> 496
   Cys Lys Thr Val Tyr Cys Lys Pro Ile Cys
   1 5 10
<210> 497
   <211> 10
   <212> PRT
   <213> Homo sapiens
<400> 497
   Cys Leu Asn Gln Ser Cys Gly Ser Asn Cys
   1 5 10
<210> 498
   <211> 10
   <212> PRT
   <213> Homo sapiens
<400> 498
   Cys Leu Asn Gln Ser Cys Gly Ser Ser Cys
   1 5 10
<210> 499
   <211> 10
   <212> PRT
   <213> Homo sapiens
<400> 499
   Cys Leu Pro Gly Cys Leu Asn Gln Ser Cys
   1 5 10
<210> 500
   <211> 10
   <212> PRT
   <213> Homo sapiens
<400> 500
   Cys Leu Pro Gly Ser Cys Asp Ser Cys Ser
   1 5 10
<210> 501
   <211> 10
   <212> PRT
   <213> Homo sapiens
<400> 501
   Cys Leu Pro Pro Cys Tyr Leu Val Ser Cys
   1               5                   10
<210> 502
   <211> 10
   <212> PRT
   <213> Homo sapiens
<400> 502
   Cys Leu Pro Thr Ser Cys Gln Pro Ser Cys
   1               5                   10
<210> 503
   <211> 10
   <212> PRT
   <213> Homo sapiens
<400> 503
   Cys Leu Ser Phe Leu Cys Arg Pro Ala Cys
   1               5                   10
<210> 504
   <211> 10
   <212> PRT
   <213> Homo sapiens
<400> 504
   Cys Leu Val Ser Ser Cys Gln Pro Ser Cys
   1               5                   10
<210> 505
   <211> 10
   <212> PRT
   <213> Homo sapiens
<400> 505
   Cys Met Pro Ser Pro Cys Gln Pro Ala Cys
   1               5                   10
<210> 506
   <211> 10
   <212> PRT
   <213> Homo sapiens
<400> 506
   Cys Met Ser Gly Ser Cys Gln Ala Ala Cys
   1 5 10
<210> 507
   <211> 10
   <212> PRT
   <213> Homo sapiens
<400> 507
   Cys Asn Glu Ser Ser Tyr Cys Leu Pro Cys
   1 5 10
<210> 508
   <211> 10
   <212> PRT
   <213> Homo sapiens
<400> 508
   Cys Pro Ala Ser Cys Val Ser Leu Leu Cys
   1 5 10
<210> 509
   <211> 10
   <212> PRT
   <213> Homo sapiens
<400> 509
   Cys Pro Met Ala Cys Pro Gly Ser Pro Cys
   1 5 10
<210> 510
   <211> 10
   <212> PRT
   <213> Homo sapiens
<400> 510
   Cys Pro Ser Ser Cys Thr Ala Val Val Cys
   1 5 10
<210> 511
   <211> 10
   <212> PRT
   <213> Homo sapiens
<400> 511
   Cys Pro Val Thr Cys Glu Pro Ser Pro Cys
   1 5 10
<210> 512
   <211> 10
   <212> PRT
   <213> Homo sapiens
<400> 512
   Cys Gln Ala Ala Cys Glu Pro Ser Ala Cys
   1 5 10
<210> 513
   <211> 10
   <212> PRT
   <213> Homo sapiens
<400> 513
   Cys Gln Ala Ala Cys Glu Pro Ser Pro Cys
   1 5 10
<210> 514
   <211> 10
   <212> PRT
   <213> Homo sapiens
<400> 514
   Cys Gln Ala Ala Cys Gly Gln Ser Val Cys
   1 5 10
<210> 515
   <211> 10
   <212> PRT
   <213> Homo sapiens
<400> 515
   Cys Gln Ala Pro Cys Ser Thr Lys Asn Cys
   1 5 10
<210> 516
   <211> 10
   <212> PRT
   <213> Homo sapiens
<400> 516
   Cys Gln Ala Val Cys Glu Pro Ser Pro Cys
   1 5 10
<210> 517
   <211> 10
   <212> PRT
   <213> Homo sapiens
<400> 517
   Cys Gln Asp Ser Cys Gly Ser Ser Ser Cys
   1 5 10
<210> 518
   <211> 10
   <212> PRT
   <213> Homo sapiens
<400> 518
   Cys Gln His Ser Ser Cys Gln Pro Thr Cys
   1 5 10
<210> 519
   <211> 10
   <212> PRT
   <213> Homo sapiens
<400> 519
   Cys Gln Ile Ser Ser Cys Gly Thr Gly Cys
   1 5 10
<210> 520
   <211> 10
   <212> PRT
   <213> Homo sapiens
<400> 520
   Cys Gln Lys Ser Ser Cys Gln Pro Ala Cys
   1 5 10
<210> 521
   <211> 10
   <212> PRT
   <213> Homo sapiens
<400> 521
   Cys Gln Pro Met Cys Ser His Ala Ala Cys
   1 5 10
<210> 522
   <211> 10
   <212> PRT
   <213> Homo sapiens
<400> 522
   Cys Gln Pro Pro Cys Thr Thr Ala Leu Cys
   1 5 10
<210> 523
   <211> 10
   <212> PRT
   <213> Homo sapiens
<400> 523
   Cys Gln Pro Ser Cys Glu Ser Ser Phe Cys
   1 5 10
<210> 524
   <211> 10
   <212> PRT
   <213> Homo sapiens
<400> 524
   Cys Gln Pro Ser Cys Ser Glu Ser Thr Cys
   1 5 10
<210> 525
   <211> 10
   <212> PRT
   <213> Homo sapiens
<400> 525
   Cys Gln Pro Ser Cys Thr Ser Val Leu Cys
   1 5 10
<210> 526
   <211> 10
   <212> PRT
   <213> Homo sapiens
<400> 526
   Cys Gln Pro Thr Cys Gly Gly Ser Ser Cys
   1 5 10
<210> 527
   <211> 10
   <212> PRT
   <213> Homo sapiens
<400> 527
   Cys Gln Pro Thr Cys Ser Arg Pro Ser Cys
   1 5 10
<210> 528
   <211> 10
   <212> PRT
   <213> Homo sapiens
<400> 528
   Cys Gln Pro Val Cys Pro Thr Pro Thr Cys
   1 5 10
<210> 529
   <211> 10
   <212> PRT
   <213> Homo sapiens
<400> 529
   Cys Gln Pro Val Leu Cys Lys Ser Ser Cys
   1 5 10
<210> 530
   <211> 10
   <212> PRT
   <213> Homo sapiens
<400> 530
   Cys Gln Pro Val Val Cys Glu Pro Ser Cys
   1 5 10
<210> 531
   <211> 10
   <212> PRT
   <213> Homo sapiens
<400> 531
   Cys Gln Gln Pro Ser Cys Gln Pro Ala Cys
   1 5 10
<210> 532
   <211> 10
   <212> PRT
   <213> Homo sapiens
<400> 532
   Cys Gln Gln Ser Cys Arg Val Pro Val Cys
   1 5 10
<210> 533
   <211> 10
   <212> PRT
   <213> Homo sapiens
<400> 533
   Cys Gln Gln Ser Cys Tyr Val Pro Val Cys
   1 5 10
<210> 534
   <211> 10
   <212> PRT
   <213> Homo sapiens
<400> 534
   Cys Gln Gln Ser Gly Cys Gln Pro Ala Cys
   1 5 10
<210> 535
   <211> 10
   <212> PRT
   <213> Homo sapiens
<400> 535
   Cys Gln Gln Ser Ser Cys His Pro Ala Cys
   1 5 10
<210> 536
   <211> 10
   <212> PRT
   <213> Homo sapiens
<400> 536
   Cys Gln Gln Ser Ser Cys Lys Pro Ala Cys
   1 5 10
<210> 537
   <211> 10
   <212> PRT
   <213> Homo sapiens
<400> 537
   Cys Gln Gln Ser Ser Cys Gln Leu Ala Cys
   1 5 10
<210> 538
   <211> 10
   <212> PRT
   <213> Homo sapiens
<400> 538
   Cys Gln Gln Ser Ser Cys Gln Pro Ala Cys
   1 5 10
<210> 539
   <211> 10
   <212> PRT
   <213> Homo sapiens
<400> 539
   Cys Gln Gln Ser Ser Cys Gln Pro Thr Cys
   1 5 10
<210> 540
   <211> 10
   <212> PRT
   <213> Homo sapiens
<400> 540
   Cys Gln Gln Ser Ser Cys Gln Ser Ala Cys
   1 5 10
<210> 541
   <211> 10
   <212> PRT
   <213> Homo sapiens
<400> 541
   Cys Gln Gln Ser Ser Cys Val Ser Cys Val
   1               5                   10
<210> 542
   <211> 10
   <212> PRT
   <213> Homo sapiens
<400> 542
   Cys Gln Gln Ser Tyr Cys Val Pro Val Cys
   1               5                   10
<210> 543
   <211> 10
   <212> PRT
   <213> Homo sapiens
<400> 543
   Cys Gln Ser Gly Cys Ile Ser Ser Cys Thr
   1               5                   10
<210> 544
   <211> 10
   <212> PRT
   <213> Homo sapiens
<400> 544
   Cys Gln Ser Gly Cys Thr Asp Ser Cys Thr
   1               5                   10
<210> 545
   <211> 10
   <212> PRT
   <213> Homo sapiens
<400> 545
   Cys Gln Ser Gly Cys Thr Ser Ser Cys Thr
   1               5                   10
<210> 546
   <211> 10
   <212> PRT
   <213> Homo sapiens
<400> 546
   Cys Gln Ser Ser Cys Tyr Arg Pro Thr Cys
   1               5                   10
<210> 547
   <211> 10
   <212> PRT
   <213> Homo sapiens
<400> 547
   Cys Gln Ser Val Cys Tyr Gln Pro Thr Cys
   1 5 10
<210> 548
   <211> 10
   <212> PRT
   <213> Homo sapiens
<400> 548
   Cys Gln Ser Val Tyr Cys Gln Pro Thr Cys
   1 5 10
<210> 549
   <211> 10
   <212> PRT
   <213> Homo sapiens
<400> 549
   Cys Gln Thr Ala Cys Glu Pro Ser Ala Cys
   1 5 10
<210> 550
   <211> 10
   <212> PRT
   <213> Homo sapiens
<400> 550
   Cys Gln Thr Ser Ser Cys Gly Thr Gly Cys
   1 5 10
<210> 551
   <211> 10
   <212> PRT
   <213> Homo sapiens
<400> 551
   Cys Gln Thr Thr Cys His Pro Ser Cys Gly
   1 5 10
<210> 552
   <211> 10
   <212> PRT
   <213> Homo sapiens
<400> 552
   Cys Gln Thr Thr Cys Arg Pro Ser Cys Gly
   1 5 10
<210> 553
   <211> 10
   <212> PRT
   <213> Homo sapiens
<400> 553
   Cys Gln Thr Thr Cys Tyr Arg Thr Thr Cys
   1               5                   10
<210> 554
   <211> 10
   <212> PRT
   <213> Homo sapiens
<400> 554
   Cys Gln Thr Thr Arg Cys Arg Thr Thr Cys
   1               5                   10
<210> 555
   <211> 10
   <212> PRT
   <213> Homo sapiens
<400> 555
   Cys Gln Val Thr Cys Glu Pro Ser Pro Cys
   1               5                   10
<210> 556
   <211> 10
   <212> PRT
   <213> Homo sapiens
<400> 556
   Cys Arg Asn Thr Ser Cys Gln Pro Thr Cys
   1               5                   10
<210> 557
   <211> 10
   <212> PRT
   <213> Homo sapiens
<400> 557
   Cys Arg Pro Ala Cys Ser Pro Leu Ala Cys
   1               5                   10
<210> 558
   <211> 10
   <212> PRT
   <213> Homo sapiens
<400> 558
   Cys Arg Pro Ala Cys Ser Arg Leu Ala Cys
   1               5                   10
<210> 559
   <211> 10
   <212> PRT
   <213> Homo sapiens
<400> 559
   Cys Arg Pro Ala Cys Ser Arg Pro Ala Cys
   1               5                   10
<210> 560
   <211> 10
   <212> PRT
   <213> Homo sapiens
<400> 560
   Cys Arg Pro Met Cys Ser Arg Pro Ala Cys
   1               5                   10
<210> 561
   <211> 10
   <212> PRT
   <213> Homo sapiens
<400> 561
   Cys Arg Pro Ser Cys Gly Gln Thr Thr Cys
   1               5                   10
<210> 562
   <211> 10
   <212> PRT
   <213> Homo sapiens
<400> 562
   Cys Arg Pro Ser Cys Gly Val Ser Ser Cys
   1               5                   10
<210> 563
   <211> 10
   <212> PRT
   <213> Homo sapiens
<400> 563
   Cys Arg Pro Ser Cys Ser Ile Ser Ser Cys
   1               5                   10
<210> 564
   <211> 10
   <212> PRT
   <213> Homo sapiens
<400> 564
   Cys Arg Pro Ser Cys Ser Gln Thr Thr Cys
   1 5 10
<210> 565
   <211> 10
   <212> PRT
   <213> Homo sapiens
<400> 565
   Cys Arg Pro Ser Tyr Cys Gly Gln Ser Cys
   1 5 10
<210> 566
   <211> 10
   <212> PRT
   <213> Homo sapiens
<400> 566
   Cys Arg Pro Ser Tyr Cys Ile Ser Ser Cys
   1 5 10
<210> 567
   <211> 10
   <212> PRT
   <213> Homo sapiens
<400> 567
   Cys Arg Pro Ser Tyr Cys Gln Thr Thr Cys
   1 5 10
<210> 568
   <211> 10
   <212> PRT
   <213> Homo sapiens
<400> 568
   Cys Arg Pro Thr Cys Ser Arg Leu Ala Cys
   1 5 10
<210> 569
   <211> 10
   <212> PRT
   <213> Homo sapiens
<400> 569
   Cys Arg Pro Thr Cys Ser Ser Gly Ser Cys
   1 5 10
<210> 570
   <211> 10
   <212> PRT
   <213> Homo sapiens
<400> 570
   Cys Arg Pro Thr Ser Cys Gln Asn Thr Cys
   1 5 10
<210> 571
   <211> 10
   <212> PRT
   <213> Homo sapiens
<400> 571
   Cys Arg Pro Val Cys Arg Ser Thr Tyr Cys
   1 5 10
<210> 572
   <211> 10
   <212> PRT
   <213> Homo sapiens
<400> 572
   Cys Arg Pro Val Cys Ser Arg Pro Ala Cys
   1 5 10
<210> 573
   <211> 10
   <212> PRT
   <213> Homo sapiens
<400> 573
   Cys Arg Pro Val Thr Cys Val Pro Arg Cys
   1 5 10
<210> 574
   <211> 10
   <212> PRT
   <213> Homo sapiens
<400> 574
   Cys Arg Gln Ser Ser Cys Gln Pro Ala Cys
   1 5 10
<210> 575
   <211> 10
   <212> PRT
   <213> Homo sapiens
<400> 575
   Cys Arg Thr Thr Cys Phe His Pro Ile Cys
   1 5 10
<210> 576
   <211> 10
   <212> PRT
   <213> Homo sapiens
<400> 576
   Cys Arg Thr Thr Cys Phe Gln Pro Thr Cys
   1 5 10
<210> 577
   <211> 10
   <212> PRT
   <213> Homo sapiens
<400> 577
   Cys Arg Thr Thr Cys Tyr Arg Pro Ser Cys
   1 5 10
<210> 578
   <211> 10
   <212> PRT
   <213> Homo sapiens
<400> 578
   Cys Arg Thr Thr Tyr Cys Arg Pro Ser Cys
   1 5 10
<210> 579
   <211> 10
   <212> PRT
   <213> Homo sapiens
<400> 579
   Cys Arg Val Thr Cys Glu Pro Ser Pro Cys
   1 5 10
<210> 580
   <211> 10
   <212> PRT
   <213> Homo sapiens
<400> 580
   Cys Arg Tyr Gly Cys Gly His Arg Gly Cys
   1 5 10
<210> 581
   <211> 10
   <212> PRT
   <213> Homo sapiens
<400> 581
   Cys Ser Ala Pro Cys Val Ala Leu Leu Cys
   1 5 10
<210> 582
   <211> 10
   <212> PRT
   <213> Homo sapiens
<400> 582
   Cys Ser Asp Asp Ser Gly Ser Cys Cys Gln
   1 5 10
<210> 583
   <211> 10
   <212> PRT
   <213> Homo sapiens
<400> 583
   Cys Ser Glu Asp Ser Ser Ser Cys Cys Gln
   1 5 10
<210> 584
   <211> 10
   <212> PRT
   <213> Homo sapiens
<400> 584
   Cys Ser Glu Asp Ser Tyr Ser Cys Cys Gln
   1 5 10
<210> 585
   <211> 10
   <212> PRT
   <213> Homo sapiens
<400> 585
   Cys Ser Glu Gly Cys Gly Ser Gly Cys Gly
   1 5 10
<210> 586
   <211> 10
   <212> PRT
   <213> Homo sapiens
<400> 586
   Cys Ser Glu Ser Ser Pro Ser Cys Cys Gln
   1 5 10
<210> 587
   <211> 10
   <212> PRT
   <213> Homo sapiens
<400> 587
   Cys Ser Glu Ser Ser Ser Ser Cys Cys Gln
   1 5 10
<210> 588
   <211> 10
   <212> PRT
   <213> Homo sapiens
<400> 588
   Cys Ser Phe Asp Lys Ser Cys Arg Cys Gly
   1 5 10
<210> 589
   <211> 10
   <212> PRT
   <213> Homo sapiens
<400> 589
   Cys Ser Gly Ala Ser Ser Leu Cys Cys Gln
   1 5 10
<210> 590
   <211> 10
   <212> PRT
   <213> Homo sapiens
<400> 590
   Cys Ser Gly Ala Ser Ser Pro Cys Cys Gln
   1 5 10
<210> 591
   <211> 10
   <212> PRT
   <213> Homo sapiens
<400> 591
   Cys Ser Gly Ala Ser Ser Ser Cys Cys Gln
   1 5 10
<210> 592
   <211> 10
   <212> PRT
   <213> Homo sapiens
<400> 592
   Cys Ser Gly Ala Ser Thr Ser Cys Cys Gln
   1 5 10
<210> 593
   <211> 10
   <212> PRT
   <213> Homo sapiens
<400> 593
   Cys Ser Gly Gly Cys Gly Ser Gly Cys Gly
   1 5 10
<210> 594
   <211> 10
   <212> PRT
   <213> Homo sapiens
<400> 594
   Cys Ser Gly Gly Cys Gly Ser Ser Cys Gly
   1 5 10
<210> 595
   <211> 10
   <212> PRT
   <213> Homo sapiens
<400> 595
   Cys Ser Gly Ile Ser Ser Ser Cys Cys Gln
   1 5 10
<210> 596
   <211> 10
   <212> PRT
   <213> Homo sapiens
<400> 596
   Cys Ser Lys Asp Ser Ser Ser Cys Cys Gln
   1 5 10
<210> 597
   <211> 10
   <212> PRT
   <213> Homo sapiens
<400> 597
   Cys Ser Lys Gly Ala Cys Gly Ser Cys Gly
   1 5 10
<210> 598
   <211> 10
   <212> PRT
   <213> Homo sapiens
<400> 598
   Cys Ser Leu Ser Cys Gly Ser Arg Ser Cys
   1 5 10
<210> 599
   <211> 10
   <212> PRT
   <213> Homo sapiens
<400> 599
   Cys Ser Gln Asp Leu Cys Gln Glu Thr Cys
   1 5 10
<210> 600
   <211> 10
   <212> PRT
   <213> Homo sapiens
<400> 600
   Cys Ser Arg Gly Cys Gly Ser Gly Cys Gly
   1 5 10
<210> 601
   <211> 10
   <212> PRT
   <213> Homo sapiens
<400> 601
   Cys Ser Arg Leu Ser Ser Ala Cys Cys Gly
   1 5 10
<210> 602
   <211> 10
   <212> PRT
   <213> Homo sapiens
<400> 602
   Cys Ser Ser Cys Gly Lys Gly Gly Cys Gly
   1 5 10
<210> 603
   <211> 10
   <212> PRT
   <213> Homo sapiens
<400> 603
   Cys Ser Ser Cys Gly Lys Arg Gly Cys Gly
   1 5 10
<210> 604
   <211> 10
   <212> PRT
   <213> Homo sapiens
<400> 604
   Cys Ser Ser Asp Lys Ser Cys Arg Cys Gly
   1 5 10
<210> 605
   <211> 10
   <212> PRT
   <213> Homo sapiens
<400> 605
   Cys Ser Ser Gly Asn Phe Ser Ser Cys Cys
   1 5 10
<210> 606
   <211> 10
   <212> PRT
   <213> Homo sapiens
<400> 606
   Cys Ser Ser Ser Gly Cys Gly Ser Phe Cys
   1 5 10
<210> 607
   <211> 10
   <212> PRT
   <213> Homo sapiens
<400> 607
   Cys Ser Ser Ser Gly Cys Gly Ser Ser Cys
   1 5 10
<210> 608
   <211> 10
   <212> PRT
   <213> Homo sapiens
<400> 608
   Cys Ser Thr Pro Cys Tyr Gln Pro Ile Cys
   1 5 10
<210> 609
   <211> 10
   <212> PRT
   <213> Homo sapiens
<400> 609
   Cys Ser Thr Thr Cys Arg Thr Ser Ser Cys
   1 5 10
<210> 610
   <211> 10
   <212> PRT
   <213> Homo sapiens
<400> 610
   Cys Ser Trp Val Pro Ala Cys Ser Cys Thr
   1 5 10
<210> 611
   <211> 10
   <212> PRT
   <213> Homo sapiens
<400> 611
   Cys Thr Phe Ser Pro Cys Gln Gln Ala Cys
   1               5                   10
<210> 612
   <211> 10
   <212> PRT
   <213> Homo sapiens
<400> 612
   Cys Thr Met Ser Val Cys Ser Ser Ala Cys
   1               5                   10
<210> 613
   <211> 10
   <212> PRT
   <213> Homo sapiens
<400> 613
   Cys Thr Arg Pro Ile Cys Glu Pro Cys Arg
   1               5                   10
<210> 614
   <211> 10
   <212> PRT
   <213> Homo sapiens
<400> 614
   Cys Thr Ser Ser Pro Cys Gln His Ala Cys
   1               5                   10
<210> 615
   <211> 10
   <212> PRT
   <213> Homo sapiens
<400> 615
   Cys Thr Ser Ser Pro Cys Gln Gln Ala Cys
   1               5                   10
<210> 616
   <211> 10
   <212> PRT
   <213> Homo sapiens
<400> 616
   Cys Thr Ser Ser Pro Cys Gln Gln Ser Cys
   1               5                   10
<210> 617
   <211> 10
   <212> PRT
   <213> Homo sapiens
<400> 617
   Cys Thr Ser Ser Ser Cys Gln Gln Ala Cys
   1 5 10
<210> 618
   <211> 10
   <212> PRT
   <213> Homo sapiens
<400> 618
   Cys Val Ala Leu Leu Cys Arg Pro Leu Cys
   1 5 10
<210> 619
   <211> 10
   <212> PRT
   <213> Homo sapiens
<400> 619
   Cys Val Ala Leu Val Cys Glu Pro Val Cys
   1 5 10
<210> 620
   <211> 10
   <212> PRT
   <213> Homo sapiens
<400> 620
   Cys Val Phe Ser Ser Cys Asn Thr Thr Cys
   1 5 10
<210> 621
   <211> 10
   <212> PRT
   <213> Homo sapiens
<400> 621
   Cys Val Gly Phe Val Cys Gln Pro Met Cys
   1 5 10
<210> 622
   <211> 10
   <212> PRT
   <213> Homo sapiens
<400> 622
   Cys Val Pro Arg Cys Thr Arg Pro Ile Cys
   1 5 10
<210> 623
   <211> 10
   <212> PRT
   <213> Homo sapiens
<400> 623
   Cys Val Pro Ser Pro Cys Gln Val Ala Cys
   1 5 10
<210> 624
   <211> 10
   <212> PRT
   <213> Homo sapiens
<400> 624
   Cys Val Pro Ser Arg Cys Gln Ala Ser Cys
   1 5 10
<210> 625
   <211> 10
   <212> PRT
   <213> Homo sapiens
<400> 625
   Cys Val Pro Ser Ser Cys Gln Ala Ser Cys
   1 5 10
<210> 626
   <211> 10
   <212> PRT
   <213> Homo sapiens
<400> 626
   Cys Val Pro Val Cys Asn Lys Pro Val Cys
   1 5 10
<210> 627
   <211> 10
   <212> PRT
   <213> Homo sapiens
<400> 627
   Cys Val Pro Val Cys Ser Lys Ser Val Cys
   1 5 10
<210> 628
   <211> 10
   <212> PRT
   <213> Homo sapiens
<400> 628
   Cys Val Pro Val Arg Cys Lys Pro Val Cys
   1 5 10
<210> 629
   <211> 10
   <212> PRT
   <213> Homo sapiens
<400> 629
   Cys Val Ser Leu Leu Cys Arg Pro Ala Cys
   1 5 10
<210> 630
   <211> 10
   <212> PRT
   <213> Homo sapiens
<400> 630
   Cys Val Ser Leu Leu Cys Arg Pro Met Cys
   1 5 10
<210> 631
   <211> 10
   <212> PRT
   <213> Homo sapiens
<400> 631
   Cys Val Ser Leu Leu Cys Arg Pro Thr Cys
   1 5 10
<210> 632
   <211> 10
   <212> PRT
   <213> Homo sapiens
<400> 632
   Cys Val Ser Leu Leu Cys Arg Pro Val Cys
   1 5 10
<210> 633
   <211> 10
   <212> PRT
   <213> Homo sapiens
<400> 633
   Cys Val Ser Asn Pro Cys Gln Val Thr Cys
   1 5 10
<210> 634
   <211> 10
   <212> PRT
   <213> Homo sapiens
<400> 634
   Cys Val Ser Arg Cys Tyr Arg Pro His Cys
   1               5                   10
<210> 635
   <211> 10
   <212> PRT
   <213> Homo sapiens
<400> 635
   Cys Val Ser Ser Cys Phe Arg Pro Gln Cys
   1               5                   10
<210> 636
   <211> 10
   <212> PRT
   <213> Homo sapiens
<400> 636
   Cys Val Ser Ser Ile Cys Gln Pro Ile Cys
   1               5                   10
<210> 637
   <211> 10
   <212> PRT
   <213> Homo sapiens
<400> 637
   Cys Val Ser Ser Pro Cys Gln Pro Thr Cys
   1               5                   10
<210> 638
   <211> 10
   <212> PRT
   <213> Homo sapiens
<400> 638
   Cys Val Val Ser Cys Thr Pro Pro Ser Cys
   1               5                   10
<210> 639
   <211> 10
   <212> PRT
   <213> Homo sapiens
<400> 639
   Cys Val Val Ser Cys Thr Pro Pro Thr Cys
   1               5                   10
<210> 640
   <211> 10
   <212> PRT
   <213> Homo sapiens
<400> 640
   Cys Tyr Cys Pro Lys Asn Ser Ile Phe Cys
   1 5 10
<210> 641
   <211> 10
   <212> PRT
   <213> Homo sapiens
<400> 641
   Cys Tyr Glu Pro Cys Leu Pro Arg Gly Cys
   1 5 10
<210> 642
   <211> 10
   <212> PRT
   <213> Homo sapiens
<400> 642
   Cys Tyr Arg Arg Cys Tyr Ser Ser Cys Tyr
   1 5 10
<210> 643
   <211> 10
   <212> PRT
   <213> Homo sapiens
<400> 643
   Gly Cys Cys Gly Tyr Gly Gly Leu Gly Cys
   1 5 10
<210> 644
   <211> 10
   <212> PRT
   <213> Homo sapiens
<400> 644
   Gly Cys Gly Gly Cys Gly Ser Gly Cys Ala
   1 5 10
<210> 645
   <211> 10
   <212> PRT
   <213> Homo sapiens
<400> 645
   Gly Cys Gly Gly Cys Gly Ser Gly Cys Gly
   1 5 10
<210> 646
   <211> 10
   <212> PRT
   <213> Homo sapiens
<400> 646
   Gly Cys Gly Gly Cys Gly Ser Ser Cys Gly
   1 5 10
<210> 647
   <211> 10
   <212> PRT
   <213> Homo sapiens
<400> 647
   Gly Cys Gly Gly Cys Ser Ser Ser Cys Gly
   1 5 10
<210> 648
   <211> 10
   <212> PRT
   <213> Homo sapiens
<400> 648
   Gly Cys Gly Gly Ser Gly Ser Ser Cys Cys
   1 5 10
<210> 649
   <211> 10
   <212> PRT
   <213> Homo sapiens
<400> 649
   Gly Cys Gly Ser Gly Cys Ala Gly Cys Gly
   1 5 10
<210> 650
   <211> 10
   <212> PRT
   <213> Homo sapiens
<400> 650
   Gly Cys Gly Ser Gly Cys Gly Gly Cys Gly
   1 5 10
<210> 651
   <211> 10
   <212> PRT
   <213> Homo sapiens
<400> 651
   Gly Cys Gly Ser Gly Cys Gly Gly Cys Ser
   1 5 10
<210> 652
   <211> 10
   <212> PRT
   <213> Homo sapiens
<400> 652
   Gly Cys Gly Ser Ser Cys Gly Gly Cys Asp
   1 5 10
<210> 653
   <211> 10
   <212> PRT
   <213> Homo sapiens
<400> 653
   Gly Cys Gly Ser Ser Cys Gly Gly Cys Gly
   1 5 10
<210> 654
   <211> 10
   <212> PRT
   <213> Homo sapiens
<400> 654
   Gly Cys Gly Ser Ser Cys Ser Gln Cys Ser
   1 5 10
<210> 655
   <211> 10
   <212> PRT
   <213> Homo sapiens
<400> 655
   Gly Cys Gly Tyr Ser Ser Ser Cys Cys Gly
   1 5 10
<210> 656
   <211> 10
   <212> PRT
   <213> Homo sapiens
<400> 656
   Gly Cys Lys Gly Gly Cys Gly Ser Cys Gly
   1 5 10
<210> 657
   <211> 10
   <212> PRT
   <213> Homo sapiens
<400> 657
   Gly Cys Ser Gly Cys Ser Gly Gly Cys Gly
   1 5 10
<210> 658
   <211> 10
   <212> PRT
   <213> Homo sapiens
<400> 658
   Ile Cys Ser Gly Ala Ser Ser Leu Cys Cys
   1 5 10
<210> 659
   <211> 10
   <212> PRT
   <213> Homo sapiens
<400> 659
   Ile Cys Ser Gly Ala Ser Ser Pro Cys Cys
   1 5 10
<210> 660
   <211> 10
   <212> PRT
   <213> Homo sapiens
<400> 660
   Met Cys Cys Asn Tyr Tyr Gly Asn Ser Cys
   1 5 10
<210> 661
   <211> 10
   <212> PRT
   <213> Homo sapiens
<400> 661
   Met Cys Cys Asn Tyr Tyr Arg Asn Ser Cys
   1 5 10
<210> 662
   <211> 10
   <212> PRT
   <213> Homo sapiens
<400> 662
   Met Cys Tyr Gly Tyr Gly Cys Gly Cys Gly
   1 5 10
<210> 663
   <211> 10
   <212> PRT
   <213> Homo sapiens
<400> 663
   Asn Cys Cys Ser Arg Asn Phe Ser Ser Cys
   1 5 10
<210> 664
   <211> 10
   <212> PRT
   <213> Homo sapiens
<400> 664
   Pro Cys Ser Leu Gln Glu Gly Cys Cys Arg
   1 5 10
<210> 665
   <211> 10
   <212> PRT
   <213> Homo sapiens
<400> 665
   Pro Cys Ser Ser Gln Ser Ser Cys Cys Val
   1 5 10
<210> 666
   <211> 10
   <212> PRT
   <213> Homo sapiens
<400> 666
   Ser Cys Cys Ala Pro Ala Ser Ser Cys Gln
   1 5 10
<210> 667
   <211> 10
   <212> PRT
   <213> Homo sapiens
<400> 667
   Ser Cys Cys Ala Pro Ala Ser Thr Cys Gln
   1 5 10
<210> 668
   <211> 10
   <212> PRT
   <213> Homo sapiens
<400> 668
   Ser Cys Cys Ala Pro Thr Ser Ser Cys Gln
   1 5 10
<210> 669
   <211> 10
   <212> PRT
   <213> Homo sapiens
<400> 669
   Ser Cys Cys Gly Tyr Arg Pro Leu Cys Tyr
   1 5 10
<210> 670
   <211> 10
   <212> PRT
   <213> Homo sapiens
<400> 670
   Ser Cys Cys Val Pro Ala Ser Ser Cys Gln
   1 5 10
<210> 671
   <211> 10
   <212> PRT
   <213> Homo sapiens
<400> 671
   Ser Cys Cys Val Pro Thr Ser Ser Cys Gln
   1 5 10
<210> 672
   <211> 10
   <212> PRT
   <213> Homo sapiens
<400> 672
   Ser Cys Gly Cys Ser Lys Gly Ala Cys Gly
   1 5 10
<210> 673
   <211> 10
   <212> PRT
   <213> Homo sapiens
<400> 673
   Ser Cys Gly Gly Cys Asp Ser Ser Cys Gly
   1 5 10
<210> 674
   <211> 10
   <212> PRT
   <213> Homo sapiens
<400> 674
   Ser Cys Gly Gly Cys Gly Ser Gly Cys Gly
   1 5 10
<210> 675
   <211> 10
   <212> PRT
   <213> Homo sapiens
<400> 675
   Ser Cys Gly Gly Cys Gly Ser Ser Cys Gly
   1 5 10
<210> 676
   <211> 10
   <212> PRT
   <213> Homo sapiens
<400> 676
   Ser Cys Gly Gly Cys Lys Gly Gly Cys Gly
   1 5 10
<210> 677
   <211> 10
   <212> PRT
   <213> Homo sapiens
<400> 677
   Ser Cys Gly Gly Ser Lys Gly Cys Cys Gly
   1 5 10
<210> 678
   <211> 10
   <212> PRT
   <213> Homo sapiens
<400> 678
   Ser Cys Gly Ser Gly Cys Arg Gly Cys Gly
   1 5 10
<210> 679
   <211> 10
   <212> PRT
   <213> Homo sapiens
<400> 679
   Ser Cys Tyr Gly Cys Gly Tyr Gly Cys Ile
   1 5 10
<210> 680
   <211> 10
   <212> PRT
   <213> Homo sapiens
<400> 680
   Thr Cys Cys Val Pro Val Pro Ser Cys Gly
   1 5 10
<210> 681
   <211> 10
   <212> PRT
   <213> Homo sapiens
<400> 681
   Thr Cys Ser Asp Asp Ser Gly Ser Cys Cys
   1 5 10
<210> 682
   <211> 10
   <212> PRT
   <213> Homo sapiens
<400> 682
   Thr Cys Ser Glu Asp Ser Ser Ser Cys Cys
   1 5 10
<210> 683
   <211> 10
   <212> PRT
   <213> Homo sapiens
<400> 683
   Thr Cys Ser Glu Asp Ser Tyr Ser Cys Cys
   1 5 10
<210> 684
   <211> 10
   <212> PRT
   <213> Homo sapiens
<400> 684
   Thr Cys Ser Glu Ser Ser Pro Ser Cys Cys
   1 5 10
<210> 685
   <211> 10
   <212> PRT
   <213> Homo sapiens
<400> 685
   Thr Cys Ser Glu Ser Ser Ser Ser Cys Cys
   1 5 10
<210> 686
   <211> 10
   <212> PRT
   <213> Homo sapiens
<400> 686
   Thr Cys Ser Lys Asp Ser Ser Ser Cys Cys
   1 5 10
<210> 687
   <211> 10
   <212> PRT
   <213> Homo sapiens
<400> 687
   Thr Cys Ser Arg Leu Ser Ser Ala Cys Cys
   1 5 10
<210> 688
   <211> 10
   <212> PRT
   <213> Homo sapiens
<400> 688
   Val Cys Cys Gln Pro Thr Pro Ile Cys Asp
   1 5 10
<210> 689
   <211> 10
   <212> PRT
   <213> Homo sapiens
<400> 689
   Val Cys Ser Glu Asp Ser Ser Ser Cys Cys
   1 5 10
<210> 690
   <211> 10
   <212> PRT
   <213> Homo sapiens
<400> 690
   Val Cys Ser Gly Ala Ser Ser Leu Cys Cys
   1 5 10
<210> 691
   <211> 10
   <212> PRT
   <213> Homo sapiens
<400> 691
   Val Cys Ser Gly Ala Ser Ser Pro Cys Cys
   1 5 10
<210> 692
   <211> 10
   <212> PRT
   <213> Homo sapiens
<400> 692
   Val Cys Ser Gly Ala Ser Ser Ser Cys Cys
   1               5                   10
<210> 693
   <211> 10
   <212> PRT
   <213> Homo sapiens
<400> 693
   Val Cys Ser Gly Ala Ser Thr Ser Cys Cys
   1               5                   10
<210> 694
   <211> 10
   <212> PRT
   <213> Homo sapiens
<400> 694
   Val Cys Ser Gly Asp Ser Ser Cys Cys Gln
   1               5                   10
<210> 695
   <211> 10
   <212> PRT
   <213> Homo sapiens
<400> 695
   Val Cys Ser Gly Ile Ser Ser Ser Cys Cys
   1               5                   10
<210> 696
   <211> 10
   <212> PRT
   <213> Homo sapiens
<400> 696
   Tyr Cys Val Pro Ile Pro Ser Cys Cys Ala
   1               5                   10
<210> 697
   <211> 10
   <212> PRT
   <213> Homo sapiens
<400> 697
   Cys Ala Ser Ser Cys Cys Thr Pro Ser Cys
   1               5                   10
<210> 698
   <211> 10
   <212> PRT
   <213> Homo sapiens
<400> 698
   Cys Cys Asp Asn Cys Pro Pro Pro Cys His
   1 5 10
<210> 699
   <211> 10
   <212> PRT
   <213> Homo sapiens
<400> 699
   Cys Cys Glu Pro Cys Leu Pro Arg Gly Cys
   1 5 10
<210> 700
   <211> 10
   <212> PRT
   <213> Homo sapiens
<400> 700
   Cys Cys Gly Ala Ala Ser Ser Cys Cys Arg
   1 5 10
<210> 701
   <211> 10
   <212> PRT
   <213> Homo sapiens
<400> 701
   Cys Cys Gly Cys Gly Gly Ser Gly Cys Gly
   1 5 10
<210> 702
   <211> 10
   <212> PRT
   <213> Homo sapiens
<400> 702
   Cys Cys Gly Pro Ser Ser Ser Cys Cys Gln
   1 5 10
<210> 703
   <211> 10
   <212> PRT
   <213> Homo sapiens
<400> 703
   Cys Cys Gly Ser Gly Cys Gly Gly Cys Gly
   1 5 10
<210> 704
   <211> 10
   <212> PRT
   <213> Homo sapiens
<400> 704
   Cys Cys Lys Pro Tyr Cys Ser Gln Cys Ser
   1               5                   10
<210> 705
   <211> 10
   <212> PRT
   <213> Homo sapiens
<400> 705
   Cys Cys Met Pro Val Ser Ser Cys Cys Ala
   1               5                   10
<210> 706
   <211> 10
   <212> PRT
   <213> Homo sapiens
<400> 706
   Cys Cys Asn Tyr Tyr Arg Asn Cys Cys Gly
   1               5                   10
<210> 707
   <211> 10
   <212> PRT
   <213> Homo sapiens
<400> 707
   Cys Cys Pro Ser Cys Val Val Ser Ser Cys
   1               5                   10
<210> 708
   <211> 10
   <212> PRT
   <213> Homo sapiens
<400> 708
   Cys Cys Pro Ser Tyr Cys Val Ser Ser Cys
   1               5                   10
<210> 709
   <211> 10
   <212> PRT
   <213> Homo sapiens
<400> 709
   Cys Cys Gln Pro Ile Cys Gly Ser Ser Cys
   1               5                   10
<210> 710
   <211> 10
   <212> PRT
   <213> Homo sapiens
<400> 710
   Cys Cys Gln Pro Ile Cys Val Thr Ser Cys
   1               5                   10
<210> 711
   <211> 10
   <212> PRT
   <213> Homo sapiens
<400> 711
   Cys Cys Gln Pro Thr Cys Leu Ser Ser Cys
   1               5                   10
<210> 712
   <211> 10
   <212> PRT
   <213> Homo sapiens
<400> 712
   Cys Cys Gln Pro Thr Cys Leu Thr Ser Cys
   1               5                   10
<210> 713
   <211> 10
   <212> PRT
   <213> Homo sapiens
<400> 713
   Cys Cys Gln Pro Thr Cys Val Ala Ser Cys
   1               5                   10
<210> 714
   <211> 10
   <212> PRT
   <213> Homo sapiens
<400> 714
   Cys Cys Gln Pro Thr Cys Val Thr Ser Cys
   1               5                   10
<210> 715
   <211> 10
   <212> PRT
   <213> Homo sapiens
<400> 715
   Cys Cys Gln Pro Tyr Cys His Pro Thr Cys
   1 5 10
<210> 716
   <211> 10
   <212> PRT
   <213> Homo sapiens
<400> 716
   Cys Cys Gln Gln Ser Ser Cys Val Ser Cys
   1 5 10
<210> 717
   <211> 10
   <212> PRT
   <213> Homo sapiens
<400> 717
   Cys Cys Gln Ser Ser Cys Phe Lys Pro Cys
   1 5 10
<210> 718
   <211> 10
   <212> PRT
   <213> Homo sapiens
<400> 718
   Cys Cys Gln Ser Ser Cys Ser Lys Pro Cys
   1 5 10
<210> 719
   <211> 10
   <212> PRT
   <213> Homo sapiens
<400> 719
   Cys Cys Gln Ser Ser Cys Tyr Lys Pro Cys
   1 5 10
<210> 720
   <211> 10
   <212> PRT
   <213> Homo sapiens
<400> 720
   Cys Cys Gln Thr Ile Cys Arg Ser Thr Cys
   1 5 10
<210> 721
   <211> 10
   <212> PRT
   <213> Homo sapiens
<400> 721
   Cys Cys Gln Thr Thr Cys His Pro Ser Cys
   1               5                   10
<210> 722
   <211> 10
   <212> PRT
   <213> Homo sapiens
<400> 722
   Cys Cys Gln Thr Thr Cys Arg Pro Ser Cys
   1               5                   10
<210> 723
   <211> 10
   <212> PRT
   <213> Homo sapiens
<400> 723
   Cys Cys Arg Val Pro Thr Cys Ser Cys Ser
   1               5                   10
<210> 724
   <211> 10
   <212> PRT
   <213> Homo sapiens
<400> 724
   Cys Cys Ser Pro Gly Cys Gln Pro Thr Cys
   1               5                   10
<210> 725
   <211> 10
   <212> PRT
   <213> Homo sapiens
<400> 725
   Cys Cys Ser Ser Gly Cys Gly Ser Ser Cys
   1               5                   10
<210> 726
   <211> 10
   <212> PRT
   <213> Homo sapiens
<400> 726
   Cys Cys Ser Ser Ser Cys Gly Ser Cys Gly
   1               5                   10
<210> 727
   <211> 10
   <212> PRT
   <213> Homo sapiens
<400> 727
   Cys Cys Thr Gln Glu Gln Asn Cys Cys Glu
   1               5                   10
<210> 728
   <211> 10
   <212> PRT
   <213> Homo sapiens
<400> 728
   Cys Cys Val Pro Ile Pro Ser Cys Cys Ala
   1               5                   10
<210> 729
   <211> 10
   <212> PRT
   <213> Homo sapiens
<400> 729
   Cys Cys Val Pro Ile Ser Ser Cys Cys Ala
   1               5                   10
<210> 730
   <211> 10
   <212> PRT
   <213> Homo sapiens
<400> 730
   Cys Cys Val Pro Val Cys Tyr Gln Cys Lys
   1               5                   10
<210> 731
   <211> 10
   <212> PRT
   <213> Homo sapiens
<400> 731
   Cys Cys Val Pro Val Pro Ser Cys Cys Ala
   1               5                   10
<210> 732
   <211> 10
   <212> PRT
   <213> Homo sapiens
<400> 732
   Cys Cys Val Pro Val Pro Ser Cys Cys Val
   1               5                   10
<210> 733
   <211> 10
   <212> PRT
   <213> Homo sapiens
<400> 733
   Cys Cys Val Pro Val Ser Ser Cys Cys Ala
   1               5                   10
<210> 734
   <211> 10
   <212> PRT
   <213> Homo sapiens
<400> 734
   Cys Asp Ser Ser Cys Cys Gln Pro Ser Cys
   1               5                   10
<210> 735
   <211> 10
   <212> PRT
   <213> Homo sapiens
<400> 735
   Cys Asp Thr Cys Pro Pro Pro Cys Cys Lys
   1               5                   10
<210> 736
   <211> 10
   <212> PRT
   <213> Homo sapiens
<400> 736
   Cys Glu Pro Cys Arg Arg Pro Val Cys Cys
   1               5                   10
<210> 737
   <211> 10
   <212> PRT
   <213> Homo sapiens
<400> 737
   Cys Glu Pro Ser Cys Cys Gln Pro Val Cys
   1               5                   10
<210> 738
   <211> 10
   <212> PRT
   <213> Homo sapiens
<400> 738
   Cys Glu Pro Ser Cys Cys Ser Ala Val Cys
   1 5 10
<210> 739
   <211> 10
   <212> PRT
   <213> Homo sapiens
<400> 739
   Cys Glu Thr Ser Cys Cys Gln Pro Ser Cys
   1 5 10
<210> 740
   <211> 10
   <212> PRT
   <213> Homo sapiens
<400> 740
   Cys Glu Thr Thr Cys Cys Arg Thr Thr Cys
   1 5 10
<210> 741
   <211> 10
   <212> PRT
   <213> Homo sapiens
<400> 741
   Cys Phe Ser Gly Cys Gly Ser Ser Cys Cys
   1 5 10
<210> 742
   <211> 10
   <212> PRT
   <213> Homo sapiens
<400> 742
   Cys Gly Cys Ser Gln Ser Asn Cys Cys Lys
   1 5 10
<210> 743
   <211> 10
   <212> PRT
   <213> Homo sapiens
<400> 743
   Cys Gly Cys Ser Gln Ser Ser Cys Cys Lys
   1 5 10
<210> 744
   <211> 10
   <212> PRT
   <213> Homo sapiens
<400> 744
   Cys Gly Gly Cys Gly Gly Cys Gly Gly Cys
   1 5 10
<210> 745
   <211> 10
   <212> PRT
   <213> Homo sapiens
<400> 745
   Cys Gly Gly Cys Gly Gly Gly Cys Cys Gly
   1 5 10
<210> 746
   <211> 10
   <212> PRT
   <213> Homo sapiens
<400> 746
   Cys Gly Gly Cys Gly Ser Gly Cys Cys Val
   1 5 10
<210> 747
   <211> 10
   <212> PRT
   <213> Homo sapiens
<400> 747
   Cys Gly Gly Cys Gly Ser Ser Cys Cys Val
   1 5 10
<210> 748
   <211> 10
   <212> PRT
   <213> Homo sapiens
<400> 748
   Cys Gly Gly Gly Cys Cys Gly Ser Ser Cys
   1 5 10
<210> 749
   <211> 10
   <212> PRT
   <213> Homo sapiens
<400> 749
   Cys Gly Gly Ser Cys Cys Gly Ser Ser Cys
   1 5 10
<210> 750
   <211> 10
   <212> PRT
   <213> Homo sapiens
<400> 750
   Cys Gly Gln Ser Cys Cys Arg Pro Ala Cys
   1               5                   10
<210> 751
   <211> 10
   <212> PRT
   <213> Homo sapiens
<400> 751
   Cys Gly Gln Ser Cys Cys Arg Pro Val Cys
   1               5                   10
<210> 752
   <211> 10
   <212> PRT
   <213> Homo sapiens
<400> 752
   Cys Gly Ser Cys Gly Cys Ser Gln Cys Asn
   1               5                   10
<210> 753
   <211> 10
   <212> PRT
   <213> Homo sapiens
<400> 753
   Cys Gly Ser Cys Gly Cys Ser Gln Cys Ser
   1               5                   10
<210> 754
   <211> 10
   <212> PRT
   <213> Homo sapiens
<400> 754
   Cys Gly Ser Phe Cys Cys Gln Ser Ser Cys
   1               5                   10
<210> 755
   <211> 10
   <212> PRT
   <213> Homo sapiens
<400> 755
   Cys Gly Ser Gly Cys Cys Val Pro Val Cys
   1               5                   10
<210> 756
   <211> 10
   <212> PRT
   <213> Homo sapiens
<400> 756
   Cys Gly Ser Ser Cys Cys Gly Ser Gly Cys
   1               5                   10
<210> 757
   <211> 10
   <212> PRT
   <213> Homo sapiens
<400> 757
   Cys Gly Ser Ser Cys Cys Gln Pro Cys Tyr
   1               5                   10
<210> 758
   <211> 10
   <212> PRT
   <213> Homo sapiens
<400> 758
   Cys Gly Ser Ser Cys Cys Gln Pro Ile Cys
   1               5                   10
<210> 759
   <211> 10
   <212> PRT
   <213> Homo sapiens
<400> 759
   Cys Gly Ser Ser Cys Cys Gln Pro Ser Cys
   1               5                   10
<210> 760
   <211> 10
   <212> PRT
   <213> Homo sapiens
<400> 760
   Cys Gly Ser Ser Cys Cys Gln Ser Ser Cys
   1               5                   10
<210> 761
   <211> 10
   <212> PRT
   <213> Homo sapiens
<400> 761
   Cys Gly Ser Ser Cys Cys Val Pro Ile Cys
   1               5                   10
<210> 762
   <211> 10
   <212> PRT
   <213> Homo sapiens
<400> 762
   Cys Gly Ser Ser Cys Cys Val Pro Val Cys
   1               5                   10
<210> 763
   <211> 10
   <212> PRT
   <213> Homo sapiens
<400> 763
   Cys Gly Ser Ser Cys Ser Gln Cys Ser Cys
   1               5                   10
<210> 764
   <211> 10
   <212> PRT
   <213> Homo sapiens
<400> 764
   Cys Gly Tyr Gly Ser Cys Cys Gly Cys Gly
   1               5                   10
<210> 765
   <211> 10
   <212> PRT
   <213> Homo sapiens
<400> 765
   Cys His Pro Arg Cys Cys Ile Ser Ser Cys
   1               5                   10
<210> 766
   <211> 10
   <212> PRT
   <213> Homo sapiens
<400> 766
   Cys His Pro Ser Cys Cys Glu Ser Ser Cys
   1               5                   10
<210> 767
   <211> 10
   <212> PRT
   <213> Homo sapiens
<400> 767
   Cys His Pro Ser Cys Cys Ile Ser Ser Cys
   1 5 10
<210> 768
   <211> 10
   <212> PRT
   <213> Homo sapiens
<400> 768
   Cys His Pro Thr Cys Cys Gln Asn Thr Cys
   1 5 10
<210> 769
   <211> 10
   <212> PRT
   <213> Homo sapiens
<400> 769
   Cys His Pro Thr Cys Cys Gln Thr Ile Cys
   1 5 10
<210> 770
   <211> 10
   <212> PRT
   <213> Homo sapiens
<400> 770
   Cys His Pro Val Cys Cys Gln Thr Thr Cys
   1 5 10
<210> 771
   <211> 10
   <212> PRT
   <213> Homo sapiens
<400> 771
   Cys His Pro Val Cys Lys Ser Thr Cys Cys
   1 5 10
<210> 772
   <211> 10
   <212> PRT
   <213> Homo sapiens
<400> 772
   Cys His Pro Val Cys Arg Ser Thr Cys Cys
   1 5 10
<210> 773
   <211> 10
   <212> PRT
   <213> Homo sapiens
<400> 773
   Cys Ile Ser Ser Cys Cys His Pro Ser Cys
   1 5 10
<210> 774
   <211> 10
   <212> PRT
   <213> Homo sapiens
<400> 774
   Cys Ile Ser Ser Cys Cys Lys Pro Ser Cys
   1 5 10
<210> 775
   <211> 10
   <212> PRT
   <213> Homo sapiens
<400> 775
   Cys Ile Ser Ser Cys Cys Arg Pro Ser Cys
   1 5 10
<210> 776
   <211> 10
   <212> PRT
   <213> Homo sapiens
<400> 776
   Cys Ile Ser Ser Cys Thr Pro Ser Cys Cys
   1 5 10
<210> 777
   <211> 10
   <212> PRT
   <213> Homo sapiens
<400> 777
   Cys Ile Ser Ser Ser Cys Cys Pro Ser Cys
   1 5 10
<210> 778
   <211> 10
   <212> PRT
   <213> Homo sapiens
<400> 778
   Cys Lys Ala Val Cys Cys Val Pro Thr Cys
   1 5 10
<210> 779
   <211> 10
   <212> PRT
   <213> Homo sapiens
<400> 779
   Cys Lys Pro Cys Cys Ser Gln Ala Ser Cys
   1 5 10
<210> 780
   <211> 10
   <212> PRT
   <213> Homo sapiens
<400> 780
   Cys Lys Pro Cys Cys Ser Gln Ser Arg Cys
   1 5 10
<210> 781
   <211> 10
   <212> PRT
   <213> Homo sapiens
<400> 781
   Cys Lys Pro Cys Cys Ser Gln Ser Ser Cys
   1 5 10
<210> 782
   <211> 10
   <212> PRT
   <213> Homo sapiens
<400> 782
   Cys Lys Pro Cys Cys Ser Ser Ser Gly Cys
   1 5 10
<210> 783
   <211> 10
   <212> PRT
   <213> Homo sapiens
<400> 783
   Cys Lys Pro Cys Ser Cys Phe Ser Gly Cys
   1 5 10
<210> 784
   <211> 10
   <212> PRT
   <213> Homo sapiens
<400> 784
   Cys Lys Pro Cys Ser Cys Ser Ser Gly Cys
   1 5 10
<210> 785
   <211> 10
   <212> PRT
   <213> Homo sapiens
<400> 785
   Cys Lys Pro Cys Tyr Cys Ser Ser Gly Cys
   1 5 10
<210> 786
   <211> 10
   <212> PRT
   <213> Homo sapiens
<400> 786
   Cys Lys Pro Ile Cys Cys Val Pro Val Cys
   1 5 10
<210> 787
   <211> 10
   <212> PRT
   <213> Homo sapiens
<400> 787
   Cys Lys Pro Gln Cys Cys Gln Ser Val Cys
   1 5 10
<210> 788
   <211> 10
   <212> PRT
   <213> Homo sapiens
<400> 788
   Cys Lys Pro Ser Cys Cys Gln Thr Thr Cys
   1 5 10
<210> 789
   <211> 10
   <212> PRT
   <213> Homo sapiens
<400> 789
   Cys Lys Pro Val Cys Cys Ala Pro Thr Cys
   1 5 10
<210> 790
   <211> 10
   <212> PRT
   <213> Homo sapiens
<400> 790
   Cys Lys Pro Val Cys Cys Lys Pro Ile Cys
   1 5 10
<210> 791
   <211> 10
   <212> PRT
   <213> Homo sapiens
<400> 791
   Cys Lys Pro Val Cys Cys Lys Ser Ile Cys
   1 5 10
<210> 792
   <211> 10
   <212> PRT
   <213> Homo sapiens
<400> 792
   Cys Lys Pro Val Cys Cys Leu Pro Thr Cys
   1 5 10
<210> 793
   <211> 10
   <212> PRT
   <213> Homo sapiens
<400> 793
   Cys Lys Pro Val Cys Cys Val Pro Thr Cys
   1 5 10
<210> 794
   <211> 10
   <212> PRT
   <213> Homo sapiens
<400> 794
   Cys Lys Pro Val Cys Cys Val Pro Val Cys
   1 5 10
<210> 795
   <211> 10
   <212> PRT
   <213> Homo sapiens
<400> 795
   Cys Lys Pro Val Cys Cys Val Ser Thr Cys
   1 5 10
<210> 796
   <211> 10
   <212> PRT
   <213> Homo sapiens
<400> 796
   Cys Lys Pro Tyr Cys Cys Gln Ser Ser Cys
   1 5 10
<210> 797
   <211> 10
   <212> PRT
   <213> Homo sapiens
<400> 797
   Cys Lys Pro Tyr Cys Ser Gln Cys Ser Cys
   1 5 10
<210> 798
   <211> 10
   <212> PRT
   <213> Homo sapiens
<400> 798
   Cys Lys Ser Asn Cys Cys Lys Pro Val Cys
   1 5 10
<210> 799
   <211> 10
   <212> PRT
   <213> Homo sapiens
<400> 799
   Cys Lys Thr Val Cys Cys Lys Pro Val Cys
   1 5 10
<210> 800
   <211> 10
   <212> PRT
   <213> Homo sapiens
<400> 800
   Cys Leu Pro Pro Cys Cys Val Val Ser Cys
   1 5 10
<210> 801
   <211> 10
   <212> PRT
   <213> Homo sapiens
<400> 801
   Cys Leu Thr Ser Cys Cys Gln Pro Ser Cys
   1 5 10
<210> 802
   <211> 10
   <212> PRT
   <213> Homo sapiens
<400> 802
   Cys Asn Pro Cys Cys Ser Gln Ser Ser Cys
   1 5 10
<210> 803
   <211> 10
   <212> PRT
   <213> Homo sapiens
<400> 803
   Cys Pro Glu Ser Cys Cys Glu Leu Pro Cys
   1 5 10
<210> 804
   <211> 10
   <212> PRT
   <213> Homo sapiens
<400> 804
   Cys Pro Glu Ser Cys Cys Glu Pro His Cys
   1 5 10
<210> 805
   <211> 10
   <212> PRT
   <213> Homo sapiens
<400> 805
   Cys Pro Glu Ser Cys Cys Glu Pro Pro Cys
   1 5 10
<210> 806
   <211> 10
   <212> PRT
   <213> Homo sapiens
<400> 806
   Cys Pro Phe Ser Cys Pro Thr Thr Cys Cys
   1 5 10
<210> 807
   <211> 10
   <212> PRT
   <213> Homo sapiens
<400> 807
   Cys Pro Gly Asp Cys Phe Thr Cys Cys Thr
   1 5 10
<210> 808
   <211> 10
   <212> PRT
   <213> Homo sapiens
<400> 808
   Cys Pro Ser Cys Val Val Ser Ser Cys Cys
   1 5 10
<210> 809
   <211> 10
   <212> PRT
   <213> Homo sapiens
<400> 809
   Cys Pro Ser Tyr Cys Val Ser Ser Cys Cys
   1 5 10
<210> 810
   <211> 10
   <212> PRT
   <213> Homo sapiens
<400> 810
   Cys Pro Thr Thr Cys Cys Arg Thr Thr Cys
   1 5 10
<210> 811
   <211> 10
   <212> PRT
   <213> Homo sapiens
<400> 811
   Cys Gln Glu Thr Cys Cys Arg Pro Ser Cys
   1 5 10
<210> 812
   <211> 10
   <212> PRT
   <213> Homo sapiens
<400> 812
   Cys Gln His Ala Cys Cys Val Pro Val Cys
   1 5 10
<210> 813
   <211> 10
   <212> PRT
   <213> Homo sapiens
<400> 813
   Cys Gln Asn Thr Cys Cys Arg Thr Thr Cys
   1 5 10
<210> 814
   <211> 10
   <212> PRT
   <213> Homo sapiens
<400> 814
   Cys Gln Pro Ala Cys Cys Gln Pro Thr Cys
   1 5 10
<210> 815
   <211> 10
   <212> PRT
   <213> Homo sapiens
<400> 815
   Cys Gln Pro Ala Cys Cys Thr Ala Ser Cys
   1 5 10
<210> 816
   <211> 10
   <212> PRT
   <213> Homo sapiens
<400> 816
   Cys Gln Pro Ala Cys Cys Thr Ser Ser Cys
   1 5 10
<210> 817
   <211> 10
   <212> PRT
   <213> Homo sapiens
<400> 817
   Cys Gln Pro Ala Cys Cys Thr Thr Ser Cys
   1 5 10
<210> 818
   <211> 10
   <212> PRT
   <213> Homo sapiens
<400> 818
   Cys Gln Pro Ala Cys Cys Val Pro Val Cys
   1 5 10
<210> 819
   <211> 10
   <212> PRT
   <213> Homo sapiens
<400> 819
   Cys Gln Pro Ala Cys Cys Val Ser Ser Cys
   1 5 10
<210> 820
   <211> 10
   <212> PRT
   <213> Homo sapiens
<400> 820
   Cys Gln Pro Cys Cys His Pro Thr Cys Tyr
   1               5                   10
<210> 821
   <211> 10
   <212> PRT
   <213> Homo sapiens
<400> 821
   Cys Gln Pro Cys Cys Arg Pro Thr Ser Cys
   1               5                   10
<210> 822
   <211> 10
   <212> PRT
   <213> Homo sapiens
<400> 822
   Cys Gln Pro Ile Cys Cys Gly Ser Ser Cys
   1               5                   10
<210> 823
   <211> 10
   <212> PRT
   <213> Homo sapiens
<400> 823
   Cys Gln Pro Ile Cys Gly Ser Ser Cys Cys
   1               5                   10
<210> 824
   <211> 10
   <212> PRT
   <213> Homo sapiens
<400> 824
   Cys Gln Pro Ile Cys Val Thr Ser Cys Cys
   1               5                   10
<210> 825
   <211> 10
   <212> PRT
   <213> Homo sapiens
<400> 825
   Cys Gln Pro Asn Cys Cys Arg Pro Ser Cys
   1 5 10
<210> 826
   <211> 10
   <212> PRT
   <213> Homo sapiens
<400> 826
   Cys Gln Pro Arg Cys Cys Glu Thr Ser Cys
   1 5 10
<210> 827
   <211> 10
   <212> PRT
   <213> Homo sapiens
<400> 827
   Cys Gln Pro Ser Cys Cys Arg Pro Ala Cys
   1 5 10
<210> 828
   <211> 10
   <212> PRT
   <213> Homo sapiens
<400> 828
   Cys Gln Pro Ser Cys Cys Ser Thr Pro Cys
   1 5 10
<210> 829
   <211> 10
   <212> PRT
   <213> Homo sapiens
<400> 829
   Cys Gln Pro Ser Cys Cys Ser Thr Thr Cys
   1 5 10
<210> 830
   <211> 10
   <212> PRT
   <213> Homo sapiens
<400> 830
   Cys Gln Pro Ser Cys Cys Val Pro Ser Cys
   1 5 10
<210> 831
   <211> 10
   <212> PRT
   <213> Homo sapiens
<400> 831
   Cys Gln Pro Ser Cys Cys Val Ser Ser Cys
   1 5 10
<210> 832
   <211> 10
   <212> PRT
   <213> Homo sapiens
<400> 832
   Cys Gln Pro Thr Cys Cys Gly Ser Ser Cys
   1 5 10
<210> 833
   <211> 10
   <212> PRT
   <213> Homo sapiens
<400> 833
   Cys Gln Pro Thr Cys Cys His Pro Ser Cys
   1 5 10
<210> 834
   <211> 10
   <212> PRT
   <213> Homo sapiens
<400> 834
   Cys Gln Pro Thr Cys Cys Gln Pro Thr Cys
   1 5 10
<210> 835
   <211> 10
   <212> PRT
   <213> Homo sapiens
<400> 835
   Cys Gln Pro Thr Cys Cys Arg Pro Arg Cys
   1 5 10
<210> 836
   <211> 10
   <212> PRT
   <213> Homo sapiens
<400> 836
   Cys Gln Pro Thr Cys Cys Arg Pro Ser Cys
   1 5 10
<210> 837
   <211> 10
   <212> PRT
   <213> Homo sapiens
<400> 837
   Cys Gln Pro Thr Cys Cys Arg Thr Thr Cys
   1               5                   10
<210> 838
   <211> 10
   <212> PRT
   <213> Homo sapiens
<400> 838
   Cys Gln Pro Thr Cys Leu Ser Ser Cys Cys
   1               5                   10
<210> 839
   <211> 10
   <212> PRT
   <213> Homo sapiens
<400> 839
   Cys Gln Pro Thr Cys Leu Thr Ser Cys Cys
   1               5                   10
<210> 840
   <211> 10
   <212> PRT
   <213> Homo sapiens
<400> 840
   Cys Gln Pro Thr Cys Val Ala Ser Cys Cys
   1               5                   10
<210> 841
   <211> 10
   <212> PRT
   <213> Homo sapiens
<400> 841
   Cys Gln Pro Thr Cys Val Thr Ser Cys Cys
   1               5                   10
<210> 842
   <211> 10
   <212> PRT
   <213> Homo sapiens
<400> 842
   Cys Gln Pro Val Cys Cys Gln Pro Thr Cys
   1               5                   10
<210> 843
   <211> 10
   <212> PRT
   <213> Homo sapiens
<400> 843
   Cys Gln Pro Tyr Cys His Pro Thr Cys Cys
   1 5 10
<210> 844
   <211> 10
   <212> PRT
   <213> Homo sapiens
<400> 844
   Cys Gln Gln Ala Cys Cys Met Pro Val Cys
   1 5 10
<210> 845
   <211> 10
   <212> PRT
   <213> Homo sapiens
<400> 845
   Cys Gln Gln Ala Cys Cys Val Pro Ile Cys
   1 5 10
<210> 846
   <211> 10
   <212> PRT
   <213> Homo sapiens
<400> 846
   Cys Gln Gln Ala Cys Cys Val Pro Val Cys
   1 5 10
<210> 847
   <211> 10
   <212> PRT
   <213> Homo sapiens
<400> 847
   Cys Gln Gln Ser Cys Cys Val Pro Val Cys
   1 5 10
<210> 848
   <211> 10
   <212> PRT
   <213> Homo sapiens
<400> 848
   Cys Gln Gln Ser Cys Cys Val Ser Val Cys
   1 5 10
<210> 849
   <211> 10
   <212> PRT
   <213> Homo sapiens
<400> 849
   Cys Gln Ser Met Cys Cys Gln Pro Thr Cys
   1               5                   10
<210> 850
   <211> 10
   <212> PRT
   <213> Homo sapiens
<400> 850
   Cys Gln Ser Asn Cys Cys Val Pro Val Cys
   1               5                   10
<210> 851
   <211> 10
   <212> PRT
   <213> Homo sapiens
<400> 851
   Cys Gln Ser Ser Cys Cys Lys Pro Cys Ser
   1               5                   10
<210> 852
   <211> 10
   <212> PRT
   <213> Homo sapiens
<400> 852
   Cys Gln Ser Ser Cys Cys Gln Ser Ser Cys
   1               5                   10
<210> 853
   <211> 10
   <212> PRT
   <213> Homo sapiens
<400> 853
   Cys Gln Ser Ser Cys Cys Val Pro Val Cys
   1               5                   10
<210> 854
   <211> 10
   <212> PRT
   <213> Homo sapiens
<400> 854
   Cys Gln Ser Ser Cys Phe Lys Pro Cys Cys
   1 5 10
<210> 855
   <211> 10
   <212> PRT
   <213> Homo sapiens
<400> 855
   Cys Gln Ser Ser Cys Ser Lys Pro Cys Cys
   1 5 10
<210> 856
   <211> 10
   <212> PRT
   <213> Homo sapiens
<400> 856
   Cys Gln Ser Val Cys Cys Gln Pro Thr Cys
   1 5 10
<210> 857
   <211> 10
   <212> PRT
   <213> Homo sapiens
<400> 857
   Cys Gln Thr Ile Cys Arg Ser Thr Cys Cys
   1 5 10
<210> 858
   <211> 10
   <212> PRT
   <213> Homo sapiens
<400> 858
   Cys Gln Thr Thr Cys Cys Arg Pro Ser Cys
   1 5 10
<210> 859
   <211> 10
   <212> PRT
   <213> Homo sapiens
<400> 859
   Cys Gln Thr Thr Cys Cys Arg Thr Thr Cys
   1 5 10
<210> 860
   <211> 10
   <212> PRT
   <213> Homo sapiens
<400> 860
   Cys Arg Ala Thr Cys Cys Arg Pro Ser Cys
   1 5 10
<210> 861
   <211> 10
   <212> PRT
   <213> Homo sapiens
<400> 861
   Cys Arg Gly Cys Gly Pro Ser Cys Cys Ala
   1 5 10
<210> 862
   <211> 10
   <212> PRT
   <213> Homo sapiens
<400> 862
   Cys Arg Pro Ala Cys Cys Glu Thr Thr Cys
   1 5 10
<210> 863
   <211> 10
   <212> PRT
   <213> Homo sapiens
<400> 863
   Cys Arg Pro Ala Cys Cys Gln Asn Thr Cys
   1 5 10
<210> 864
   <211> 10
   <212> PRT
   <213> Homo sapiens
<400> 864
   Cys Arg Pro Cys Cys Trp Ala Thr Thr Cys
   1 5 10
<210> 865
   <211> 10
   <212> PRT
   <213> Homo sapiens
<400> 865
   Cys Arg Pro Ile Cys Arg Pro Ala Cys Cys
   1 5 10
<210> 866
   <211> 10
   <212> PRT
   <213> Homo sapiens
<400> 866
   Cys Arg Pro Leu Cys Cys Gln Thr Thr Cys
   1 5 10
<210> 867
   <211> 10
   <212> PRT
   <213> Homo sapiens
<400> 867
   Cys Arg Pro Gln Cys Cys Gln Ser Val Cys
   1 5 10
<210> 868
   <211> 10
   <212> PRT
   <213> Homo sapiens
<400> 868
   Cys Arg Pro Gln Cys Cys Gln Thr Thr Cys
   1 5 10
<210> 869
   <211> 10
   <212> PRT
   <213> Homo sapiens
<400> 869
   Cys Arg Pro Arg Cys Cys Ile Ser Ser Cys
   1 5 10
<210> 870
   <211> 10
   <212> PRT
   <213> Homo sapiens
<400> 870
   Cys Arg Pro Ser Cys Cys Glu Ser Ser Cys
   1 5 10
<210> 871
   <211> 10
   <212> PRT
   <213> Homo sapiens
<400> 871
   Cys Arg Pro Ser Cys Cys Glu Thr Thr Cys
   1 5 10
<210> 872
   <211> 10
   <212> PRT
   <213> Homo sapiens
<400> 872
   Cys Arg Pro Ser Cys Cys Ile Ser Ser Cys
   1 5 10
<210> 873
   <211> 10
   <212> PRT
   <213> Homo sapiens
<400> 873
   Cys Arg Pro Ser Cys Cys Lys Pro Gln Cys
   1 5 10
<210> 874
   <211> 10
   <212> PRT
   <213> Homo sapiens
<400> 874
   Cys Arg Pro Ser Cys Cys Met Ser Ser Cys
   1 5 10
<210> 875
   <211> 10
   <212> PRT
   <213> Homo sapiens
<400> 875
   Cys Arg Pro Ser Cys Cys Gln Thr Thr Cys
   1 5 10
<210> 876
   <211> 10
   <212> PRT
   <213> Homo sapiens
<400> 876
   Cys Arg Pro Ser Cys Cys Arg Pro Ser Cys
   1 5 10
<210> 877
   <211> 10
   <212> PRT
   <213> Homo sapiens
<400> 877
   Cys Arg Pro Ser Cys Cys Val Ser Arg Cys
   1 5 10
<210> 878
   <211> 10
   <212> PRT
   <213> Homo sapiens
<400> 878
   Cys Arg Pro Ser Cys Cys Val Ser Ser Cys
   1 5 10
<210> 879
   <211> 10
   <212> PRT
   <213> Homo sapiens
<400> 879
   Cys Arg Pro Thr Cys Cys Glu Thr Thr Cys
   1 5 10
<210> 880
   <211> 10
   <212> PRT
   <213> Homo sapiens
<400> 880
   Cys Arg Pro Thr Cys Cys Gln Asn Thr Cys
   1 5 10
<210> 881
   <211> 10
   <212> PRT
   <213> Homo sapiens
<400> 881
   Cys Arg Pro Thr Cys Cys Gln Thr Thr Cys
   1 5 10
<210> 882
   <211> 10
   <212> PRT
   <213> Homo sapiens
<400> 882
   Cys Arg Pro Val Cys Cys Asp Pro Cys Ser
   1 5 10
<210> 883
   <211> 10
   <212> PRT
   <213> Homo sapiens
<400> 883
   Cys Arg Pro Val Cys Cys Gln Thr Thr Cys
   1 5 10
<210> 884
   <211> 10
   <212> PRT
   <213> Homo sapiens
<400> 884
   Cys Arg Pro Val Cys Gln Pro Ala Cys Cys
   1 5 10
<210> 885
   <211> 10
   <212> PRT
   <213> Homo sapiens
<400> 885
   Cys Arg Pro Val Cys Arg Pro Ala Cys Cys
   1 5 10
<210> 886
   <211> 10
   <212> PRT
   <213> Homo sapiens
<400> 886
   Cys Arg Pro Val Cys Arg Pro Thr Cys Cys
   1 5 10
<210> 887
   <211> 10
   <212> PRT
   <213> Homo sapiens
<400> 887
   Cys Arg Pro Val Cys Arg Ser Thr Cys Cys
   1 5 10
<210> 888
   <211> 10
   <212> PRT
   <213> Homo sapiens
<400> 888
   Cys Arg Pro Tyr Cys Cys Glu Ser Ser Cys
   1 5 10
<210> 889
   <211> 10
   <212> PRT
   <213> Homo sapiens
<400> 889
   Cys Arg Arg Pro Val Cys Cys Asp Pro Cys
   1 5 10
<210> 890
   <211> 10
   <212> PRT
   <213> Homo sapiens
<400> 890
   Cys Arg Ser Gln Cys Cys Gln Ser Val Cys
   1 5 10
<210> 891
   <211> 10
   <212> PRT
   <213> Homo sapiens
<400> 891
   Cys Arg Thr Thr Cys Cys His Pro Ser Cys
   1 5 10
<210> 892
   <211> 10
   <212> PRT
   <213> Homo sapiens
<400> 892
   Cys Arg Thr Thr Cys Cys Gln Pro Ile Cys
   1 5 10
<210> 893
   <211> 10
   <212> PRT
   <213> Homo sapiens
<400> 893
   Cys Arg Thr Thr Cys Cys Gln Pro Thr Cys
   1 5 10
<210> 894
   <211> 10
   <212> PRT
   <213> Homo sapiens
<400> 894
   Cys Arg Thr Thr Cys Cys Arg Pro Ser Cys
   1 5 10
<210> 895
   <211> 10
   <212> PRT
   <213> Homo sapiens
<400> 895
   Cys Arg Thr Thr Cys Cys Arg Thr Thr Cys
   1 5 10
<210> 896
   <211> 10
   <212> PRT
   <213> Homo sapiens
<400> 896
   Cys Ser Cys Ser Ser Cys Gly Ser Cys Ala
   1 5 10
<210> 897
   <211> 10
   <212> PRT
   <213> Homo sapiens
<400> 897
   Cys Ser Cys Ser Ser Cys Gly Ser Cys Gly
   1 5 10
<210> 898
   <211> 10
   <212> PRT
   <213> Homo sapiens
<400> 898
   Cys Ser Cys Thr Ser Cys Gly Ser Cys Gly
   1 5 10
<210> 899
   <211> 10
   <212> PRT
   <213> Homo sapiens
<400> 899
   Cys Ser Pro Ala Cys Gln Pro Thr Cys Cys
   1 5 10
<210> 900
   <211> 10
   <212> PRT
   <213> Homo sapiens
<400> 900
   Cys Ser Pro Gly Cys Gln Pro Thr Cys Cys
   1 5 10
<210> 901
   <211> 10
   <212> PRT
   <213> Homo sapiens
<400> 901
   Cys Ser Pro Ser Cys Cys Gln Thr Thr Cys
   1 5 10
<210> 902
   <211> 10
   <212> PRT
   <213> Homo sapiens
<400> 902
   Cys Ser Gln Cys Ser Cys Tyr Lys Pro Cys
   1 5 10
<210> 903
   <211> 10
   <212> PRT
   <213> Homo sapiens
<400> 903
   Cys Ser Gln Ser Asn Cys Cys Lys Pro Cys
   1 5 10
<210> 904
   <211> 10
   <212> PRT
   <213> Homo sapiens
<400> 904
   Cys Ser Gln Ser Ser Cys Cys Lys Pro Cys
   1 5 10
<210> 905
   <211> 10
   <212> PRT
   <213> Homo sapiens
<400> 905
   Cys Ser Ser Gly Cys Gly Ser Cys Cys Gln
   1 5 10
<210> 906
   <211> 10
   <212> PRT
   <213> Homo sapiens
<400> 906
   Cys Ser Ser Gly Cys Gly Ser Ser Cys Cys
   1 5 10
<210> 907
   <211> 10
   <212> PRT
   <213> Homo sapiens
<400> 907
   Cys Ser Ser Gly Cys Gln Pro Ala Cys Cys
   1               5                   10
<210> 908
   <211> 10
   <212> PRT
   <213> Homo sapiens
<400> 908
   Cys Ser Ser Ser Cys Cys Gln Pro Ser Cys
   1               5                   10
<210> 909
   <211> 10
   <212> PRT
   <213> Homo sapiens
<400> 909
   Cys Ser Thr Pro Cys Cys Gln Pro Thr Cys
   1               5                   10
<210> 910
   <211> 10
   <212> PRT
   <213> Homo sapiens
<400> 910
   Cys Ser Thr Thr Cys Cys Gln Pro Ile Cys
   1               5                   10
<210> 911
   <211> 10
   <212> PRT
   <213> Homo sapiens
<400> 911
   Cys Thr Ala Val Val Cys Arg Pro Cys Cys
   1               5                   10
<210> 912
   <211> 10
   <212> PRT
   <213> Homo sapiens
<400> 912
   Cys Thr Asp Ser Cys Thr Pro Ser Cys Cys
   1 5 10
<210> 913
   <211> 10
   <212> PRT
   <213> Homo sapiens
<400> 913
   Cys Thr Pro Ser Cys Cys Gln Pro Ala Cys
   1 5 10
<210> 914
   <211> 10
   <212> PRT
   <213> Homo sapiens
<400> 914
   Cys Thr Arg Pro Ile Cys Glu Pro Cys Cys
   1 5 10
<210> 915
   <211> 10
   <212> PRT
   <213> Homo sapiens
<400> 915
   Cys Thr Ser Ser Cys Thr Pro Ser Cys Cys
   1 5 10
<210> 916
   <211> 10
   <212> PRT
   <213> Homo sapiens
<400> 916
   Cys Val Pro Ala Cys Ser Cys Ser Ser Cys
   1 5 10
<210> 917
   <211> 10
   <212> PRT
   <213> Homo sapiens
<400> 917
   Cys Val Pro Ala Cys Ser Cys Thr Ser Cys
   1 5 10
<210> 918
   <211> 10
   <212> PRT
   <213> Homo sapiens
<400> 918
   Cys Val Pro Val Cys Cys Lys Pro Val Cys
   1               5                   10
<210> 919
   <211> 10
   <212> PRT
   <213> Homo sapiens
<400> 919
   Cys Val Pro Val Cys Cys Val Pro Thr Cys
   1               5                   10
<210> 920
   <211> 10
   <212> PRT
   <213> Homo sapiens
<400> 920
   Cys Val Pro Val Cys Cys Val Pro Val Cys
   1               5                   10
<210> 921
   <211> 10
   <212> PRT
   <213> Homo sapiens
<400> 921
   Cys Val Ser Cys Val Ser Ser Pro Cys Cys
   1               5                   10
<210> 922
   <211> 10
   <212> PRT
   <213> Homo sapiens
<400> 922
   Cys Val Ser Arg Cys Cys Arg Pro Gln Cys
   1               5                   10
<210> 923
   <211> 10
   <212> PRT
   <213> Homo sapiens
<400> 923
   Cys Val Ser Ser Cys Cys Lys Pro Gln Cys
   1               5                   10
<210> 924
   <211> 10
   <212> PRT
   <213> Homo sapiens
<400> 924
   Cys Val Ser Ser Cys Cys Gln His Ser Cys
   1 5 10
<210> 925
   <211> 10
   <212> PRT
   <213> Homo sapiens
<400> 925
   Cys Val Ser Ser Cys Cys Gln Pro Phe Cys
   1 5 10
<210> 926
   <211> 10
   <212> PRT
   <213> Homo sapiens
<400> 926
   Cys Val Ser Ser Cys Cys Gln Pro Ser Cys
   1 5 10
<210> 927
   <211> 10
   <212> PRT
   <213> Homo sapiens
<400> 927
   Cys Val Ser Ser Cys Cys Arg Pro Gln Cys
   1 5 10
<210> 928
   <211> 10
   <212> PRT
   <213> Homo sapiens
<400> 928
   Cys Val Ser Thr Cys Cys Arg Pro Thr Cys
   1 5 10
<210> 929
   <211> 10
   <212> PRT
   <213> Homo sapiens
<400> 929
   Cys Val Thr Arg Cys Cys Ser Thr Pro Cys
   1 5 10
<210> 930
   <211> 10
   <212> PRT
   <213> Homo sapiens
<400> 930
   Cys Val Thr Ser Cys Cys Gln Pro Ala Cys
   1 5 10
<210> 931
   <211> 10
   <212> PRT
   <213> Homo sapiens
<400> 931
   Cys Val Thr Ser Cys Cys Gln Pro Ser Cys
   1 5 10
<210> 932
   <211> 10
   <212> PRT
   <213> Homo sapiens
<400> 932
   Cys Val Tyr Ser Cys Cys Gln Pro Phe Cys
   1 5 10
<210> 933
   <211> 10
   <212> PRT
   <213> Homo sapiens
<400> 933
   Cys Val Tyr Ser Cys Cys Gln Pro Ser Cys
   1 5 10
<210> 934
   <211> 10
   <212> PRT
   <213> Homo sapiens
<400> 934
   Gly Cys Cys Gly Cys Ser Glu Gly Cys Gly
   1 5 10
<210> 935
   <211> 10
   <212> PRT
   <213> Homo sapiens
<400> 935
   Gly Cys Cys Gly Cys Ser Gly Gly Cys Gly
   1 5 10
<210> 936
   <211> 10
   <212> PRT
   <213> Homo sapiens
<400> 936
   Gly Cys Cys Gly Cys Ser Arg Gly Cys Gly
   1 5 10
<210> 937
   <211> 10
   <212> PRT
   <213> Homo sapiens
<400> 937
   Gly Cys Cys Arg Pro Ile Thr Cys Cys Pro
   1 5 10
<210> 938
   <211> 10
   <212> PRT
   <213> Homo sapiens
<400> 938
   Gly Cys Gly Ser Ser Cys Cys Gln Cys Ser
   1 5 10
<210> 939
   <211> 10
   <212> PRT
   <213> Homo sapiens
<400> 939
   Gly Cys Gly Val Pro Val Cys Cys Cys Ser
   1 5 10
<210> 940
   <211> 10
   <212> PRT
   <213> Homo sapiens
<400> 940
   Leu Cys Cys Pro Cys Gln Thr Thr Cys Ser
   1 5 10
<210> 941
   <211> 10
   <212> PRT
   <213> Homo sapiens
<400> 941
   Pro Cys Cys Cys Leu Arg Pro Val Cys Gly
   1 5 10
<210> 942
   <211> 10
   <212> PRT
   <213> Homo sapiens
<400> 942
   Pro Cys Cys Cys Arg Pro Val Thr Cys Gln
   1 5 10
<210> 943
   <211> 10
   <212> PRT
   <213> Homo sapiens
<400> 943
   Pro Cys Cys Cys Val Arg Pro Val Cys Gly
   1 5 10
<210> 944
   <211> 10
   <212> PRT
   <213> Homo sapiens
<400> 944
   Pro Cys Cys Ser Gln Ala Ser Cys Cys Val
   1 5 10
<210> 945
   <211> 10
   <212> PRT
   <213> Homo sapiens
<400> 945
   Pro Cys Cys Ser Gln Ser Arg Cys Cys Val
   1 5 10
<210> 946
   <211> 10
   <212> PRT
   <213> Homo sapiens
<400> 946
   Pro Cys Cys Ser Gln Ser Ser Cys Cys Lys
   1 5 10
<210> 947
   <211> 10
   <212> PRT
   <213> Homo sapiens
<400> 947
   Pro Cys Cys Ser Gln Ser Ser Cys Cys Val
   1 5 10
<210> 948
   <211> 10
   <212> PRT
   <213> Homo sapiens
<400> 948
   Pro Cys Cys Trp Ala Thr Thr Cys Cys Gln
   1 5 10
<210> 949
   <211> 10
   <212> PRT
   <213> Homo sapiens
<400> 949
   Gln Cys Ser Cys Cys Lys Pro Tyr Cys Ser
   1 5 10
<210> 950
   <211> 10
   <212> PRT
   <213> Homo sapiens
<400> 950
   Arg Cys Tyr Val Pro Val Cys Cys Cys Lys
   1 5 10
<210> 951
   <211> 10
   <212> PRT
   <213> Homo sapiens
<400> 951
   Ser Cys Cys Ala Pro Val Tyr Cys Cys Lys
   1 5 10
<210> 952
   <211> 10
   <212> PRT
   <213> Homo sapiens
<400> 952
   Ser Cys Cys Ile Ser Ser Ser Cys Cys Pro
   1 5 10
<210> 953
   <211> 10
   <212> PRT
   <213> Homo sapiens
<400> 953
   Ser Cys Cys Val Ser Ser Cys Arg Cys Pro
   1 5 10
<210> 954
   <211> 10
   <212> PRT
   <213> Homo sapiens
<400> 954
   Ser Cys Gly Cys Ser Gln Cys Ser Cys Tyr
   1 5 10
<210> 955
   <211> 10
   <212> PRT
   <213> Homo sapiens
<400> 955
   Ser Cys Gly Leu Glu Asn Cys Cys Cys Pro
   1 5 10
<210> 956
   <211> 10
   <212> PRT
   <213> Homo sapiens
<400> 956
   Val Cys Cys Gly Ala Ser Ser Cys Cys Gln
   1 5 10
<210> 957
   <211> 10
   <212> PRT
   <213> Homo sapiens
<400> 957
   Val Cys Cys Gly Asp Ser Ser Cys Cys Gln
   1 5 10
<210> 958
   <211> 11
   <212> PRT
   <213> Homo sapiens
<400> 958
   Cys Ala Ser Ser Cys Cys Thr Pro Ser Cys Cys
   1 5 10
<210> 959
   <211> 11
   <212> PRT
   <213> Homo sapiens
<400> 959
   Cys Cys Cys Pro Ser Cys Val Val Ser Ser Cys
   1 5 10
<210> 960
   <211> 11
   <212> PRT
   <213> Homo sapiens
<400> 960
   Cys Cys Cys Pro Ser Tyr Cys Val Ser Ser Cys
   1 5 10
<210> 961
   <211> 11
   <212> PRT
   <213> Homo sapiens
<400> 961
   Cys Cys Cys Ser Ser Gly Cys Gly Ser Ser Cys
   1 5 10
<210> 962
   <211> 11
   <212> PRT
   <213> Homo sapiens
<400> 962
   Cys Cys Asp Thr Cys Pro Pro Pro Cys Cys Lys
   1 5 10
<210> 963
   <211> 11
   <212> PRT
   <213> Homo sapiens
<400> 963
   Cys Cys Glu Pro His Cys Cys Ala Leu Ser Cys
   1 5 10
<210> 964
   <211> 11
   <212> PRT
   <213> Homo sapiens
<400> 964
   Cys Cys Glu Pro Pro Cys Cys Ala Pro Ser Cys
   1 5 10
<210> 965
   <211> 11
   <212> PRT
   <213> Homo sapiens
<400> 965
   Cys Cys Glu Pro Pro Cys Cys Ala Thr Ser Cys
   1               5                   10
<210> 966
   <211> 11
   <212> PRT
   <213> Homo sapiens
<400> 966
   Cys Cys Glu Thr Ser Cys Cys Gln Pro Ser Cys
   1               5                   10
<210> 967
   <211> 11
   <212> PRT
   <213> Homo sapiens
<400> 967
   Cys Cys Gly Ser Ser Cys Cys Gly Ser Gly Cys
   1               5                   10
<210> 968
   <211> 11
   <212> PRT
   <213> Homo sapiens
<400> 968
   Cys Cys Gly Ser Ser Cys Cys Gly Ser Ser Cys
   1               5                   10
<210> 969
   <211> 11
   <212> PRT
   <213> Homo sapiens
<400> 969
   Cys Cys His Pro Arg Cys Cys Ile Ser Ser Cys
   1               5                   10
<210> 970
   <211> 11
   <212> PRT
   <213> Homo sapiens
<400> 970
   Cys Cys His Pro Ser Cys Cys Glu Ser Ser Cys
   1 5 10
<210> 971
   <211> 11
   <212> PRT
   <213> Homo sapiens
<400> 971
   Cys Cys His Pro Ser Cys Cys Ile Ser Ser Cys
   1 5 10
<210> 972
   <211> 11
   <212> PRT
   <213> Homo sapiens
<400> 972
   Cys Cys His Pro Ser Cys Cys Val Ser Ser Cys
   1 5 10
<210> 973
   <211> 11
   <212> PRT
   <213> Homo sapiens
<400> 973
   Cys Cys His Pro Thr Cys Cys Gln Asn Thr Cys
   1 5 10
<210> 974
   <211> 11
   <212> PRT
   <213> Homo sapiens
<400> 974
   Cys Cys His Pro Thr Cys Cys Gln Thr Ile Cys
   1 5 10
<210> 975
   <211> 11
   <212> PRT
   <213> Homo sapiens
<400> 975
   Cys Cys Ile Ser Ser Cys Cys Lys Pro Ser Cys
   1 5 10
<210> 976
   <211> 11
   <212> PRT
   <213> Homo sapiens
<400> 976
   Cys Cys Ile Ser Ser Cys Cys Arg Pro Ser Cys
   1 5 10
<210> 977
   <211> 11
   <212> PRT
   <213> Homo sapiens
<400> 977
   Cys Cys Ile Ser Ser Ser Cys Cys Pro Ser Cys
   1 5 10
<210> 978
   <211> 11
   <212> PRT
   <213> Homo sapiens
<400> 978
   Cys Cys Lys Ala Val Cys Cys Val Pro Thr Cys
   1 5 10
<210> 979
   <211> 11
   <212> PRT
   <213> Homo sapiens
<400> 979
   Cys Cys Lys Pro Cys Cys Ser Gln Ala Ser Cys
   1 5 10
<210> 980
   <211> 11
   <212> PRT
   <213> Homo sapiens
<400> 980
   Cys Cys Lys Pro Cys Cys Ser Gln Ser Arg Cys
   1 5 10
<210> 981
   <211> 11
   <212> PRT
   <213> Homo sapiens
<400> 981
   Cys Cys Lys Pro Cys Cys Ser Gln Ser Ser Cys
   1 5 10
<210> 982
   <211> 11
   <212> PRT
   <213> Homo sapiens
<400> 982
   Cys Cys Lys Pro Cys Cys Ser Ser Ser Gly Cys
   1               5                   10
<210> 983
   <211> 11
   <212> PRT
   <213> Homo sapiens
<400> 983
   Cys Cys Lys Pro Cys Ser Cys Phe Ser Gly Cys
   1               5                   10
<210> 984
   <211> 11
   <212> PRT
   <213> Homo sapiens
<400> 984
   Cys Cys Lys Pro Cys Ser Cys Ser Ser Gly Cys
   1               5                   10
<210> 985
   <211> 11
   <212> PRT
   <213> Homo sapiens
<400> 985
   Cys Cys Lys Pro Cys Tyr Cys Ser Ser Gly Cys
   1               5                   10
<210> 986
   <211> 11
   <212> PRT
   <213> Homo sapiens
<400> 986
   Cys Cys Lys Pro Ile Cys Cys Val Pro Val Cys
   1               5                   10
<210> 987
   <211> 11
   <212> PRT
   <213> Homo sapiens
<400> 987
   Cys Cys Lys Pro Gln Cys Cys Gln Ser Val Cys
   1               5                   10
<210> 988
   <211> 11
   <212> PRT
   <213> Homo sapiens
<400> 988
   Cys Cys Lys Pro Val Cys Cys Lys Pro Ile Cys
   1 5 10
<210> 989
   <211> 11
   <212> PRT
   <213> Homo sapiens
<400> 989
   Cys Cys Lys Pro Tyr Cys Cys Gln Ser Ser Cys
   1 5 10
<210> 990
   <211> 11
   <212> PRT
   <213> Homo sapiens
<400> 990
   Cys Cys Lys Pro Tyr Cys Ser Gln Cys Ser Cys
   1 5 10
<210> 991
   <211> 11
   <212> PRT
   <213> Homo sapiens
<400> 991
   Cys Cys Met Pro Val Cys Cys Lys Pro Val Cys
   1 5 10
<210> 992
   <211> 11
   <212> PRT
   <213> Homo sapiens
<400> 992
   Cys Cys Met Pro Val Cys Cys Lys Thr Val Cys
   1 5 10
<210> 993
   <211> 11
   <212> PRT
   <213> Homo sapiens
<400> 993
   Cys Cys Met Ser Ser Cys Cys Lys Pro Gln Cys
   1 5 10
<210> 994
   <211> 11
   <212> PRT
   <213> Homo sapiens
<400> 994
   Cys Cys Asn Pro Cys Cys Ser Gln Ser Ser Cys
   1 5 10
<210> 995
   <211> 11
   <212> PRT
   <213> Homo sapiens
<400> 995
   Cys Cys Pro Gly Asp Cys Phe Thr Cys Cys Thr
   1 5 10
<210> 996
   <211> 11
   <212> PRT
   <213> Homo sapiens
<400> 996
   Cys Cys Pro Ser Cys Val Val Ser Ser Cys Cys
   1 5 10
<210> 997
   <211> 11
   <212> PRT
   <213> Homo sapiens
<400> 997
   Cys Cys Pro Ser Tyr Cys Val Ser Ser Cys Cys
   1 5 10
<210> 998
   <211> 11
   <212> PRT
   <213> Homo sapiens
<400> 998
   Cys Cys Gln Asn Thr Cys Cys Arg Thr Thr Cys
   1 5 10
<210> 999
   <211> 11
   <212> PRT
   <213> Homo sapiens
<400> 999
   Cys Cys Gln Pro Ala Cys Cys Val Ser Ser Cys
   1 5 10
<210> 1000
   <211> 11
   <212> PRT
   <213> Homo sapiens
<400> 1000
   Cys Cys Gln Pro Cys Cys His Pro Thr Cys Tyr
   1 5 10
<210> 1001
   <211> 11
   <212> PRT
   <213> Homo sapiens
<400> 1001
   Cys Cys Gln Pro Cys Cys Arg Pro Thr Ser Cys
   1 5 10
<210> 1002
   <211> 11
   <212> PRT
   <213> Homo sapiens
<400> 1002
   Cys Cys Gln Pro Ile Cys Gly Ser Ser Cys Cys
   1 5 10
<210> 1003
   <211> 11
   <212> PRT
   <213> Homo sapiens
<400> 1003
   Cys Cys Gln Pro Ile Cys Val Thr Ser Cys Cys
   1 5 10
<210> 1004
   <211> 11
   <212> PRT
   <213> Homo sapiens
<400> 1004
   Cys Cys Gln Pro Asn Cys Cys Arg Pro Ser Cys
   1 5 10
<210> 1005
   <211> 11
   <212> PRT
   <213> Homo sapiens
<400> 1005
   Cys Cys Gln Pro Ser Cys Cys Glu Thr Ser Cys
   1 5 10
<210> 1006
   <211> 11
   <212> PRT
   <213> Homo sapiens
<400> 1006
   Cys Cys Gln Pro Ser Cys Cys Arg Pro Ala Cys
   1 5 10
<210> 1007
   <211> 11
   <212> PRT
   <213> Homo sapiens
<400> 1007
   Cys Cys Gln Pro Ser Cys Cys Ser Thr Pro Cys
   1 5 10
<210> 1008
   <211> 11
   <212> PRT
   <213> Homo sapiens
<400> 1008
   Cys Cys Gln Pro Ser Cys Cys Ser Thr Thr Cys
   1 5 10
<210> 1009
   <211> 11
   <212> PRT
   <213> Homo sapiens
<400> 1009
   Cys Cys Gln Pro Ser Cys Cys Val Pro Ser Cys
   1 5 10
<210> 1010
   <211> 11
   <212> PRT
   <213> Homo sapiens
<400> 1010
   Cys Cys Gln Pro Ser Cys Cys Val Ser Ser Cys
   1 5 10
<210> 1011
   <211> 11
   <212> PRT
   <213> Homo sapiens
<400> 1011
   Cys Cys Gln Pro Thr Cys Cys His Pro Ser Cys
   1 5 10
<210> 1012
   <211> 11
   <212> PRT
   <213> Homo sapiens
<400> 1012
   Cys Cys Gln Pro Thr Cys Cys Gln Pro Thr Cys
   1 5 10
<210> 1013
   <211> 11
   <212> PRT
   <213> Homo sapiens
<400> 1013
   Cys Cys Gln Pro Thr Cys Cys Arg Pro Arg Cys
   1 5 10
<210> 1014
   <211> 11
   <212> PRT
   <213> Homo sapiens
<400> 1014
   Cys Cys Gln Pro Thr Cys Cys Arg Pro Ser Cys
   1 5 10
<210> 1015
   <211> 11
   <212> PRT
   <213> Homo sapiens
<400> 1015
   Cys Cys Gln Pro Thr Cys Cys Arg Pro Thr Cys
   1 5 10
<210> 1016
   <211> 11
   <212> PRT
   <213> Homo sapiens
<400> 1016
   Cys Cys Gln Pro Thr Cys Cys Arg Thr Thr Cys
   1 5 10
<210> 1017
   <211> 11
   <212> PRT
   <213> Homo sapiens
<400> 1017
   Cys Cys Gln Pro Thr Cys Leu Ser Ser Cys Cys
   1 5 10
<210> 1018
   <211> 11
   <212> PRT
   <213> Homo sapiens
<400> 1018
   Cys Cys Gln Pro Thr Cys Leu Thr Ser Cys Cys
   1 5 10
<210> 1019
   <211> 11
   <212> PRT
   <213> Homo sapiens
<400> 1019
   Cys Cys Gln Pro Thr Cys Val Ala Ser Cys Cys
   1 5 10
<210> 1020
   <211> 11
   <212> PRT
   <213> Homo sapiens
<400> 1020
   Cys Cys Gln Pro Thr Cys Val Thr Ser Cys Cys
   1 5 10
<210> 1021
   <211> 11
   <212> PRT
   <213> Homo sapiens
<400> 1021
   Cys Cys Gln Pro Tyr Cys His Pro Thr Cys Cys
   1 5 10
<210> 1022
   <211> 11
   <212> PRT
   <213> Homo sapiens
<400> 1022
   Cys Cys Gln Ser Met Cys Cys Gln Pro Thr Cys
   1 5 10
<210> 1023
   <211> 11
   <212> PRT
   <213> Homo sapiens
<400> 1023
   Cys Cys Gln Ser Asn Cys Cys Val Pro Val Cys
   1 5 10
<210> 1024
   <211> 11
   <212> PRT
   <213> Homo sapiens
<400> 1024
   Cys Cys Gln Ser Ser Cys Cys Lys Pro Cys Ser
   1 5 10
<210> 1025
   <211> 11
   <212> PRT
   <213> Homo sapiens
<400> 1025
   Cys Cys Gln Ser Ser Cys Cys Lys Pro Ser Cys
   1 5 10
<210> 1026
   <211> 11
   <212> PRT
   <213> Homo sapiens
<400> 1026
   Cys Cys Gln Ser Ser Cys Cys Lys Pro Tyr Cys
   1 5 10
<210> 1027
   <211> 11
   <212> PRT
   <213> Homo sapiens
<400> 1027
   Cys Cys Gln Ser Ser Cys Cys Gln Ser Ser Cys
   1 5 10
<210> 1028
   <211> 11
   <212> PRT
   <213> Homo sapiens
<400> 1028
   Cys Cys Gln Ser Ser Cys Cys Val Pro Val Cys
   1 5 10
<210> 1029
   <211> 11
   <212> PRT
   <213> Homo sapiens
<400> 1029
   Cys Cys Gln Ser Ser Cys Phe Lys Pro Cys Cys
   1 5 10
<210> 1030
   <211> 11
   <212> PRT
   <213> Homo sapiens
<400> 1030
   Cys Cys Gln Ser Ser Cys Ser Lys Pro Cys Cys
   1 5 10
<210> 1031
   <211> 11
   <212> PRT
   <213> Homo sapiens
<400> 1031
   Cys Cys Gln Ser Ser Cys Tyr Lys Pro Cys Cys
   1 5 10
<210> 1032
   <211> 11
   <212> PRT
   <213> Homo sapiens
<400> 1032
   Cys Cys Gln Ser Val Cys Cys Gln Pro Thr Cys
   1 5 10
<210> 1033
   <211> 11
   <212> PRT
   <213> Homo sapiens
<400> 1033
   Cys Cys Gln Thr Ile Cys Arg Ser Thr Cys Cys
   1 5 10
<210> 1034
   <211> 11
   <212> PRT
   <213> Homo sapiens
<400> 1034
   Cys Cys Gln Thr Thr Cys Cys Arg Pro Ser Cys
   1 5 10
<210> 1035
   <211> 11
   <212> PRT
   <213> Homo sapiens
<400> 1035
   Cys Cys Gln Thr Thr Cys Cys Arg Thr Thr Cys
   1 5 10
<210> 1036
   <211> 11
   <212> PRT
   <213> Homo sapiens
<400> 1036
   Cys Cys Arg Pro Ala Cys Cys Glu Thr Thr Cys
   1 5 10
<210> 1037
   <211> 11
   <212> PRT
   <213> Homo sapiens
<400> 1037
   Cys Cys Arg Pro Ala Cys Cys Gln Asn Thr Cys
   1 5 10
<210> 1038
   <211> 11
   <212> PRT
   <213> Homo sapiens
<400> 1038
   Cys Cys Arg Pro Leu Cys Cys Gln Thr Thr Cys
   1 5 10
<210> 1039
   <211> 11
   <212> PRT
   <213> Homo sapiens
<400> 1039
   Cys Cys Arg Pro Gln Cys Cys Gln Ser Val Cys
   1 5 10
<210> 1040
   <211> 11
   <212> PRT
   <213> Homo sapiens
<400> 1040
   Cys Cys Arg Pro Gln Cys Cys Gln Thr Thr Cys
   1 5 10
<210> 1041
   <211> 11
   <212> PRT
   <213> Homo sapiens
<400> 1041
   Cys Cys Arg Pro Ser Cys Cys Glu Ser Ser Cys
   1 5 10
<210> 1042
   <211> 11
   <212> PRT
   <213> Homo sapiens
<400> 1042
   Cys Cys Arg Pro Ser Cys Cys Glu Thr Thr Cys
   1 5 10
<210> 1043
   <211> 11
   <212> PRT
   <213> Homo sapiens
<400> 1043
   Cys Cys Arg Pro Ser Cys Cys Gly Ser Ser Cys
   1 5 10
<210> 1044
   <211> 11
   <212> PRT
   <213> Homo sapiens
<400> 1044
   Cys Cys Arg Pro Ser Cys Cys Ile Ser Ser Cys
   1 5 10
<210> 1045
   <211> 11
   <212> PRT
   <213> Homo sapiens
<400> 1045
   Cys Cys Arg Pro Ser Cys Cys Lys Pro Gln Cys
   1 5 10
<210> 1046
   <211> 11
   <212> PRT
   <213> Homo sapiens
<400> 1046
   Cys Cys Arg Pro Ser Cys Cys Gln Thr Thr Cys
   1               5                   10
<210> 1047
   <211> 11
   <212> PRT
   <213> Homo sapiens
<400> 1047
   Cys Cys Arg Pro Ser Cys Cys Val Ser Arg Cys
   1               5                   10
<210> 1048
   <211> 11
   <212> PRT
   <213> Homo sapiens
<400> 1048
   Cys Cys Arg Pro Ser Cys Cys Val Ser Ser Cys
   1               5                   10
<210> 1049
   <211> 11
   <212> PRT
   <213> Homo sapiens
<400> 1049
   Cys Cys Arg Pro Thr Cys Cys Gln Asn Thr Cys
   1               5                   10
<210> 1050
   <211> 11
   <212> PRT
   <213> Homo sapiens
<400> 1050
   Cys Cys Arg Pro Thr Cys Cys Gln Thr Thr Cys
   1               5                   10
<210> 1051
   <211> 11
   <212> PRT
   <213> Homo sapiens
<400> 1051
   Cys Cys Arg Pro Val Cys Cys Asp Pro Cys Ser
   1               5                   10
<210> 1052
   <211> 11
   <212> PRT
   <213> Homo sapiens
<400> 1052
   Cys Cys Arg Thr Thr Cys Cys Gln Pro Thr Cys
   1 5 10
<210> 1053
   <211> 11
   <212> PRT
   <213> Homo sapiens
<400> 1053
   Cys Cys Arg Thr Thr Cys Cys Arg Pro Ser Cys
   1 5 10
<210> 1054
   <211> 11
   <212> PRT
   <213> Homo sapiens
<400> 1054
   Cys Cys Arg Thr Thr Cys Cys Arg Thr Thr Cys
   1 5 10
<210> 1055
   <211> 11
   <212> PRT
   <213> Homo sapiens
<400> 1055
   Cys Cys Ser Cys Ser Ser Cys Gly Ser Cys Ala
   1 5 10
<210> 1056
   <211> 11
   <212> PRT
   <213> Homo sapiens
<400> 1056
   Cys Cys Ser Pro Gly Cys Gln Pro Thr Cys Cys
   1 5 10
<210> 1057
   <211> 11
   <212> PRT
   <213> Homo sapiens
<400> 1057
   Cys Cys Ser Gln Ser Ser Cys Cys Lys Pro Cys
   1 5 10
<210> 1058
   <211> 11
   <212> PRT
   <213> Homo sapiens
<400> 1058
   Cys Cys Ser Ser Gly Cys Gly Ser Cys Cys Gln
   1 5 10
<210> 1059
   <211> 11
   <212> PRT
   <213> Homo sapiens
<400> 1059
   Cys Cys Ser Ser Gly Cys Gly Ser Ser Cys Cys
   1 5 10
<210> 1060
   <211> 11
   <212> PRT
   <213> Homo sapiens
<400> 1060
   Cys Cys Ser Thr Pro Cys Cys Gln Pro Thr Cys
   1 5 10
<210> 1061
   <211> 11
   <212> PRT
   <213> Homo sapiens
<400> 1061
   Cys Cys Val Pro Ala Cys Ser Cys Ser Ser Cys
   1 5 10
<210> 1062
   <211> 11
   <212> PRT
   <213> Homo sapiens
<400> 1062
   Cys Cys Val Pro Ala Cys Ser Cys Thr Ser Cys
   1 5 10
<210> 1063
   <211> 11
   <212> PRT
   <213> Homo sapiens
<400> 1063
   Cys Cys Val Pro Ile Cys Cys Lys Pro Ile Cys
   1               5                   10
<210> 1064
   <211> 11
   <212> PRT
   <213> Homo sapiens
<400> 1064
   Cys Cys Val Pro Ile Cys Cys Lys Pro Val Cys
   1               5                   10
<210> 1065
   <211> 11
   <212> PRT
   <213> Homo sapiens
<400> 1065
   Cys Cys Val Pro Val Cys Cys Lys Pro Ile Cys
   1               5                   10
<210> 1066
   <211> 11
   <212> PRT
   <213> Homo sapiens
<400> 1066
   Cys Cys Val Pro Val Cys Cys Lys Pro Val Cys
   1               5                   10
<210> 1067
   <211> 11
   <212> PRT
   <213> Homo sapiens
<400> 1067
   Cys Cys Val Pro Val Cys Cys Lys Ser Asn Cys
   1               5                   10
<210> 1068
   <211> 11
   <212> PRT
   <213> Homo sapiens
<400> 1068
   Cys Cys Val Pro Val Cys Cys Lys Thr Val Cys
   1               5                   10
<210> 1069
   <211> 11
   <212> PRT
   <213> Homo sapiens
<400> 1069
   Cys Cys Val Pro Val Cys Cys Ser Ser Ser Cys
   1               5                   10
<210> 1070
   <211> 11
   <212> PRT
   <213> Homo sapiens
<400> 1070
   Cys Cys Val Pro Val Cys Cys Val Pro Val Cys
   1               5                   10
<210> 1071
   <211> 11
   <212> PRT
   <213> Homo sapiens
<400> 1071
   Cys Cys Val Ser Ser Cys Cys Lys Pro Gln Cys
   1               5                   10
<210> 1072
   <211> 11
   <212> PRT
   <213> Homo sapiens
<400> 1072
   Cys Cys Val Ser Ser Cys Cys Gln His Ser Cys
   1               5                   10
<210> 1073
   <211> 11
   <212> PRT
   <213> Homo sapiens
<400> 1073
   Cys Cys Val Ser Ser Cys Cys Gln Pro Ser Cys
   1               5                   10
<210> 1074
   <211> 11
   <212> PRT
   <213> Homo sapiens
<400> 1074
   Cys Cys Val Ser Ser Cys Cys Arg Pro Gln Cys
   1               5                   10
<210> 1075
   <211> 11
   <212> PRT
   <213> Homo sapiens
<400> 1075
   Cys Cys Val Ser Thr Cys Cys Arg Pro Thr Cys
   1 5 10
<210> 1076
   <211> 11
   <212> PRT
   <213> Homo sapiens
<400> 1076
   Cys Cys Val Ser Val Cys Cys Lys Pro Val Cys
   1 5 10
<210> 1077
   <211> 11
   <212> PRT
   <213> Homo sapiens
<400> 1077
   Cys Asp Ser Ser Cys Cys Gln Pro Ser Cys Cys
   1 5 10
<210> 1078
   <211> 11
   <212> PRT
   <213> Homo sapiens
<400> 1078
   Cys Glu Pro Cys Cys Arg Pro Val Cys Cys Asp
   1 5 10
<210> 1079
   <211> 11
   <212> PRT
   <213> Homo sapiens
<400> 1079
   Cys Phe Lys Pro Cys Cys Cys Gln Ser Ser Cys
   1 5 10
<210> 1080
   <211> 11
   <212> PRT
   <213> Homo sapiens
<400> 1080
   Cys Gly Asp Gly Cys Cys Cys Pro Ser Cys Tyr
   1 5 10
<210> 1081
   <211> 11
   <212> PRT
   <213> Homo sapiens
<400> 1081
   Cys Gly Gly Gly Cys Cys Gly Ser Ser Cys Cys
   1 5 10
<210> 1082
   <211> 11
   <212> PRT
   <213> Homo sapiens
<400> 1082
   Cys Gly Gly Ser Cys Cys Gly Ser Ser Cys Cys
   1 5 10
<210> 1083
   <211> 11
   <212> PRT
   <213> Homo sapiens
<400> 1083
   Cys Gly Leu Glu Asn Cys Cys Cys Pro Ser Cys
   1 5 10
<210> 1084
   <211> 11
   <212> PRT
   <213> Homo sapiens
<400> 1084
   Cys Gly Gln Ser Cys Cys Arg Pro Ala Cys Cys
   1 5 10
<210> 1085
   <211> 11
   <212> PRT
   <213> Homo sapiens
<400> 1085
   Cys Gly Gln Ser Cys Cys Arg Pro Val Cys Cys
   1 5 10
<210> 1086
   <211> 11
   <212> PRT
   <213> Homo sapiens
<400> 1086
   Cys Gly Ser Cys Cys Gln Ser Ser Cys Cys Asn
   1 5 10
<210> 1087
   <211> 11
   <212> PRT
   <213> Homo sapiens
<400> 1087
   Cys Gly Ser Cys Gly Cys Ser Gln Cys Asn Cys
   1 5 10
<210> 1088
   <211> 11
   <212> PRT
   <213> Homo sapiens
<400> 1088
   Cys Gly Ser Cys Gly Cys Ser Gln Cys Ser Cys
   1 5 10
<210> 1089
   <211> 11
   <212> PRT
   <213> Homo sapiens
<400> 1089
   Cys Gly Ser Gly Cys Cys Gly Pro Val Cys Cys
   1 5 10
<210> 1090
   <211> 11
   <212> PRT
   <213> Homo sapiens
<400> 1090
   Cys Gly Ser Gly Cys Cys Val Pro Val Cys Cys
   1 5 10
<210> 1091
   <211> 11
   <212> PRT
   <213> Homo sapiens
<400> 1091
   Cys Gly Ser Asn Cys Cys Gln Pro Cys Cys Arg
   1 5 10
<210> 1092
   <211> 11
   <212> PRT
   <213> Homo sapiens
<400> 1092
   Cys Gly Ser Ser Cys Cys Gln Pro Cys Cys His
   1 5 10
<210> 1093
   <211> 11
   <212> PRT
   <213> Homo sapiens
<400> 1093
   Cys Gly Ser Ser Cys Cys Gln Pro Cys Cys Arg
   1 5 10
<210> 1094
   <211> 11
   <212> PRT
   <213> Homo sapiens
<400> 1094
   Cys Gly Ser Ser Cys Cys Gln Pro Cys Tyr Cys
   1 5 10
<210> 1095
   <211> 11
   <212> PRT
   <213> Homo sapiens
<400> 1095
   Cys Gly Ser Ser Cys Cys Gln Pro Ser Cys Cys
   1 5 10
<210> 1096
   <211> 11
   <212> PRT
   <213> Homo sapiens
<400> 1096
   Cys Gly Ser Ser Cys Cys Gln Ser Ser Cys Cys
   1 5 10
<210> 1097
   <211> 11
   <212> PRT
   <213> Homo sapiens
<400> 1097
   Cys Gly Ser Ser Cys Cys Val Pro Ile Cys Cys
   1 5 10
<210> 1098
   <211> 11
   <212> PRT
   <213> Homo sapiens
<400> 1098
   Cys Gly Ser Ser Cys Cys Val Pro Val Cys Cys
   1 5 10
<210> 1099
   <211> 11
   <212> PRT
   <213> Homo sapiens
<400> 1099
   Cys Gly Ser Ser Cys Ser Gln Cys Ser Cys Cys
   1 5 10
<210> 1100
   <211> 11
   <212> PRT
   <213> Homo sapiens
<400> 1100
   Cys Gly Val Pro Val Cys Cys Cys Ser Cys Ser
   1 5 10
<210> 1101
   <211> 11
   <212> PRT
   <213> Homo sapiens
<400> 1101
   Cys His Pro Arg Cys Cys Ile Ser Ser Cys Cys
   1 5 10
<210> 1102
   <211> 11
   <212> PRT
   <213> Homo sapiens
<400> 1102
   Cys His Pro Ser Cys Cys Glu Ser Ser Cys Cys
   1 5 10
<210> 1103
   <211> 11
   <212> PRT
   <213> Homo sapiens
<400> 1103
   Cys His Pro Ser Cys Cys Ile Ser Ser Cys Cys
   1 5 10
<210> 1104
   <211> 11
   <212> PRT
   <213> Homo sapiens
<400> 1104
   Cys His Pro Thr Cys Cys Gln Asn Thr Cys Cys
   1               5                   10
<210> 1105
   <211> 11
   <212> PRT
   <213> Homo sapiens
<400> 1105
   Cys Ile Ser Ser Cys Cys His Pro Ser Cys Cys
   1               5                   10
<210> 1106
   <211> 11
   <212> PRT
   <213> Homo sapiens
<400> 1106
   Cys Ile Ser Ser Cys Cys Lys Pro Ser Cys Cys
   1               5                   10
<210> 1107
   <211> 11
   <212> PRT
   <213> Homo sapiens
<400> 1107
   Cys Ile Ser Ser Cys Cys Arg Pro Ser Cys Cys
   1               5                   10
<210> 1108
   <211> 11
   <212> PRT
   <213> Homo sapiens
<400> 1108
   Cys Ile Ser Ser Ser Cys Cys Pro Ser Cys Cys
   1               5                   10
<210> 1109
   <211> 11
   <212> PRT
   <213> Homo sapiens
<400> 1109
   Cys Lys Pro Cys Cys Cys Ser Ser Gly Cys Gly
   1               5                   10
<210> 1110
   <211> 11
   <212> PRT
   <213> Homo sapiens
<400> 1110
   Cys Lys Pro Cys Cys Ser Gln Ala Ser Cys Cys
   1               5                   10
<210> 1111
   <211> 11
   <212> PRT
   <213> Homo sapiens
<400> 1111
   Cys Lys Pro Cys Cys Ser Gln Ser Arg Cys Cys
   1               5                   10
<210> 1112
   <211> 11
   <212> PRT
   <213> Homo sapiens
<400> 1112
   Cys Lys Pro Cys Cys Ser Gln Ser Ser Cys Cys
   1               5                   10
<210> 1113
   <211> 11
   <212> PRT
   <213> Homo sapiens
<400> 1113
   Cys Lys Pro Gln Cys Cys Gln Ser Met Cys Cys
   1               5                   10
<210> 1114
   <211> 11
   <212> PRT
   <213> Homo sapiens
<400> 1114
   Cys Lys Pro Gln Cys Cys Gln Ser Val Cys Cys
   1               5                   10
<210> 1115
   <211> 11
   <212> PRT
   <213> Homo sapiens
<400> 1115
   Cys Lys Pro Val Cys Cys Cys Val Pro Ala Cys
   1 5 10
<210> 1116
   <211> 11
   <212> PRT
   <213> Homo sapiens
<400> 1116
   Cys Lys Pro Val Cys Cys Lys Pro Ile Cys Cys
   1 5 10
<210> 1117
   <211> 11
   <212> PRT
   <213> Homo sapiens
<400> 1117
   Cys Lys Pro Val Cys Cys Met Pro Val Cys Cys
   1 5 10
<210> 1118
   <211> 11
   <212> PRT
   <213> Homo sapiens
<400> 1118
   Cys Lys Pro Val Cys Cys Val Pro Val Cys Cys
   1 5 10
<210> 1119
   <211> 11
   <212> PRT
   <213> Homo sapiens
<400> 1119
   Cys Lys Pro Val Cys Cys Val Ser Val Cys Cys
   1 5 10
<210> 1120
   <211> 11
   <212> PRT
   <213> Homo sapiens
<400> 1120
   Cys Lys Pro Tyr Cys Ser Gln Cys Ser Cys Cys
   1 5 10
<210> 1121
   <211> 11
   <212> PRT
   <213> Homo sapiens
<400> 1121
   Cys Leu Pro Cys Cys Arg Pro Thr Cys Cys Gln
   1 5 10
<210> 1122
   <211> 11
   <212> PRT
   <213> Homo sapiens
<400> 1122
   Cys Leu Thr Ser Cys Cys Gln Pro Ser Cys Cys
   1 5 10
<210> 1123
   <211> 11
   <212> PRT
   <213> Homo sapiens
<400> 1123
   Cys Met Ser Ser Cys Cys Lys Pro Gln Cys Cys
   1 5 10
<210> 1124
   <211> 11
   <212> PRT
   <213> Homo sapiens
<400> 1124
   Cys Asn Pro Cys Cys Ser Gln Ser Ser Cys Cys
   1 5 10
<210> 1125
   <211> 11
   <212> PRT
   <213> Homo sapiens
<400> 1125
   Cys Pro Ala Cys Cys Val Ser Ser Cys Cys Gln
   1 5 10
<210> 1126
   <211> 11
   <212> PRT
   <213> Homo sapiens
<400> 1126
   Cys Pro Glu Ser Cys Cys Glu Pro His Cys Cys
   1 5 10
<210> 1127
   <211> 11
   <212> PRT
   <213> Homo sapiens
<400> 1127
   Cys Pro Glu Ser Cys Cys Glu Pro Pro Cys Cys
   1 5 10
<210> 1128
   <211> 11
   <212> PRT
   <213> Homo sapiens
<400> 1128
   Cys Pro Ser Cys Cys Glu Ser Ser Cys Cys Arg
   1 5 10
<210> 1129
   <211> 11
   <212> PRT
   <213> Homo sapiens
<400> 1129
   Cys Pro Ser Cys Cys Gln Thr Thr Cys Cys Arg
   1 5 10
<210> 1130
   <211> 11
   <212> PRT
   <213> Homo sapiens
<400> 1130
   Cys Pro Ser Cys Cys Val Ser Ser Cys Cys Arg
   1 5 10
<210> 1131
   <211> 11
   <212> PRT
   <213> Homo sapiens
<400> 1131
   Cys Gln Cys Ser Cys Cys Lys Pro Tyr Cys Ser
   1 5 10
<210> 1132
   <211> 11
   <212> PRT
   <213> Homo sapiens
<400> 1132
   Cys Gln Glu Thr Cys Cys Arg Pro Ser Cys Cys
   1 5 10
<210> 1133
   <211> 11
   <212> PRT
   <213> Homo sapiens
<400> 1133
   Cys Gln Asn Thr Cys Cys Arg Thr Thr Cys Cys
   1 5 10
<210> 1134
   <211> 11
   <212> PRT
   <213> Homo sapiens
<400> 1134
   Cys Gln Pro Ala Cys Cys Thr Ala Ser Cys Cys
   1 5 10
<210> 1135
   <211> 11
   <212> PRT
   <213> Homo sapiens
<400> 1135
   Cys Gln Pro Ala Cys Cys Thr Ser Ser Cys Cys
   1 5 10
<210> 1136
   <211> 11
   <212> PRT
   <213> Homo sapiens
<400> 1136
   Cys Gln Pro Ala Cys Cys Thr Thr Ser Cys Cys
   1 5 10
<210> 1137
   <211> 11
   <212> PRT
   <213> Homo sapiens
<400> 1137
   Cys Gln Pro Ala Cys Cys Val Pro Val Cys Cys
   1 5 10
<210> 1138
   <211> 11
   <212> PRT
   <213> Homo sapiens
<400> 1138
   Cys Gln Pro Ala Cys Cys Val Ser Ser Cys Cys
   1 5 10
<210> 1139
   <211> 11
   <212> PRT
   <213> Homo sapiens
<400> 1139
   Cys Gln Pro Cys Cys His Pro Thr Cys Cys Gln
   1               5                   10
<210> 1140
   <211> 11
   <212> PRT
   <213> Homo sapiens
<400> 1140
   Cys Gln Pro Cys Cys Arg Pro Ala Cys Cys Glu
   1               5                   10
<210> 1141
   <211> 11
   <212> PRT
   <213> Homo sapiens
<400> 1141
   Cys Gln Pro Cys Cys Arg Pro Ala Cys Cys Gln
   1               5                   10
<210> 1142
   <211> 11
   <212> PRT
   <213> Homo sapiens
<400> 1142
   Cys Gln Pro Cys Cys Arg Pro Thr Cys Cys Gln
   1               5                   10
<210> 1143
   <211> 11
   <212> PRT
   <213> Homo sapiens
<400> 1143
   Cys Gln Pro Cys Tyr Cys Pro Ala Cys Cys Val
   1               5                   10
<210> 1144
   <211> 11
   <212> PRT
   <213> Homo sapiens
<400> 1144
   Cys Gln Pro Ile Cys Cys Gly Ser Ser Cys Cys
   1 5 10
<210> 1145
   <211> 11
   <212> PRT
   <213> Homo sapiens
<400> 1145
   Cys Gln Pro Arg Cys Cys Glu Thr Ser Cys Cys
   1 5 10
<210> 1146
   <211> 11
   <212> PRT
   <213> Homo sapiens
<400> 1146
   Cys Gln Pro Ser Cys Cys Glu Thr Ser Cys Cys
   1 5 10
<210> 1147
   <211> 11
   <212> PRT
   <213> Homo sapiens
<400> 1147
   Cys Gln Pro Ser Cys Cys Arg Pro Ala Cys Cys
   1 5 10
<210> 1148
   <211> 11
   <212> PRT
   <213> Homo sapiens
<400> 1148
   Cys Gln Pro Ser Cys Cys Val Pro Ser Cys Cys
   1 5 10
<210> 1149
   <211> 11
   <212> PRT
   <213> Homo sapiens
<400> 1149
   Cys Gln Pro Ser Cys Cys Val Ser Ser Cys Cys
   1 5 10
<210> 1150
   <211> 11
   <212> PRT
   <213> Homo sapiens
<400> 1150
   Cys Gln Pro Thr Cys Cys Cys Pro Ser Tyr Cys
   1 5 10
<210> 1151
   <211> 11
   <212> PRT
   <213> Homo sapiens
<400> 1151
   Cys Gln Pro Thr Cys Cys Gly Ser Ser Cys Cys
   1 5 10
<210> 1152
   <211> 11
   <212> PRT
   <213> Homo sapiens
<400> 1152
   Cys Gln Pro Thr Cys Cys His Pro Ser Cys Cys
   1 5 10
<210> 1153
   <211> 11
   <212> PRT
   <213> Homo sapiens
<400> 1153
   Cys Gln Pro Thr Cys Cys Gln Pro Thr Cys Cys
   1 5 10
<210> 1154
   <211> 11
   <212> PRT
   <213> Homo sapiens
<400> 1154
   Cys Gln Pro Thr Cys Cys Arg Pro Ser Cys Cys
   1 5 10
<210> 1155
   <211> 11
   <212> PRT
   <213> Homo sapiens
<400> 1155
   Cys Gln Pro Thr Cys Cys Arg Pro Thr Cys Cys
   1 5 10
<210> 1156
   <211> 11
   <212> PRT
   <213> Homo sapiens
<400> 1156
   Cys Gln Pro Thr Cys Cys Arg Thr Thr Cys Cys
   1 5 10
<210> 1157
   <211> 11
   <212> PRT
   <213> Homo sapiens
<400> 1157
   Cys Gln Gln Ala Cys Cys Met Pro Val Cys Cys
   1 5 10
<210> 1158
   <211> 11
   <212> PRT
   <213> Homo sapiens
<400> 1158
   Cys Gln Gln Ala Cys Cys Val Pro Ile Cys Cys
   1 5 10
<210> 1159
   <211> 11
   <212> PRT
   <213> Homo sapiens
<400> 1159
   Cys Gln Gln Ala Cys Cys Val Pro Val Cys Cys
   1 5 10
<210> 1160
   <211> 11
   <212> PRT
   <213> Homo sapiens
<400> 1160
   Cys Gln Gln Ser Cys Cys Val Pro Val Cys Cys
   1 5 10
<210> 1161
   <211> 11
   <212> PRT
   <213> Homo sapiens
<400> 1161
   Cys Gln Gln Ser Cys Cys Val Ser Val Cys Cys
   1 5 10
<210> 1162
   <211> 11
   <212> PRT
   <213> Homo sapiens
<400> 1162
   Cys Gln Ser Asn Cys Cys Val Pro Val Cys Cys
   1               5                   10
<210> 1163
   <211> 11
   <212> PRT
   <213> Homo sapiens
<400> 1163
   Cys Gln Ser Ser Cys Cys Cys Pro Ala Ser Cys
   1               5                   10
<210> 1164
   <211> 11
   <212> PRT
   <213> Homo sapiens
<400> 1164
   Cys Gln Ser Ser Cys Cys Lys Pro Cys Cys Ser
   1               5                   10
<210> 1165
   <211> 11
   <212> PRT
   <213> Homo sapiens
<400> 1165
   Cys Gln Ser Ser Cys Cys Lys Pro Cys Ser Cys
   1               5                   10
<210> 1166
   <211> 11
   <212> PRT
   <213> Homo sapiens
<400> 1166
   Cys Gln Ser Ser Cys Cys Lys Pro Tyr Cys Cys
   1               5                   10
<210> 1167
   <211> 11
   <212> PRT
   <213> Homo sapiens
<400> 1167
   Cys Gln Ser Ser Cys Cys Asn Pro Cys Cys Ser
   1               5                   10
<210> 1168
   <211> 11
   <212> PRT
   <213> Homo sapiens
<400> 1168
   Cys Gln Ser Ser Cys Cys Gln Ser Ser Cys Cys
   1               5                   10
<210> 1169
   <211> 11
   <212> PRT
   <213> Homo sapiens
<400> 1169
   Cys Gln Ser Ser Cys Cys Val Pro Val Cys Cys
   1               5                   10
<210> 1170
   <211> 11
   <212> PRT
   <213> Homo sapiens
<400> 1170
   Cys Gln Ser Ser Cys Phe Lys Pro Cys Cys Cys
   1               5                   10
<210> 1171
   <211> 11
   <212> PRT
   <213> Homo sapiens
<400> 1171
   Cys Gln Ser Ser Cys Ser Lys Pro Cys Cys Cys
   1               5                   10
<210> 1172
   <211> 11
   <212> PRT
   <213> Homo sapiens
<400> 1172
   Cys Gln Ser Ser Cys Tyr Lys Pro Cys Cys Cys
   1               5                   10
<210> 1173
   <211> 11
   <212> PRT
   <213> Homo sapiens
<400> 1173
   Cys Gln Ser Val Cys Cys Gln Pro Thr Cys Cys
   1               5                   10
<210> 1174
   <211> 11
   <212> PRT
   <213> Homo sapiens
<400> 1174
   Cys Gln Thr Thr Cys Cys Cys Pro Ser Cys Val
   1               5                   10
<210> 1175
   <211> 11
   <212> PRT
   <213> Homo sapiens
<400> 1175
   Cys Gln Thr Thr Cys Cys Arg Pro Ser Cys Cys
   1               5                   10
<210> 1176
   <211> 11
   <212> PRT
   <213> Homo sapiens
<400> 1176
   Cys Gln Thr Thr Cys Cys Arg Thr Thr Cys Cys
   1               5                   10
<210> 1177
   <211> 11
   <212> PRT
   <213> Homo sapiens
<400> 1177
   Cys Arg Pro Ala Cys Cys Glu Thr Thr Cys Cys
   1               5                   10
<210> 1178
   <211> 11
   <212> PRT
   <213> Homo sapiens
<400> 1178
   Cys Arg Pro Ala Cys Cys Gln Asn Thr Cys Cys
   1               5                   10
<210> 1179
   <211> 11
   <212> PRT
   <213> Homo sapiens
<400> 1179
   Cys Arg Pro Cys Cys Cys Leu Arg Pro Val Cys
   1 5 10
<210> 1180
   <211> 11
   <212> PRT
   <213> Homo sapiens
<400> 1180
   Cys Arg Pro Cys Cys Cys Val Arg Pro Val Cys
   1 5 10
<210> 1181
   <211> 11
   <212> PRT
   <213> Homo sapiens
<400> 1181
   Cys Arg Pro Cys Cys Trp Ala Thr Thr Cys Cys
   1 5 10
<210> 1182
   <211> 11
   <212> PRT
   <213> Homo sapiens
<400> 1182
   Cys Arg Pro Leu Cys Cys Gln Thr Thr Cys Cys
   1 5 10
<210> 1183
   <211> 11
   <212> PRT
   <213> Homo sapiens
<400> 1183
   Cys Arg Pro Gln Cys Cys Gln Ser Val Cys Cys
   1 5 10
<210> 1184
   <211> 11
   <212> PRT
   <213> Homo sapiens
<400> 1184
   Cys Arg Pro Gln Cys Cys Gln Thr Thr Cys Cys
   1 5 10
<210> 1185
   <211> 11
   <212> PRT
   <213> Homo sapiens
<400> 1185
   Cys Arg Pro Arg Cys Cys Ile Ser Ser Cys Cys
   1 5 10
<210> 1186
   <211> 11
   <212> PRT
   <213> Homo sapiens
<400> 1186
   Cys Arg Pro Ser Cys Cys Glu Ser Ser Cys Cys
   1 5 10
<210> 1187
   <211> 11
   <212> PRT
   <213> Homo sapiens
<400> 1187
   Cys Arg Pro Ser Cys Cys Ile Ser Ser Cys Cys
   1 5 10
<210> 1188
   <211> 11
   <212> PRT
   <213> Homo sapiens
<400> 1188
   Cys Arg Pro Ser Cys Cys Lys Pro Gln Cys Cys
   1 5 10
<210> 1189
   <211> 11
   <212> PRT
   <213> Homo sapiens
<400> 1189
   Cys Arg Pro Ser Cys Cys Pro Ser Cys Cys Gln
   1 5 10
<210> 1190
   <211> 11
   <212> PRT
   <213> Homo sapiens
<400> 1190
   Cys Arg Pro Ser Cys Cys Gln Thr Thr Cys Cys
   1 5 10
<210> 1191
   <211> 11
   <212> PRT
   <213> Homo sapiens
<400> 1191
   Cys Arg Pro Ser Cys Cys Arg Pro Gln Cys Cys
   1 5 10
<210> 1192
   <211> 11
   <212> PRT
   <213> Homo sapiens
<400> 1192
   Cys Arg Pro Ser Cys Cys Val Ser Arg Cys Cys
   1 5 10
<210> 1193
   <211> 11
   <212> PRT
   <213> Homo sapiens
<400> 1193
   Cys Arg Pro Ser Cys Cys Val Ser Ser Cys Cys
   1 5 10
<210> 1194
   <211> 11
   <212> PRT
   <213> Homo sapiens
<400> 1194
   Cys Arg Pro Thr Cys Cys Gln Asn Thr Cys Cys
   1 5 10
<210> 1195
   <211> 11
   <212> PRT
   <213> Homo sapiens
<400> 1195
   Cys Arg Pro Val Cys Cys Cys Glu Pro Thr Cys
   1 5 10
<210> 1196
   <211> 11
   <212> PRT
   <213> Homo sapiens
<400> 1196
   Cys Arg Pro Val Cys Cys Cys Tyr Ser Cys Glu
   1 5 10
<210> 1197
   <211> 11
   <212> PRT
   <213> Homo sapiens
<400> 1197
   Cys Arg Thr Thr Cys Cys His Pro Ser Cys Cys
   1 5 10
<210> 1198
   <211> 11
   <212> PRT
   <213> Homo sapiens
<400> 1198
   Cys Arg Thr Thr Cys Cys Arg Pro Ser Cys Cys
   1 5 10
<210> 1199
   <211> 11
   <212> PRT
   <213> Homo sapiens
<400> 1199
   Cys Ser Cys Cys Lys Pro Tyr Cys Ser Gln Cys
   1 5 10
<210> 1200
   <211> 11
   <212> PRT
   <213> Homo sapiens
<400> 1200
   Cys Ser Lys Pro Cys Cys Cys Gln Ser Ser Cys
   1 5 10
<210> 1201
   <211> 11
   <212> PRT
   <213> Homo sapiens
<400> 1201
   Cys Ser Pro Cys Cys Gln Pro Thr Cys Cys Arg
   1 5 10
<210> 1202
   <211> 11
   <212> PRT
   <213> Homo sapiens
<400> 1202
   Cys Ser Pro Cys Cys Val Ser Ser Cys Cys Gln
   1 5 10
<210> 1203
   <211> 11
   <212> PRT
   <213> Homo sapiens
<400> 1203
   Cys Ser Gln Cys Ser Cys Cys Lys Pro Cys Tyr
   1 5 10
<210> 1204
   <211> 11
   <212> PRT
   <213> Homo sapiens
<400> 1204
   Cys Ser Gln Cys Ser Cys Tyr Lys Pro Cys Cys
   1 5 10
<210> 1205
   <211> 11
   <212> PRT
   <213> Homo sapiens
<400> 1205
   Cys Ser Gln Ser Asn Cys Cys Lys Pro Cys Cys
   1 5 10
<210> 1206
   <211> 11
   <212> PRT
   <213> Homo sapiens
<400> 1206
   Cys Ser Gln Ser Ser Cys Cys Lys Pro Cys Cys
   1 5 10
<210> 1207
   <211> 11
   <212> PRT
   <213> Homo sapiens
<400> 1207
   Cys Ser Ser Ser Cys Cys Gln Pro Ser Cys Cys
   1 5 10
<210> 1208
   <211> 11
   <212> PRT
   <213> Homo sapiens
<400> 1208
   Cys Thr Pro Ser Cys Cys Gln Pro Ala Cys Cys
   1 5 10
<210> 1209
   <211> 11
   <212> PRT
   <213> Homo sapiens
<400> 1209
   Cys Val Ala Ser Cys Cys Gln Pro Ser Cys Cys
   1 5 10
<210> 1210
   <211> 11
   <212> PRT
   <213> Homo sapiens
<400> 1210
   Cys Val Pro Ile Cys Cys Cys Lys Pro Val Cys
   1 5 10
<210> 1211
   <211> 11
   <212> PRT
   <213> Homo sapiens
<400> 1211
   Cys Val Pro Ser Cys Cys Gln Pro Cys Cys His
   1 5 10
<210> 1212
   <211> 11
   <212> PRT
   <213> Homo sapiens
<400> 1212
   Cys Val Pro Val Cys Cys Cys Lys Pro Met Cys
   1 5 10
<210> 1213
   <211> 11
   <212> PRT
   <213> Homo sapiens
<400> 1213
   Cys Val Pro Val Cys Cys Cys Lys Pro Val Cys
   1 5 10
<210> 1214
   <211> 11
   <212> PRT
   <213> Homo sapiens
<400> 1214
   Cys Val Pro Val Cys Cys Lys Pro Val Cys Cys
   1 5 10
<210> 1215
   <211> 11
   <212> PRT
   <213> Homo sapiens
<400> 1215
   Cys Val Ser Ser Cys Cys Lys Pro Gln Cys Cys
   1 5 10
<210> 1216
   <211> 11
   <212> PRT
   <213> Homo sapiens
<400> 1216
   Cys Val Ser Ser Cys Cys Gln His Ser Cys Cys
   1 5 10
<210> 1217
   <211> 11
   <212> PRT
   <213> Homo sapiens
<400> 1217
   Cys Val Ser Ser Cys Cys Gln Pro Cys Cys His
   1 5 10
<210> 1218
   <211> 11
   <212> PRT
   <213> Homo sapiens
<400> 1218
   Cys Val Ser Ser Cys Cys Gln Pro Cys Cys Arg
   1 5 10
<210> 1219
   <211> 11
   <212> PRT
   <213> Homo sapiens
<400> 1219
   Cys Val Ser Ser Cys Cys Gln Pro Phe Cys Cys
   1 5 10
<210> 1220
   <211> 11
   <212> PRT
   <213> Homo sapiens
<400> 1220
   Cys Val Ser Ser Cys Cys Gln Pro Ser Cys Cys
   1 5 10
<210> 1221
   <211> 11
   <212> PRT
   <213> Homo sapiens
<400> 1221
   Cys Val Ser Ser Cys Cys Arg Pro Gln Cys Cys
   1 5 10
<210> 1222
   <211> 11
   <212> PRT
   <213> Homo sapiens
<400> 1222
   Cys Val Thr Arg Cys Cys Ser Thr Pro Cys Cys
   1 5 10
<210> 1223
   <211> 11
   <212> PRT
   <213> Homo sapiens
<400> 1223
   Cys Val Thr Ser Cys Cys Gln Pro Ala Cys Cys
   1 5 10
<210> 1224
   <211> 11
   <212> PRT
   <213> Homo sapiens
<400> 1224
   Cys Val Thr Ser Cys Cys Gln Pro Ser Cys Cys
   1 5 10
<210> 1225
   <211> 11
   <212> PRT
   <213> Homo sapiens
<400> 1225
   Cys Val Tyr Ser Cys Cys Gln Pro Phe Cys Cys
   1 5 10
<210> 1226
   <211> 11
   <212> PRT
   <213> Homo sapiens
<400> 1226
   Cys Val Tyr Ser Cys Cys Gln Pro Ser Cys Cys
   1 5 10
<210> 1227
   <211> 11
   <212> PRT
   <213> Homo sapiens
<400> 1227
   Cys Tyr Cys Pro Ala Cys Cys Val Ser Ser Cys
   1 5 10
<210> 1228
   <211> 11
   <212> PRT
   <213> Homo sapiens
<400> 1228
   Cys Tyr Lys Pro Cys Cys Cys Gln Ser Ser Cys
   1 5 10
<210> 1229
   <211> 11
   <212> PRT
   <213> Homo sapiens
<400> 1229
   Cys Tyr Lys Pro Cys Cys Cys Ser Ser Gly Cys
   1 5 10
<210> 1230
   <211> 11
   <212> PRT
   <213> Homo sapiens
<400> 1230
   Met Cys Cys Cys Val Pro Ala Cys Ser Cys Ser
   1 5 10
<210> 1231
   <211> 11
   <212> PRT
   <213> Homo sapiens
<400> 1231
   Asn Cys Cys Val Pro Val Cys Cys Gln Cys Lys
   1 5 10
<210> 1232
   <211> 11
   <212> PRT
   <213> Homo sapiens
<400> 1232
   Gln Cys Ser Cys Cys Lys Pro Cys Tyr Cys Ser
   1 5 10
<210> 1233
   <211> 11
   <212> PRT
   <213> Homo sapiens
<400> 1233
   Gln Cys Ser Cys Tyr Lys Pro Cys Cys Cys Ser
   1 5 10
<210> 1234
   <211> 11
   <212> PRT
   <213> Homo sapiens
<400> 1234
   Ser Cys Cys Val Pro Ile Cys Cys Gln Cys Lys
   1 5 10
<210> 1235
   <211> 11
   <212> PRT
   <213> Homo sapiens
<400> 1235
   Ser Cys Cys Val Pro Val Cys Cys Gln Cys Lys
   1 5 10
<210> 1236
   <211> 11
   <212> PRT
   <213> Homo sapiens
<400> 1236
   Ser Cys Gly Cys Ser Gln Cys Asn Cys Cys Lys
   1 5 10
<210> 1237
   <211> 11
   <212> PRT
   <213> Homo sapiens
<400> 1237
   Ser Cys Gly Cys Ser Gln Cys Ser Cys Cys Lys
   1               5                   10
<210> 1238
   <211> 11
   <212> PRT
   <213> Homo sapiens
<400> 1238
   Val Cys Cys Cys Val Pro Ala Cys Ser Cys Ser
   1               5                   10
<210> 1239
   <211> 11
   <212> PRT
   <213> Homo sapiens
<400> 1239
   Val Cys Cys Cys Val Pro Ala Cys Ser Cys Thr
   1               5                   10